# EUROPÄISCHE PATENTSCHRIFT

(11) **EP 0 561 853 B1**
(45) Veröffentlichungstag und Bekanntmachung des Hinweises auf die Patenterteilung: **25.02.1998**
(21) Anmeldenummer: 92900007.3
(22) Anmeldetag: 03.12.1991
(51) Int. Cl.: A01N 25/32, C07D 333/26, C07D 333/62

(54) **SUBSTITUIERTE 2-AMINOTHIOPHENE ENTHALTENDE HERBIZIDE MITTEL**
HERBICIDAL AGENTS CONTAINING SUBSTITUTED 2-AMINOTHIOPHENE
AGENTS HERBICIDES CONTENANT DES 2-AMINOTHIOPHENES SUBSTITUES

(30) Priorität: 13.12.1990 DE 4039734
(43) Veröffentlichungstag der Anmeldung: 29.09.1993
(73) Patentinhaber: BASF Aktiengesellschaft, 67063 Ludwigshafen (DE)
(72) Erfinder: HAGEN, Helmut, D-6710 Frankenthal (DE); NILZ, Gerhard, D-6701 Dannstadt-Schauernheim (DE); WALTER, Helmut, D-6719 Obrigheim (DE); LANDES, Andreas, D-6703 Limburgerhof (DE)
(86) Internationale Anmeldenummer: EP9102287
(87) Internationale Veröffentlichungsnummer: WO9210094

(56) Entgegenhaltungen:
- EP-A- 0 127 469
- US-A- 4 415 743

## Beschreibung

Die vorliegende Erfindung betrifft herbizide Mittel, enthaltend mindestens ein substituiertes 2-Aminothiophen der allgemeinen Formel I 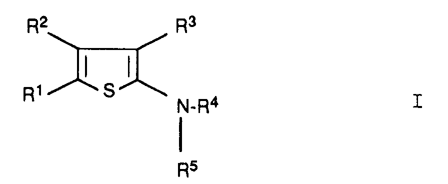 in der die Variablen die folgende Bedeutung haben:
- R¹ u. R²: zusammen eine C₃-C₆-Alkylenkette, an die ein Benzolring anelliert sein kann, wobei dieser Benzolring noch einen bis drei der folgenden Reste tragen kann: Halogen, C₁-C₄-Alkyl, partiell oder vollständig halogeniertes C₁-C₄-Alkyl, C₁-C₄-Alkoxy, partiell oder vollständig halogeniertes C₁-C₄-Alkoxy oder C₁-C₄-Alkylthio und der zusätzlich noch ein weiteres Halogenatom tragen kann, so daß die Gesamtzahl der Reste 4 beträgt;
eine durch Sauerstoff oder eine Gruppe -N(R⁷)- unterbrochene C₂-C₃-Alkylenkette, die noch einen bis vier C₁-C₄-Alkylreste tragen kann, wobei R⁷ Wasserstoff, Phenyl oder C₁-C₄-Alkyl bedeutet;
eine Gruppe -CO-N(R⁷)-CO-;
- R³: die Cyanogruppe, oder
eine Gruppe CX-R⁶, wobei X Sauerstoff oder Schwefel bedeutet und wobei R⁶ Hydroxyl, C₁-C₄-Alkyl, C₁-C₄-Alkoxy, C₁-C₄-Alkoxy-C₁-C₄-alkoxy, Amino, C₁-C₄-Alkylamino, Di-(C₁-C₄)-alkylamino, C₃-C₆-Cycloalkylamino oder Phenylamino, das noch einen bis drei Halogen-, C₁-C₄-Alkyl- und/oder C₁-C₄-Alkoxyreste tragen kann, bedeutet;
- R⁴: Wasserstoff, eine C₁-C₄-Alkylgruppe, eine C₂₋C₄-Alkenylgruppe, die Phenylgruppe, die noch einen bis drei der folgenden Reste tragen kann: Nitro, Halogen, C₁-C₄-Alkyl, partiell oder vollständig halogeniertes C₁-C₄-Alkyl, C₁-C₄-Alkoxy, partiell oder vollständig halogeniertes C₁-C₄-Alkoxy oder C₁-C₄-Alkylthio und wobei die Phenylgruppe noch so viele Halogenatome tragen kann, daß die Gesamtzahl der Reste 4 oder 5 beträgt;
einen über ein C-Atom gebundenen Triazinring, der an jedem der beiden anderen C-Atome noch ein Halogenatom tragen kann oder
eine Gruppe -PX(OR⁸)₂, -CX-R⁹, -SO₂R¹⁰, -SO₂-NHR¹⁰, -CX-N(R⁷)-CY-R⁸ oder -CX-N(R⁷)-SO₂-R¹¹, wobei die Variablen die folgende Bedeutung haben:
- Y: Sauerstoff oder Schwefel;
- R⁸: Wasserstoff, eine C₁-C₄-Alkylgruppe oder die Phenylgruppe, die einen bis drei der folgenden Reste tragen kann: C₁-C₄-Alkyl, partiell oder vollständig halogeniertes C₁-C₄-Alkyl, C₁-C₄-Alkoxy, partiell oder vollständig halogeniertes C₁-C₄-Alkoxy oder C₁-C₄-Alkylthio;
- R⁹: C₁-C₂₀-Alkyl, partiell oder vollständig halogeniertes C₁-C₆-Alkyl, C₂-C₆-Alkenyl, partiell oder vollständig halogeniertes C₂-C₆-Alkenyl, C₃-C₆-Cycloalkyl, C₅-C₆-Cycloalkenyl, wobei die cyclischen Reste noch eine Hydroxycarbonylgruppe tragen können, Amino, C₁-C₄-Alkylamino, Di-(C₁-C₄)-alkylamino, C₃-C₈-Cycloalkylamino, Phenyl oder Phenylamino, wobei der Aromat jeweils noch einen bis drei der folgenden Substituenten tragen kann: Nitro, Halogen, C₁-C₄-Alkyl, partiell oder vollständig halogeniertes C₁-C₄-Alkyl, C₁-C₄-Alkoxy, partiell oder vollständig halogeniertes C₁-C₄-Alkoxy oder C₁-C₄-Alkylthio, und wobei der Aromat jeweils noch so viele Halogenatome zusätzlich tragen kann, daß die Gesamtzahl der Reste 4 oder 5 beträgt;
- R¹⁰: C₁-C₄-Alkyl oder Phenyl, das einen bis drei der folgenden Reste tragen kann: Halogen, C₁-C₄-Alkyl, partiell oder vollständig halogeniertes C₁-C₄-Alkyl, C₁-C₄-Alkoxy, partiell oder vollständig halogeniertes C₁-C₄-Alkoxy oder C₁-C₄-Alkylthio, und das zusätzlich noch so viele Halogenatome tragen kann, daß die Gesamtzahl der Reste 4 oder 5 beträgt;
- R¹¹: Amino, C₁-C₄-Alkylamino, Di-(C₁-C₄)-alkylamino, Pyrrolidin-1-yl, Piperidin-1-yl, Morpholin-4-yl oder Piperazin-1-yl, wobei die Heterocyclen noch eine oder zwei C₁-C₄-Alkylgruppen tragen können;
oder
- R³ u. R⁴: zusammen eine Gruppe -CO-O-CO-;
- R⁵: Wasserstoff, eine C₁-C₄-Alkylgruppe oder eine Gruppe -CX-R⁹ oder -SO₂R¹⁰
oder
- R⁴ u.R⁵: zusammen eine Grupe =CR¹²R¹³ oder -CO-W-CO-, wobei die Variablen die folgende Bedeutung haben:
- R¹²: Wasserstoff, Amino, C₁-C₄-Alkylamino oder C₃-C₈-Cycloalkylamino;
- R¹³: Amino, C₁-C₄-Alkylamino, Di-(C₁-C₄)-alkylamino, C₃-C₈-Cycloalkylamino, Thienylamino, wobei der Heteroaromat noch einen Cyano- oder Carbonylaminorest und/oder einen anellierten Benzolring tragen kann, Pyrrolidin-1-yl, Piperidin-2-yl, Morpholin-4-yl, Phenyl oder Pyridyl, wobei die beiden letztgenannten Substituenten noch einen bis drei der folgenden Reste tragen können: Halogen, Nitro, C₁-C₄-Alkyl, partiell oder vollständig halogeniertes C₁-C₄-Alkyl, C₁-C₄-Alkoxy, partiell oder vollständig halogeniertes C₁-C₄-Alkoxy oder C₁-C₄- Alkylthio;
- W: eine Ethylen- oder Ethenylenbrücke, eine 5- oder 6-gliedrige 1,2-C-verknüpfte aromatische oder heteroaromatische Brücke mit einem Stickstoff-, Sauerstoff oder Schwefelatom als Heteroatom, wobei diese Brückenglieder an jedem substituierbaren C-Atom noch einen Rest tragen können, ausgewählt aus einer Gruppe von bis zu 2 der folgenden Reste: Halogen, C₁-C₄-Alkyl, partiell oder vollständig halogeniertes C₁-C₄-Alkyl, C₁-C₄-Alkoxy, partiell oder vollständig halogeniertes C₁-C₄-Alkoxy, C₁-C₄-Alkylthio oder partiell oder vollständig halogeniertes C₁-C₄-Alkylthio, und wobei die Brückenglieder zusätzlich noch so viele Halogenatome tragen können, wie weitere substituierbare C-Atome vorhanden sind; oder eine 5- oder 6-gliedrige 1,2-C-verknüpfte Cycloalkylen- oder Cycloalkenylenbrücke, wobei diese Substituenten noch einen bis vier der folgenden Reste tragen können: Halogen oder C₁-C₄-Alkyl,
sowie die basischen Salze der Verbindungen I, bei denen R³ eine Hydroxycarbonyl- oder Hydroxythiocarbonylgruppe bedeutet und die sauren Salze derjenigen Verbindungen I, die ein basisches Stickstoffatom enthalten, als antagonistisch wirksame Verbindung
und mindestens einen herbiziden Wirkstoff aus der Gruppe der Cyclohexenonderivate der Formel III, 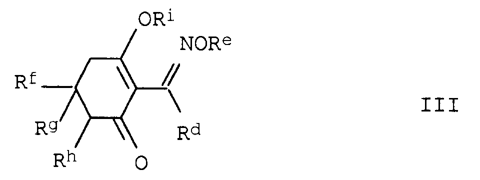 wobei die Substituenten folgende Bedeutung haben:
- R^{d}: eine C₁-C₄-Alkylgruppe;
- R^{e}: eine C₁-C₄-Alkylgruppe, eine C₃-C₄-Alkenylgruppe, eine C₃-C₄-Alkinylgruppe oder eine partiell oder vollständig halogenierte C₃-C₄-Alkenylgruppe;
eine C₁-C₄-Alkylen- oder C₂-C₄-Alkenylkette, die beide noch ein bis 3 C₁-C₃-Alkylreste und/oder Halogenatome tragen können, oder eine gewünschtenfalls durch
C₁-C₃-Alkyl substituierte 3- bis 6-gliedrige Alkylen- oder 4- bis 6-gliedrige Alkenylenkette, die jeweils ein dem Oximetherteil nicht direkt benachbartes Sauerstoff- oder Schwefelatom als Kettenglied enthält, wobei alle vorstehend genannten Ketten endständig den Phenylring tragen, der seinerseits durch ein bis drei Reste substituiert sein kann, ausgewählt aus einer Gruppe bestehend aus einem Benzyloxycarbonyl- oder Phenylrest und jeweils einem bis drei der folgenden Reste: Nitro, Cyano, Halogen, C₁-C₄-Alkyl, C₁-C₄-Alkoxy, C₁-C₄-Alkylthio, partiell oder vollständig halogeniertes C₁-C₄-Alkyl, partiell oder vollständig halogeniertes C₁-C₄-Alkoxy, Carboxyl und C₁-C₄-Alkoxycarbonyl, und wobei der Phenylring noch zusätzlich so viele Halogenatome tragen kann, daß die Gesamtzahl der Reste 4 oder 5 beträgt;
eine Thienylmethylgruppe, die noch ein Halogenatom tragen kann;
- R^{f}: eine C₁-C₄-Alkylgruppe, welche einfach oder zweifach durch C₁-C₄-Alkylthio oder C₁-C₄-Alkoxy substituiert sein kann;
ein 5- oder 6-gliedriges gesättigtes oder einfach ungesättigtes Ringsystem, welches neben Kohlenstoffgliedern ein Sauerstoff-, ein Schwefelatom oder eine Sulfoxid- oder Sulfongruppe enthalten kann, wobei dieser Ring bis zu drei der folgenden Reste tragen kann: Hydroxy, Halogen, C₁-C₄-Alkyl, partiell oder vollständig halogeniertes C₁-C₄-Alkyl, C₁-C₄-Alkoxy und/oder C₁-C₄-Alkylthio;
einen 10-gliedrigen gesättigten oder einfach ungesättigten Heterocyclus, welcher zwei Sauerstoffatome oder Schwefelatome enthält und durch bis zu drei C₁-C₄-Alkylgruppen und/oder Methoxygruppen substituiert sein kann;
die Phenylgruppe, die Pyridylgruppe, die Thiazolylgruppe, die Pyrazolylgruppe, die Pyrrolylgruppe oder die Isoxazolylgruppe, wobei diese Gruppen gewünschtenfalls einen bis drei Reste tragen können,
ausgewählt aus einer Gruppe bestehend aus C₁-C₄-Alkyl, partiell oder vollständig halogeniertem C₁-C₄-Alkyl, C₁-C₄-Alkoxy, C₁-C₄-Alkylthio, C₃-C₆-Alkenyloxy, C₃-C₆-Alkinyloxy, C₁-C₄-Alkoxy-C₁-C₃-alkyl, C₁-C₄-Alkyl-thio-C₁-C₃-alkyl, C₁-C₄-Dialkoxy-C₁-C₃-alkyl, Formyl, Halogen und Benzoylamino;
- R^{g}: Wasserstoff, Hydroxy oder, wenn R^{f} die Bedeutung C₁-C₆-Alkylgruppe hat, eine C₁-C₆-Alkylgruppe;
- R^{h}: Wasserstoff, die Cyanogruppe, ein Halogenatom, eine C₁-C₄-Alkoxycarbonylgruppe oder eine Gruppe 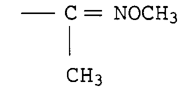 und
- Rⁱ: Wasserstoff oder das Äquivalent eines landwirtschaftlich brauchbaren Kations.

Außerdem betrifft die Erfindung ein Verfahren zur selektiven Bekämpfung von unerwünschtem Pflanzenwuchs mit diesen herbiziden Mitteln.

Substituierte 2-Aminothiophene vom Typ der Verbindungen I sind unter anderem aus folgenden Druckschriften bekannt:
- EP-A-384 314;
- EP-A-385 515;
- E.F. Elslager et al., J. Heterocycl. Chem. 9, 775 (1972);
- E.C. Taylor et al., J. Org. Chem. 32, 2376 (1967);
- K. Gewald, Chem. Ber. 98, 3571 (1965);
- K. Gewald et al., Chem. Ber. 99, 94 (1966);

Eine antagonistische Wirkung der bekannten Verbindungen in Kombination mit herbiziden Wirkstoffen ist der genannten Literatur jedoch nicht zu entnehmen.

Dagegen werden in der EP-A-378 508 antidotisch wirksame 3-Aminothiophene mit folgender Grundstruktur offenbart:

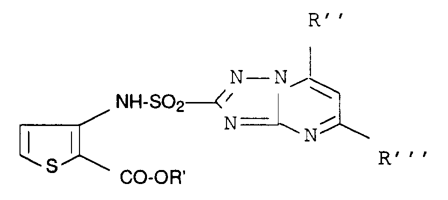

Der vorliegenden Erfindung lag nun die Aufgabe zugrunde, Verbindungen zu finden, welche die Nachteile, die bei der Verwendung der obengenannten Herbizide der Formel III bestehen, zumindest so stark zu verringern, daß der Ernteertrag der Kulturpflanzen nicht mehr oder nicht nennenswert herabgesetzt wird.

Entsprechend der Aufgabe wurden die eingangs definierten Mittel gefunden.

Weiterhin wurden Verfahren zur kombinierten Behandlung von Pflanzenkulturen mit den antidotisch wirkenden Verbindungen I einerseits und den Herbiziden III andererseits gefunden, wobei es unerheblich ist, ob der herbizide Wirkstoff und die antidotische Verbindung gemeinsam oder getrennt formuliert und ausgebracht werden bzw. bei getrennter Ausbringung, in welcher Reihenfolge herbizider Wirkstoff und Antidot appliziert werden.

Derivate I mit sauren Endgruppen oder mit basischen Stickstoffatomen können in Form ihrer landwirtschaftlich brauchbaren Salze vorliegen.

Als Säureadditionssalze eignen sich die Salze von solchen Säuren, welche die antidotische Wirkung von I nicht beeinträchtigen, also z.B. die Hydrochloride und -bromide, Sulfate, Nitrate, Phosphate, Oxalate oder die Dodecylbenzolsulfonate.

Als basische Salze eignen sich die Salze von solchen Basen, welche die antidotische Wirkung von I nicht beeinträchtigen, also beispielsweise die Alkalimetallsalze, insbesondere Natrium- und Kaliumsalze, die Erdalkalimetallsalze, insbesondere Calcium-, Magnesium- und Bariumsalze, Übergangsmetallsalze, insbesondere Mangan-, Kupfer-, Zink- und Eisensalze, Ammoniumsalze, die ein bis vier C₁-C₄-Alkyl- oder Hydroxy-C₁-C₄-Alkylsubstituenten und/oder einen Phenyl- oder Benzylsubstituenten tragen können, insbesondere Diisopropylammonium-, Tetramethylammonium-, Tetrabutylammonium-, Trimethylbenzylammonium- und Trimethyl-(2-hydroxyethyl)-ammoniumsalze, die Phosphoniumsalze, die Sulfoniumsalze, insbesondere Tri-(C₁-C₄)-alkylsulfoniumsalze sowie die Sulfoxoniumsalze, insbesondere Tri-(C₁-C₄)-alkylsulfoxoniumsalze.

Im einzelnen haben die Substituenten in den substituierten 2-Aminothiophenen I die folgende Bedeutung:
R¹ und R² zusammen
   - eine C₃-C₆-Akylenkette, an die ein Benzolring anelliert sein kann, wobei dieser Benzolring noch einen bis drei der folgenden Reste tragen kann: Halogen wie Fluor, Chlor Brom und Jod, insbesondere Fluor und Chlor; C₁-C₄-Alkyl wie Methyl, Ethyl, n-Propyl, Isopropyl, n-Butyl, Isobutyl und tert.-Butyl, insbesondere Methyl; partiell oder vollständig halogeniertes Alkyl wie Fluormethyl, Difluormethyl, Trifluormethyl, Chlormethyl, Trichlormethyl, Brommethyl, Pentafluorethyl und 2-Chlor-1,1,2-trifluorethyl, insbesondere Trifluormethyl; C₁-C₄-Alkoxy wie Methoxy, Ethoxy, n-Propoxy, Isopropoxy, n-Butoxy und tert.-Butoxy, insbesondere Methoxy, partiell oder vollständig halogeniertes Alkoxy wie Fluormethoxy, Difluormethoxy, Trifluormethoxy, Chlormethoxy, Dichlormethoxy und Trichlormethoxy, insbesondere Trifluormethoxy; C₁-C₄-Alkylthio wie Methylthio, Ethylthio, n-Propylthio, Isopropylthio, n-Butylthio und tert.-Butylthio, insbesondere Methylthio; und wobei der Benzolring zusätzlich so viele Halogenatome wie vorstehend genannt, insbesondere Fluor und Chlor, tragen kann, daß die Gesamtzahl der Reste 4 beträgt;
   - eine durch Sauerstoff oder eine Gruppe -N(R⁷)- unterbrochene C₂-C₃-Alkylenkette, die noch eine bis vier C₁-C₄-Alkylreste wie vorstehend genannt, insbesondere Methyl, tragen kann, wobei
      - R⁷: Wasserstoff, Phenyl oder C₁-C₄-Alkyl wie vorstehend genannt, insbesondere Methyl, bedeutet;
   - eine Gruppe -CO-N(R⁷)-CO-;
   bevorzugt bedeuten R¹ und R² zusammen eine C₃-C₆-Alkylenkette, wobei die Tetramethylenkette besonders bevorzugt ist;
R³
   - die Cyanogruppe;
   - eine Gruppe CX-R⁶, wobei X Sauerstoff oder Schwefel bedeutet und R⁶ Hydroxyl, C₁-C₄-Alkyl wie vorstehend genannt, insbesondere Methyl und Ethyl, C₁-C₄-Alkoxy wie vorstehend genannt, insbesondere Methoxy und Ethoxy, C₁-C₄-Alkoxy-C₁-C₄-alkoxy wie Methoxymethoxy, Ethoxymethoxy, Ethoxyethoxy und tert.-Butoxyethoxy, Amino, C₁-C₄-Alkylamino wie Methylamino, Ethylamino, n-Propylamino, Isopropylamino, n-Butylamino und tert.-Butylamino, insbesondere Methylamino und Ethylamino, Di-(C₁-C₄-)-alkylamino wie Dimethylamino, Diethylamino, Methylethylamino und Methyl-n-butylamino, C₃-C₆-Cycloalkylamino wie Cyclopropylamino, Cyclobutylamino, Cyclopentylamino und Cyclohexylamino oder Phenylamino bedeutet, wobei der Aromat noch einen bis drei der folgenden Reste tragen kann:
      Halogen wie vorstehend genannt, insbesondere Fluor und Chlor, C₁-C₄-Alkyl wie vorstehend genannt, und/oder C₁-C₄-Alkoxy wie vorstehend genannt; insbesondere Phenylamino,
   bevorzugter Substituent R³ ist die Cyanogruppe;
R⁴
   - Wasserstoff;
   - eine C₁-C₄-Alkylgruppe wie vorstehend genannt, insbesondere Methyl und Ethyl;
   - eine C₂-C₄-Alkenylgruppe wie Ethenyl, Prop-2-en-1-yl, But-2-en-1-yl, But-3-en-1-yl und But-3-en-2-yl, insbesondere Ethenyl und Prop-2-en-1-yl;
   - die Phenylgruppe, die noch einen bis drei der folgenden Reste tragen kann: Nitro; Halogen wie vorstehend genannt, insbesondere Fluor und Chlor; C₁-C₄-Alkyl wie vorstehend genannt, insbesondere Methyl; partiell oder vollständig halogeniertes Alkyl wie vorstehend genannt, insbesondere Trifluormethyl; C₁-C₄-Alkoxy wie vorstehend genannt, insbesondere Methoxy; partiell oder vollständig halogeniertes C₁-C₄-Alkoxy wie vorstehend genannt, insbesondere Trifluormethoxy; C₁-C₄-Alkylthio wie vorstehend genannt, insbesondere Methylthio;
      und wobei die Phenylgruppe zusätzlich noch so viele Halogenatome tragen kann, daß die Gesamtzahl der Reste 4 oder 5 beträgt; besonders bevorzugt ist Phenyl;
   - einen über ein C-Atom gebundenen Triazinring, der an jedem der beiden anderen C-Atome noch ein Halogenatom wie vorstehend genannt, insbesondere Fluor, tragen kann;
   - eine Gruppe -PX(OR⁸)₂, wobei R⁸ Wasserstoff, C₁-C₄-Alkyl wie vorstehend genannt, insbesondere Methyl, oder Phenyl bedeutet, und wobei die Phenylgruppe noch einen bis drei der folgenden Reste tragen kann: C₁-C₄-Alkyl wie vorstehend genannt, insbesondere Methyl; partiell oder vollständig halogeniertes Alkyl wie vorstehend genannt; insbesondere Trifluormethyl; C₁-C₄-Alkoxy wie vorstehend genannt, insbesondere Methoxy; partiell oder vollständig halogeniertes C₁-C₄-Alkoxy wie vorstehend genannt, insbesondere Trifluormethoxy; C₁-C₄-Alkylthio wie vorstehend genannt, insbesondere Methylthio;
   - eine Gruppe -CX-R⁹, wobei
      - X: Sauerstoff oder Schwefel und
      - R⁹: verzweigtes oder unverzweigtes C₁-C₂₀-Alkyl, insbesondere C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
      partiell oder vollständig halogeniertes C₁-C₆-Alkyl, insbesondere C₁-C₂-Alkyl wie Chlormethyl, Dichlormethyl, Trichlormethyl, Chlorfluormethyl, Fluormethyl, Difluormethyl, Trifluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
      C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-Pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, insbesondere Prop-2-en-1-yl;
      partiell oder vollständig halogeniertes C₂-C₆-Alkenyl wie 2-Fluorethenyl, 2-Chlorethenyl, Trifluorethenyl, Trichlorethenyl und 2-Chlor-prop-2-en-1-yl;
      C₃-C₆-Cycloalkyl wie Cyclopropyl, Cyclobutyl, Cyclopentyl und Cyclohexyl, wobei diese Reste noch eine Hydroxycarbonylgruppe tragen können;
      C₅-C₆-Cycloalkenyl wie Cyclopent-1-en-1-yl, Cyclopent-2-en-1-yl, Cyclopent-3-en-1-yl, Cyclohex-1-en-1-yl, Cyclohex-2-en-1-yl und Cyclohex-3-en-1-yl, wobei diese Reste noch eine Hydroxycarbonylgruppe tragen können;
      Amino;
      C₁-C₄-Alkylamino wie Methylamino, Ethylamino, n-Propylamino, Isopropylamino, n-Butylamino und tert.-Butylamino, insbesondere Methylamino und Ethylamino;
      Di-(C₁-C₄)-alkylamino wie N,N-Dimethylamino, N,N-Diethylamino, N,N-Dipropylamino, N,N-Di-(1-methylethyl)-amino N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methyl-propyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropylamino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)-N-(2-methylpropyl)amino, N-(1,1-Dimethylethyl)-N-(1-methylpropyl)amino und N-(1-1-Dimethylethyl)-N-(2-methylpropyl)amino;
      C₃-C₈-Cycloalkylamino wie Cyclopropylamino, Cyclobutylamino, Cyclopentylamino, Cyclohexylamino, Cycloheptylamino und Cyclooctylamino, insbesondere Cyclopropylamino;
      Phenyl oder Phenylamino, wobei der Aromat seinerseits noch einen bis drei der folgenden Reste tragen kann: Nitro, Halogen wie vorstehend genannt, insbesondere Fluor und Chlor, C₁-C₄-Alkyl wie vorstehend genannt, partiell oder vollständig halogeniertes C₁-C₄-Alkyl wie vorstehend genannt, insbesondere Trifluormethyl und Trichlormethyl, C₁-C₄-Alkoxy wie vorstehend genannt, insbesondere Methoxy und Ethoxy, partiell oder vollständig halogeniertes C₁-C₄-Alkoxy wie vorstehend genannt, insbesondere Trifluormethoxy und Trichlormethoxy oder C₁-C₄-Alkylthio wie vorstehend genannt, insbesondere Methylthio, und wobei der Aromat jeweils noch so viele Halogenatome wie vorstehend genannt, insbesondere Fluor und Chlor, tragen kann, daß die Gesamtzahl der Reste 4 oder 5 beträgt;
   - eine Gruppe -SO₂R¹⁰,-SO₂-NHR¹⁰, wobei
      - R¹⁰: für C₁-C₄-Alkyl wie vorstehend genannt oder Phenyl, das noch einen bis drei der folgenden Reste tragen kann: Halogen wie vorstehend genannt, insbesondere Fluor und Chlor; Nitro; C₁-C₄-Alkyl wie vorstehend genannt; partiell oder vollständig halogeniertes C₁-C₄-Alkyl wie vorstehend genannt, insbesondere Trifluormethyl; C₁-C₄-Alkoxy wie vorstehend genannt, insbesondere Methoxy und Ethoxy; partiell oder vollständig halogeniertes C₁-C₄-Alkoxy wie vorstehend genannt, C₁-C₄-Alkylthio wie vorstehend genannt, insbesondere Methylthio, wobei die Phenylgruppe zusätzlich noch so viele Halogenatome wie vorstehend genannt, insbesondere Fluor und Chlor, tragen kann, daß die Gesamtzahl der Reste 4 oder 5 beträgt, steht;
   - eine Gruppe -CX-N(R⁷)SO₂-R¹¹, wobei
      - R¹¹: für Amino;
      C₁-C₄-Alkylamino wie vorstehend genannt, insbesondere Methylamino und Ethylamino;
      Di-(C₁-C₄)-alkylamino wie vorstehend genannt, insbesondere Dimethylamino und Diethylamino;
      Pyrrolidin-1-yl, Piperidin-1-yl, Morpholin-4-yl oder Piperazin-1-yl, wobei die Heterocyclen noch eine oder zwei C₁-C₄-Alkylgruppen wie vorstehend genannt, tragen können, steht,
      oder
      - R³ u. R⁴: zusammen eine Gruppe -CO-O-CO-;
R⁵
   - Wasserstoff;
   - eine C₁-C₄-Akylgruppe wie vorstehend genannt oder
   - eine Gruppe -CX-R⁹ oder -SO₂R¹⁰, wobei die Variablen die vorstehend angegebene Bedeutung haben;
   oder
   - R⁴ und R⁵: zusammen eine Gruppe =CR¹²R¹³ oder -CO-W-CO, wobei die Variablen die folgende Bedeutung haben:
   - R¹²: Wasserstoff; Amino; C₁-C₄-Alkylamino wie wie vorstehend genannt, insbesondere Methylamino und Ethylamino; C₃-C₈-Cycloalkylamino wie Cyclopropylamino, Cyclobutylamino, Cyclopentylamino, Cyclohexylamino, Cycloheptylamino und Cyclooctylamino, insbesondere Cyclopropylamino, Cyclopentylamino und Cyclohexylamino;
   - R¹³: Amino; C₁-C₄-Alkylamino wie vorstehend genannt, insbesondere Methylamino und Ethylamino; Di-(C₁-C₄)-Alkylamino wie vorstehend genannt, insbesondere Dimethylamino und Diethylamino; C₃-C₈-Cycloalkylamino wie vorstehend genannt, insbesondere Cyclopropylamino, Cyclopentylamino und Cyclohexylamino; Pyrrolidin-1-yl, Piperidin-1-yl, Morpholin-4-yl, Phenyl oder Pyridyl, wobei die beiden letztgenannten Substituenten noch einen bis drei der folgenden Reste tragen können: Halogen wie vorstehend genannt, insbesondere Fluor und Chlor, Nitro, C₁-C₄-Alkyl wie vorstehend genannt, insbesondere Methyl, Ethyl, Isopropyl und tert.-Butyl, partiell oder vollständig halogeniertes C₁-C₄-Alkyl wie vorstehend genannt, insbesondere Trifluormethyl, C₁-C₄-Alkoxy wie vorstehend genannt, insbesondere Methoxy und Ethoxy, partiell oder vollständig halogeniertes C₁-C₄-Alkoxy wie vorstehend genannt, insbesondere Trifluormethoxy und/oder C₁-C₄-Alkylthio wie vorstehend genannt, insbesondere Methylthio; besonders bevorzugt sind Phenyl, 4-Ethoxyphenyl und Pyrid-2-yl;
W
   - eine Ethylen- oder Ethenylenbrücke, eine 5- oder 6-gliedrige 1,2-C-verknüpfte aromatische oder heteroaromatische Brücke mit einem Stickstoff, Sauerstoff oder Schwefelatom als Heteroatom, wobei diese Brückenglieder an jedem substituierbaren C-Atom noch einen Rest tragen können, ausgewählt aus einer Gruppe von bis zu zwei der folgenden Reste: Halogen wie vorstehend genannt, insbesondere Fluor und Chlor, C₁-C₄-Alkyl wie vorstehend genannt, insbesondere Methyl und tert.-Butyl, partiell oder vollständig halogeniertes C₁-C₄-Alkyl wie vorstehend genannt, insbesondere Trifluormethyl, C₁-C₄-Alkoxy wie vorstehend genannt, insbesondere Methoxy, partiell oder vollständig halogeniertes C₁-C₄-Alkoxy wie vorstehend genannt, insbesondere Trifluormethoxy, C₁-C₄-Alkylthio wie vorstehend genannt, insbesondere Methylthio und/oder partiell oder vollständig halogeniertes C₁-C₄-Alkylthio wie Fluormethylthio, Chlormethylthio, Brommethylthio, Trichlormethylthio, Trifluormethylthio und Pentafluorethylthio, insbesondere Trifluormethylthio;
      und wobei die Brückenglieder noch so viele Halogenatome wie vorstehend genannt, insbesondere Fluor und Chlor, tragen können, wie weitere substituierbare C-Atome am Aromat vorhanden sind; besonders bevorzugt sind 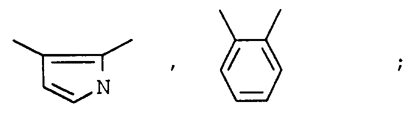
   - eine 5- oder 6-gliedrige 1,2-C-verknüpfte Cycloalkylen- oder Cycloalkenylenbrücke wie Cyclopentyliden, Cyclohexyliden, Cyclopentenyliden und Cyclohexenyliden, wobei diese Substituenten noch ein bis vier Halogenatome wie vorstehend genannt, insbesondere Fluor und Chlor und/oder C₁-C₄-Alkylgruppen wie vorstehend genannt, insbesondere Methylgruppen, tragen können; besonders bevorzugt sind

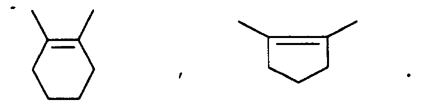

Die substituierten 2-Aminothiophene der Formel I sind auf verschiedene Weise erhältlich, und zwar vorzugsweise nach einem der folgenden Verfahren:
a) Kondensation von Ketonen XI mit Schwefel und einem Acetonitrilderivat XII zu substituierten 2-Aminothiophenen Ia: 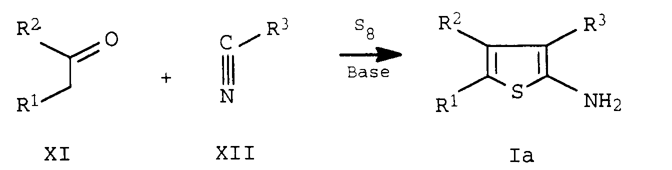
   Die Umsetzung erfolgt im allgemeinen in an sich bekannter Weise [vgl. K. Gewald et al., Chem. Ber. 99, 94 (1966) und E.F. Elslager et al., J. Heterocycl. Chem. 9, 775 (1972)] in einem inerten Lösungs- oder Verdünnungsmittel, beispielsweise in einem Alkohol wie Methanol, Ethanol, n-Propanol und Isopropanol, in einem Ether wie Dioxan und Tetrahydropyran, in einem Nitril wie Acetonitril und Propionitril in einem aprotischen Lösungsmittel wie Dimethylformamid, Dimethylsulfoxid und N-Methylpyrrolidon oder in einem Überschuß des Ketons XI.
   Die Reaktion kann auch unter Verwendung eines Phasentransferkatalysators wie Trioctylpropylammoniumchlorid und Cetyltrimethylammoniumchlorid in einem 2-Phasensystem aus Wasser und einem Kohlenwasserstoff, z.B. Tetrachlorkohlenstoff, durchgeführt werden [vgl. Synthesis, 867 (1974)].
   Vorzugsweise führt man die Umsetzung in Gegenwart einer anorganischen oder organischen Base durch.
   Als Basen eignen sich beispielsweise Alkalimetallhydroxide wie Natrium- und Kaliumhydroxid, Erdalkalimetallhydroxide wie Calciumhydroxid, Alkalimetallalkoholate wie Natriummethanolat, Natrium- und Kaliumethanolat und Kalium-tert butylat, Erdalkalimetallalkoholate wie Calciumalkoholat, Alkalimetallhydride wie Natrium- und Kaliumhydrid, Erdalkalimetallhydride wie Calciumhydrid, aliphatische Amine wie Dimethylamin, Triethylamin, Diisopropylamin, Dimethylanilin, Dimethylbenzylamin und Piperidin sowie heteroaromatische Amine wie Pyridin und 4-Dimethylaminopyridin.
   Im Falle der Amine kann auch lösungsmittelfrei in einem Überschuß an Base gearbeitet werden.
   Zweckmäßigerweise werden alle Reaktionspartner in stöchiometrischen Mengen eingesetzt, jedoch kann auch ein Überschuß der einen oder anderen Komponente, bis etwa 10 mol-%, verwendet werden.
   Verwendet man die Base als Lösungsmittel, so liegt sie in einem größeren Überschuß vor.
   Im allgemeinen liegt die Reaktionstemperatur zwischen 0 und 200°C, bevorzugt zwischen 20 und 140°C, insbesondere bei der Siedetemperatur des jeweiligen Lösungsmittels.
   Normalerweise arbeitet man bei Atmosphärendruck oder unter dem Eigendruck des verwendeten Lösungsmittels.
   Weitere Synthesemöglichkeiten bestehen in der Umsetzung von
   - α-Mercaptoketonen mit methylenaktiven Acetonitrilderivaten XII [vgl. K. Gewald, Chem. Ber. 98, 3571 (1965)).
b) Umsetzung von substituierten 2-Aminothiophenen Ia mit einer elektrophilen Verbindung XIII bis XVIII: 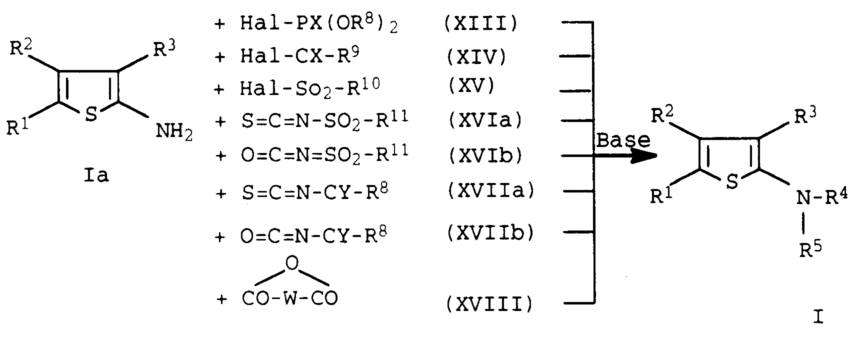
   Ha1 bedeutet Chlor oder Brom.

Die Reaktionen erfolgen normalerweise nach an sich bekannten Verfahren in einem inerten Lösungs- oder Verdünnungsmittel in Gegenwart einer Base:
- zur Umsetzung von Ia mit Carbonsäurehalogeniden und -anhydriden zu Amiden bzw. Imiden vgl. Henecka in Houben-Weyl, Methoden der Organischen Chemie, Band 8, S. 653-713;
- zur Umsetzung von Ia mit Sulfonsäurechloriden zu Sulfonamiden vgl. Mutz in Houben-Weyl, Methoden der Organischen Chemie, Band 9, S. 599-658;
- zur Umsetzung von Ia mit Iso(thio)-cyanaten zu (Thio-)Harnstoffen vgl. Petersen in: Houben-Weyl, Methoden der Organischen Chemie, Band 8 S. 129-136;
- zur Umsetzung von Ia mit aromatischen Aldehyden zu Schiff'schen Basen vgl. Freytag in Houben-Weyl, Band 11/2, S. 73-98.

Als Lösungsmittel kommen beispielsweise aliphatische Kohlenwasserstoffe wie n-Hexan, Benzin und Petrolether, aromatische Kohlenwasserstoffe wie Benzol, Toluol, o-, m-, p-Xylol, chlorierte Kohlenwasserstoffe wie Dichlormethan, Chloroform, Tetrachlormethan, 1,2-Dichlorethan und Chlorbenzol, stickstoffhaltige Heteroaromaten wie Pyridin und Chinolin, cyclische Ether wie Tetrahydrofuran und Dioxan, Nitrile wie Acetonitril und Propionitril sowie Dimethylformamid, Dimethylsulfoxid und N-Methylpyrrolidon oder eine Mischung der genannten Solventien in Betracht. Bei Anwesenheit eines Phasentransferkatalysators kann die Reaktion auch in einem 2-Phasensystem aus Wasser und einem Kohlenwasserstoff, z.B. Tetrachlorkohlenstoff, durchgeführt werden.

Bezüglich der verwendbaren Basen, Phasentransferkatalysatoren, der Mengenverhältnisse und des Druckes gelten die Angaben für Methode (a).

Im allgemeinen liegt die Reaktionstemperatur zwischen O und 200°C, bevorzugt zwischen 20 und 140°C, insbesondere bei der Siedetemperatur des jeweiligen Lösungsmittels.

Die elektrophilen Verbindungen XIII bis XVIII sind bekannt oder nach bekannten Verfahren erhältlich (vgl. Sustmann in Houben-Weyl: "Methoden der Organischen Chemie", Band E5, Seiten 590-608 und 634-652). Die Edukte XVIa und XVIb stellt man zweckmäßigerweise in situ aus Halogensulfonylisocyanaten und reaktiven Verbindungen R¹¹-H dar, die Verbindungen XVIIa und XVIIb analog aus Isocyanat-Salzen wie Ammoniumisocyanat und Verbindungen Hal-CY-R⁸.

Die substituierten 2-Aminothiophene I eignen sich als Antidots, um herbizide Wirkstoffe für Kulturpflanzen wie Kulturhirse, Reis, Mais, Getreidearten (Weizen, Roggen, Gerste, Hafer),Baumwolle, Zuckerrüben, Zuckerrohr und Soja vertraglicher zumachen. Sie wirken antagonistisch auf Herbizide verschiedenster Stoffklassen wie Triazine, Phenylharnstoffderivate, Carbamate, Thiolcarbamate, Halogenacetanilide, Benzoesäurederivate sowie insbesondere Halogenphenoxyessigsäureester, substituierte Phenoxyphenoxyessigsäureester, Phenoxyphenoxypropionsäureester und Cyclohexenonderivate.

Herbizide Wirkstoffe aus der Gruppe der Cyclohexenonderivaten der Formel III, 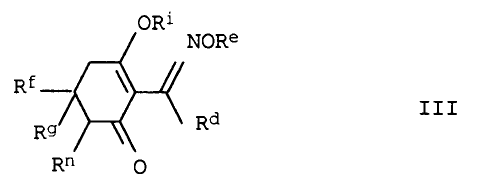 in welcher die Substituenten folgende Bedeutung haben:
- R^{d}: eine C₁-C₄-Alkylgruppe, die Methyl, Ethyl, n-Propyl, Isopropyl, n-Butyl und tert.-Butyl, vorzugsweise Ethyl und n-Propyl;
- R^{e}: eine C₁-C₄-Alkylgruppe wie Methyl, Ethyl, n-Propyl, Isopropyl, n-Butyl und tert.-Butyl, vorzugsweise Ethyl und n-Propyl; eine C₃-C₄-Alkenylgruppe, vorzugsweise Prop-2-enyl; eine C₃-C₄-Alkinylgruppe oder eine C₃-C₄-Halogenalkenylgruppe, vorzugsweise 3-Chlor-prop-2-en-1-yl;
eine C₁-C₄-Alkylen- oder C₂-C₄-Alkenylenkette, die beide noch ein bis drei C₁-C₃-Alkylreste und/oder Halogenatome tragen können, oder eine gewünschtenfalls durch C₁-C₃-Alkyl substituierte 3 bis 6-gliedrige Alkylen- oder 4- bis 6-gliedrige Alkenylenkette, die jeweils ein dem Oximetherteil nicht direkt benachbartes Sauerstoff- oder Schwefelatom als Kettenglied enthält, wobei alle vorstehend genannten Ketten endständig den Phenylring tragen, der seinerseits durch ein bis drei Reste substituiert sein kann, ausgewählt aus einer Gruppe bestehend aus einem Benzyloxycarbonyl- oder Phenylrest und jeweils einem bis drei der folgenden Reste: Nitro, Cyano, Halogen, C₁-C₄-Alkyl, C₁-C₄-Alkoxy, C₁-C₄-Alkylthio, partiell oder vollständig halogeniertes C₁-C₄-Alkyl, partiell oder vollständig halogeniertes C₁-C₄-Alkoxy, Carboxyl und C₁-C₄-Alkoxycarbonyl, und wobei der Phenylring noch zusätzlich so viele Halogenatome tragen kann, daß die Gesamtzahl der Reste 4 oder 5 beträgt; besonders bevorzugt sind 4-(p-Fluorphenyl)-but-3-enyl, 4-(p-Chlorphenyl)-but-3-enyl und 2-(p-Chlorphenoxy)-propyl;
die Thienylgruppe, die ein Halogenatom tragen kann;
- R^{f}: eine C₁-C₄-Alkylgruppe wie für R^{d} genannt, welche ein- oder zweifach durch C₁-C₄-Alkylthio oder C₁-C₄-Alkoxy substituiert sein kann;
ein 5- oder 6-gliedriges gesättigtes oder einfach ungesättigtes Ringsystem, welches neben Kohlenstoffgliedern ein Sauerstoff-, ein Schwefelatom oder eine Sulfoxid- oder Sulfongruppe enthalten kann, vorzugsweise Tetrahydropyranyl, Dihydropyranyl und Tetrahydrothiopyranyl, wobei das Ringsystem noch einen bis drei Reste tragen kann, ausgewählt aus einer Gruppe bestehend aus Hydroxyl, Halogen, C₁-C₄-Alkyl, C₁-C₄-Halogenalkyl, C₁-C₄-Alkoxy und C₁-C₄-Alkylthio;
einen 10-gliedrigen gesättigten oder einfach ungesättigten Heterocyclus, welcher zwei nicht benachbarte Sauerstoffatome oder Schwefelatome enthält und der durch bis zu drei C₁-C₄-Alkylgruppen und/oder Methoxygruppen substituiert sein kann;
die Phenylgruppe, eine Pyridylgruppe, eine Thiazolylgruppe, eine Pyrazolylgruppe, eine Pyrrolylgruppe oder eine Isoxazolylgruppe, wobei jede dieser Gruppen gewünschtenfallS ein bis drei Reste tragen kann, ausgewählt aus einer Gruppe bestehend aus: C₁-C₄-Alkyl, C₁-C₄-Halogenalkyl, C₁-C₄-Alkoxy, C₁-C₄-Alkylthio, C₃-C₆-Alkenyloxy, C₃-C₆-Alkinyloxy, C₁-C₄-Alkoxy-C₁-C₃-alkyl, C₁-C₄-Dialkoxy-C₁-C₃-alkyl, Formyl, Halogen und Benzoylamino;
- R^{g}: Wasserstoff, Hydroxy oder, wenn R^{f} die Bedeutung C₁-C₆-Alkylgruppe hat, eine C₁-C₆-Alkylgruppe, vorzugsweise Wasserstoff;
- R^{h}: Wasserstoff, die Cyanogruppe, ein Halogenatom, eine C₁-C₄-Alkoxycarbonylgruppe oder eine C₁-C₄-Alkylketoximgruppe, vorzugsweise Wasserstoff;
- Rⁱ: Wasserstoff oder das Äquivalent eines landwirtschaftlich brauchbaren Kations;
sind in der Literatur (z.B. EP-A 228 598, EP-A 230 235, EP-A 238 021, EP-A 368 227, US-A 4 432 786, DE-A 24 39 104, DE-A 40 14 986 und DE-A 40 33 423) als Herbizide beschrieben und dienen vorwiegend zur Bekämpfung unerwünschter Gräser in dicotylen Kulturen und in Gräsern, die nicht zur Familie der Gramineen zählen. Je nach Struktur der Substituenten und Dosis der Anwendung können Verbindungen aus dieser Gruppe auch zur selektiven Bekämpfung von unerwünschten Gräsern in Gramineen-Kulturen wie Weizen und Reis eingesetzt werden.

Cyclohexenonderivate der Formel III, in denen R^{e} für einen unsubstituierten oder substituierten Alkyl- oder Alkenyl-, z.B Butyl- oder Butenylphenylrest steht, können in an sich bekannter Weise aus schon bekannten Derivaten der Formel IV (EP-A-80 301, EP-A-125 094, EP-A 142 741, US-A 4 249 937, EP-A 137 174 und EP-A 177 913) und den entsprechenden Hydroxylaminen der Formel V (Houben-Weyl, 10/1, S. 1181 ff) hergestellt werden (EP-A 169 521).

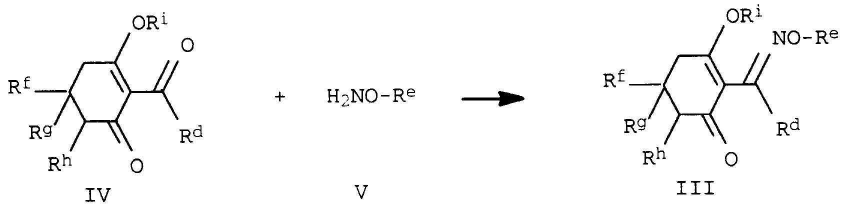

Zweckmäßig führt man die Umsetzung in heterogener Phase in einem Lösungsmittel, bei einer ausreichenden Temperatur unterhalb von etwa 80°C, in Gegenwart einer Base durch und verwendet das Hydroxylamin V in Form seines Ammoniumsalzes.

Geeignete Basen sind z. B. Carbonate, Hydrogencarbonate, Acetate, Alkoholate oder Oxide von Alkali- oder Erdalkalimetallen, insbesondere Natriumhydroxid, Kaliumhydroxid, Magnesiumoxid, Calciumoxid. Außerdem können organische Basen wie Pyridin oder tertiäre Amine Verwendung finden. Die Base wird beispielsweise in einer Menge von 0,5 bis 2 Mol-Äquivalent bezogen auf die Ammoniumverbindung zugesetzt.

Als Lösungsmittel eignen sich beispielsweise Dimethylsulfoxid, Alkohole wie Methanol, Ethanol und Isopropanol, aromatische Kohlenwasserstoffe wie Benzol und Toluol, chlorierte Kohlenwasserstoffe wie Chloroform und Dichlorethan, aliphatische Kohlenwasserstoffe wie Hexan und Cyclohexan, Ester wie Essigsäureethylester sowie Ether wie Diethylether, Dioxan und Tetrahydrofuran. Vorzugsweise führt man die Umsetzung in Methanol mit Natriumhydrogencarbonat als Base durch.

Die Reaktion ist nach wenigen Stunden beendet. Das Produkt kann z.B. durch Einengen der Mischung, Verteilung des Rückstandes in Methylenchlorid/ Wasser und Abdestillieren des Lösungsmittels unter vermindertem Druck isoliert werden.

Man kann für diese Umsetzung aber auch unmittelbar die freie Hydroxylaminbasen z.B. in Form einer wäßrigen Lösung, verwenden; je nach verwendetem Lösungsmittel für die Verbindung IV erhält man ein ein- oder zweiphasiges Reaktionsgemisch.

Geeignete Lösungsmittel für diese Variante sind beispielsweise Alkohole wie Methanol, Ethanol, Isopropanol und Cyclohexanol, aliphatische und aromatische, gegebenenfalls chlorierte Kohlenwasserstoffe wie Hexan, Cyclohexan, Methylenchlorid, Toluol und Dichlorethan, Ester wie Essigsäureethylester, Nitrile wie Acetonitril sowie cyclische Ether wie Dioxan und Tetrahydrofuran.

Alkalimetallsalze der Verbindungen III können durch Behandeln der 3-Hydroxyverbindungen mit Natrium- oder Kaliumhydroxid bzw. -alkoholat in wäßriger Lösung oder in einem organischen Lösungsmittel wie Methanol, Ethanol, Aceton und Toluol erhalten werden. Andere Metallsalze wie Mangan-, Kupfer-, Zink-, Eisen-, Calcium-, Magnesium und Bariumsalze können aus den Natriumsalzen in üblicher Weise hergestellt werden, ebenso Ammonium- und Phosphoniumsalze mittels Ammoniak, Ammonium-, Phosphonium-, Sulfonium- oder Sulfoxoniumhydroxiden.

Die Verbindungen des Typs IV können z.B. aus den entsprechenden Cyclohexan-1,3-dionen der Formel VI 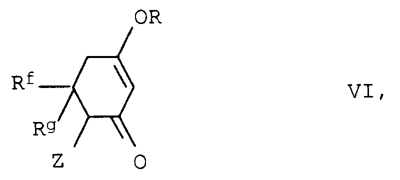 in der
- Z: Wasserstoff oder Methoxycarbonyl bedeutet und
- R^{g}: Wasserstoff bedeutet
nach bekannten Methoden (Tetrahedron Lett., 2491 (1975)) hergestellt werden.

Es ist auch möglich, die Verbindungen der Formel IV über die Zwischenstufe der Enolester herzustellen, die bei der Umsetzung von Verbindungen der Formel VI mit Säurechloriden in Gegenwart von Basen anfallen und anschließend mit bestimmten Imidazol- oder Pyridinderivaten umgelagert werden (JP-OS 79/063 052).

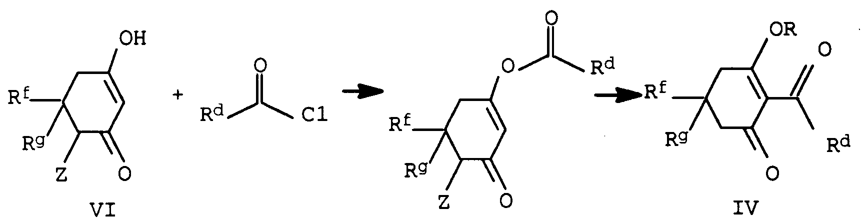

Zu den Verbindungen der Formel VI gelangt man über eine Reihe bekannter Verfahrensschritte ausgehend von bekannten Vorprodukten.

Die Synthese der Hydroxylamine V, in denen R^{e} für einen ggf. substituierten Phenylbutylrest steht, erfolgt gemäß dem nachstehenden Reaktionsschema beispielsweise über
- α): die Alkylierung von cyclischen Hydroxyimiden VII mit geeigneten Phenylbutylhalogeniden und anschließende Schutzgruppenabspaltung beispielsweise mit Hydrazin oder Ethanolamin analog Beispielen aus EP-A-244 786 bzw. Houben-Weyl, Methoden der organischen Chemie, Band X/l, Seite 1152ff.
- β): Hydrierung von N-4-Phenylbutenyloxyphthalimiden, deren Herstellung in DE-A 38 38 310 beschrieben ist, mittels geeigneten Katalysatoren wie z.B. Palladium auf Aktivkohle, in geeigneten inerten Lösungsmitteln wie z.B. Methanol, Tetrahydrofuran, Dioxan und anschließende Schutzgruppenabspaltung wie voranstehend beschrieben.
Vorteilhaft wird die Hydrierung bei Temperaturen von 20°C bis zum Siedepunkt des Lösungsmittels insbesondere bei Raumtemperatur nach den dafür üblichen Techniken bei Atmosphärendruck, Über- oder Unterdruck durchgeführt. Bevorzugt ist ein Druckbereich von 1 bis 10, insbesondere 1 bis 2 bar.

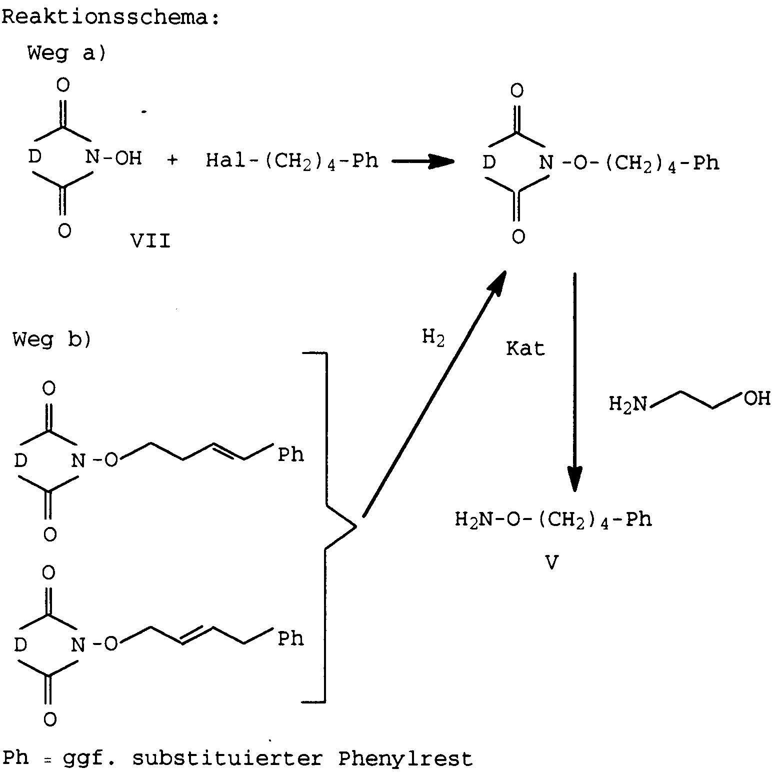

Als cyclische Hydroxyimide VII kommen beispielsweise folgende Substanzen in Betracht:

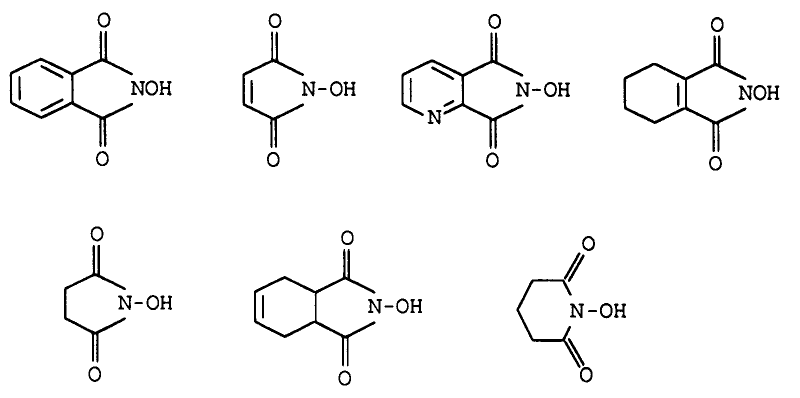

Die Synthese der Hydroxylamine V, in denen R^{e} für einen unsubstituierten oder substituierten Butenylphenylrest steht, wobei der nachstehend mit Ph abgekürzte Phenylrest seinerseits substituiert oder unsubstituiert sein kann, erfolgt gemäß dem nachstehenden Reaktionsschema ausgehend von Anilinderivaten durch Diazotierung und anschließende Kupplung des Diazoniumsalzes mit einem entsprechend substituierten Butadien VIII. Das so erhaltene Gemisch aus IXa und IXb wird mit einem cyclischen Hydroxyimid XI gekoppelt und das erhaltene geschützte Hydroxylaminderivat X mit 2-Aminoethanol zum freien Hydroxylamin V gespalten:

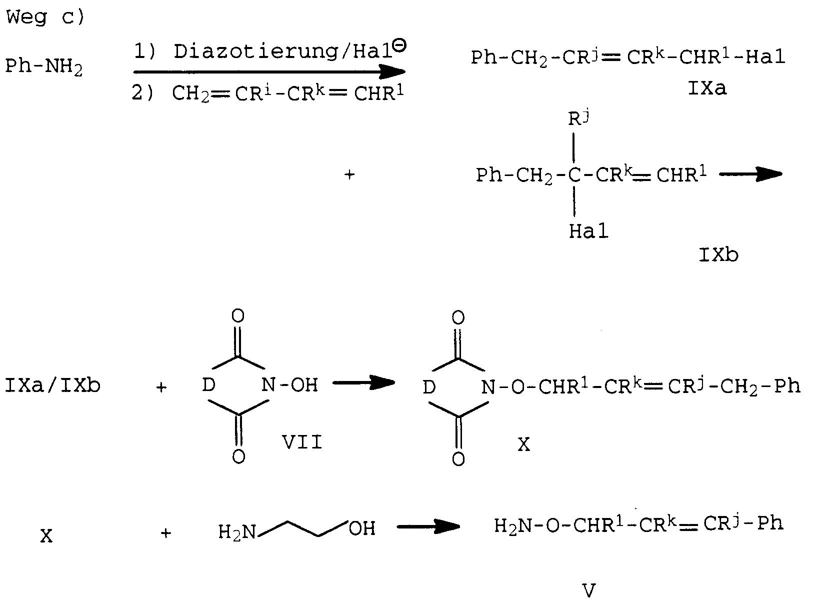

Die Reste R^{j}, R^{k} und R¹ bedeuten hierbei unabhängig voneinander Wasserstoff, C₁-C₃-Alkylgruppen und/oder Halogenatome. Hal bedeutet Halogenatom, vorzugsweise Chloratom.

Die zur obigen Synthese der Hydroxylamine der Formel V benötigten Halogenide IXa lassen sich nach literaturbekannten Verfahren im Gemisch mit IXb herstellen, beispielsweise durch die Umsetzung von Diazoniumsalzen aromatischer und heteroaromatischer Aniline mit Dienen. Die Anwendungsbreite der Reaktion ist in Organic Reactions 11 (1960) 189 bzw. 24 (1976) 225 abgehandelt.

Bei der Kopplung der isomeren Halogenide IXa und IXb an ein cyclisches Hydroxyimid der Formel VII entstehen ausschließlich die cyclischen Imidether der Formel X, die nach Abspaltung der Schutzgruppe am Stickstoff die Hydroxylamine V liefern.

Die Umsetzung mit einem Hydroxyimid VII (Weg a und c) erfolgt in Gegenwart eines säurebindenden Mittels und eines Lösungsmittels. Aus Kostengründen kommt als Hydroxyimid VII bevorzugt Hydroxyphthalimid zum Einsatz.

Als säurebindende Mittel eignen sich Alkalimetallcarbonate wie Kalium- oder Natriumcarbonat, Alkalimetallhydrogencarbonate wie Kalium- und Natriumhydrogencarbonat, tertiäre Amine wie Trimethyl- und Triethylamin und basische Heterocyclen wie Pyridin. Aus Kostengründen kommen bevorzugt Kalium- und Natriumcarbonat in Betracht.

Als Lösungsmittel eignen sich aprotisch dipolare organische Lösungsmittel wie z.B. Dimethylformamid, Dimethylsulfoxid und/oder Sulfolan.

Ferner ist auch eine Alkylierung unter Phasentransferbedingungen möglich. Als organische Lösungsmittel werden hierbei mit Wasser nicht mischbare Verbindungen wie Kohlenwasserstoffe oder Chlorkohlenwasserstoffe eingesetzt. Als Phasentransferkatalysatoren eignen sich quartäre Ammonium- und Phosphoniumsalze.

Die Spaltung der cyclischen Imidether X gelingt analog einem in EP-A 244 786 beschriebenen Verfahren mit Alkanolaminen. Die Hydroxylamine V können nach diesem Verfahren als freie Basen oder nach Fällung mit Säuren als Salze isoliert werden. Gut kristallisierende Salze erhält man durch Umsetzung der Basen mit Oxalsäure.

Spezielle Beispiele für herbizide Cyclohexenone der Formel III, deren Kulturpflanzenverträglichkeit durch substituierte 2-Aminothiophene I verbessert werden kann, sind in den folgenden Tabellen 1 bis 12 aufgeführt.

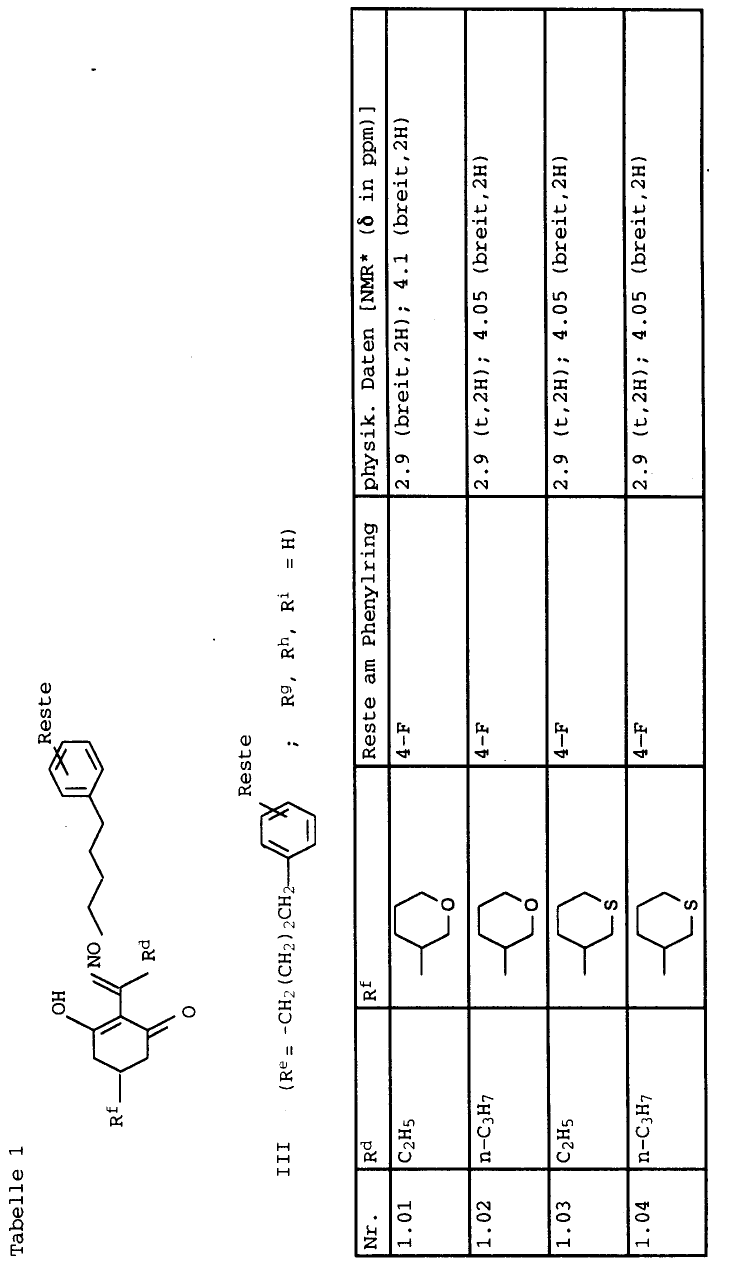

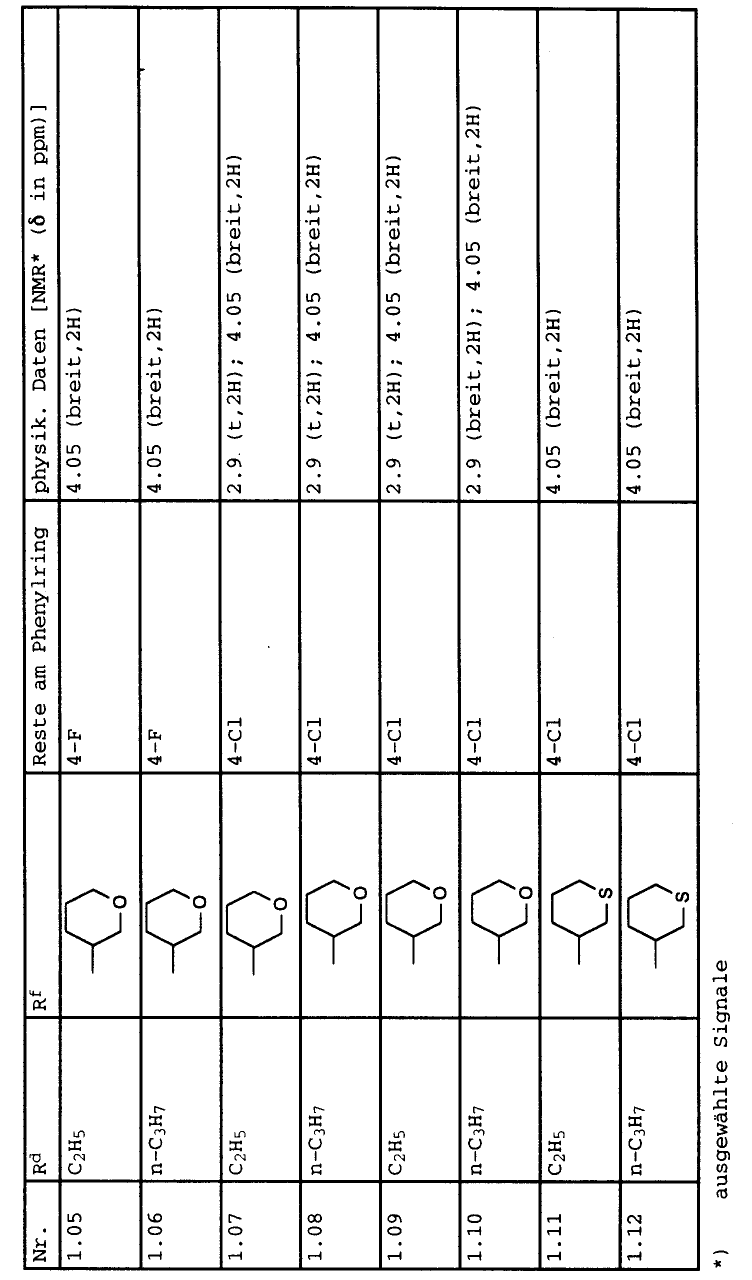

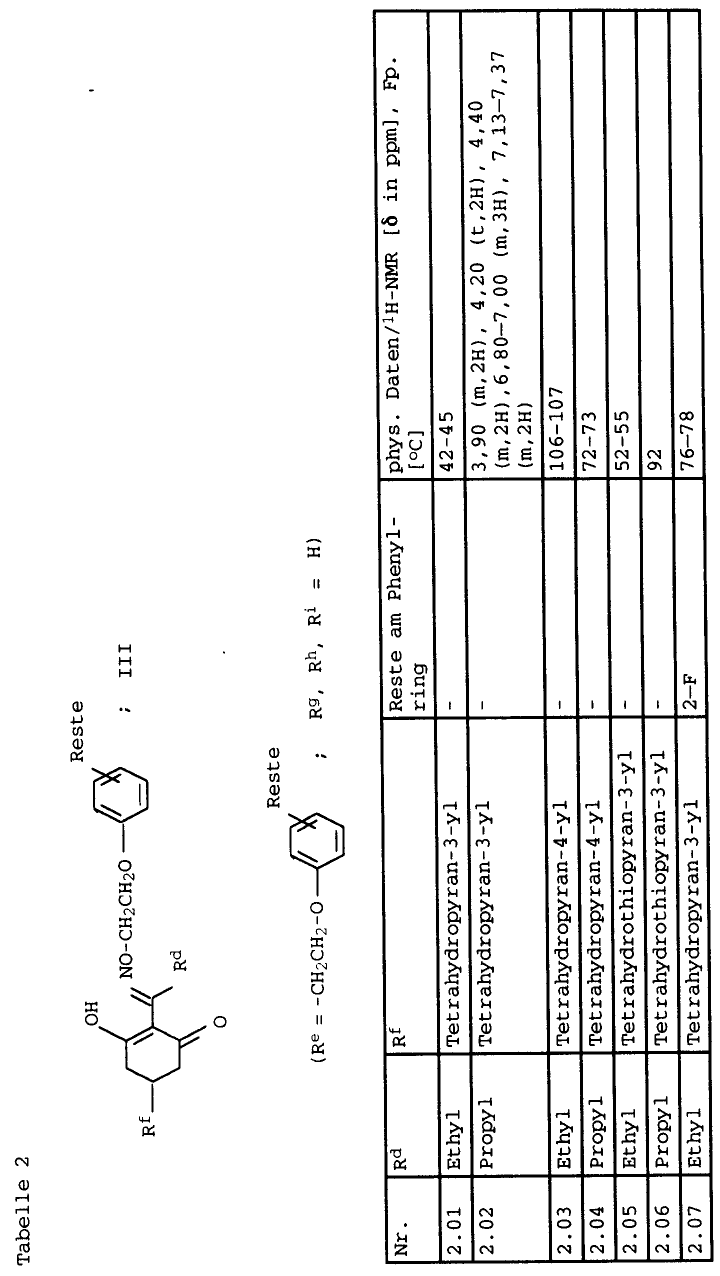

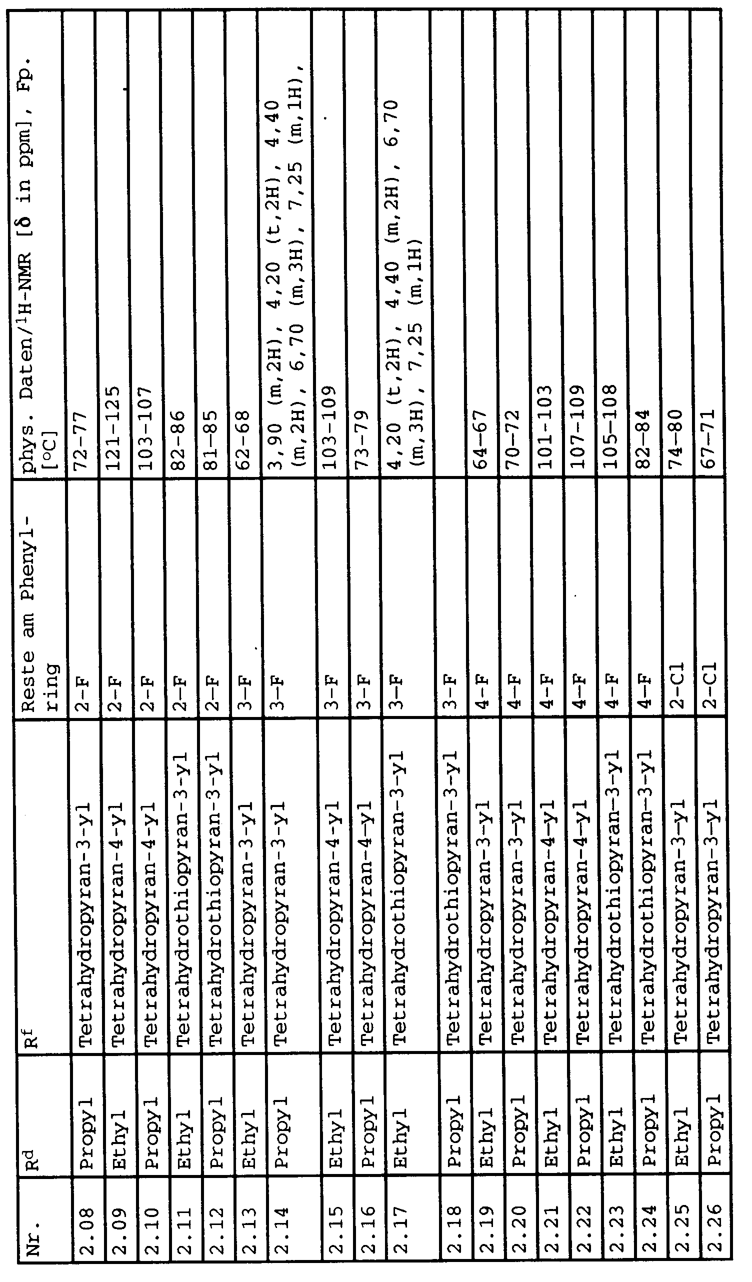

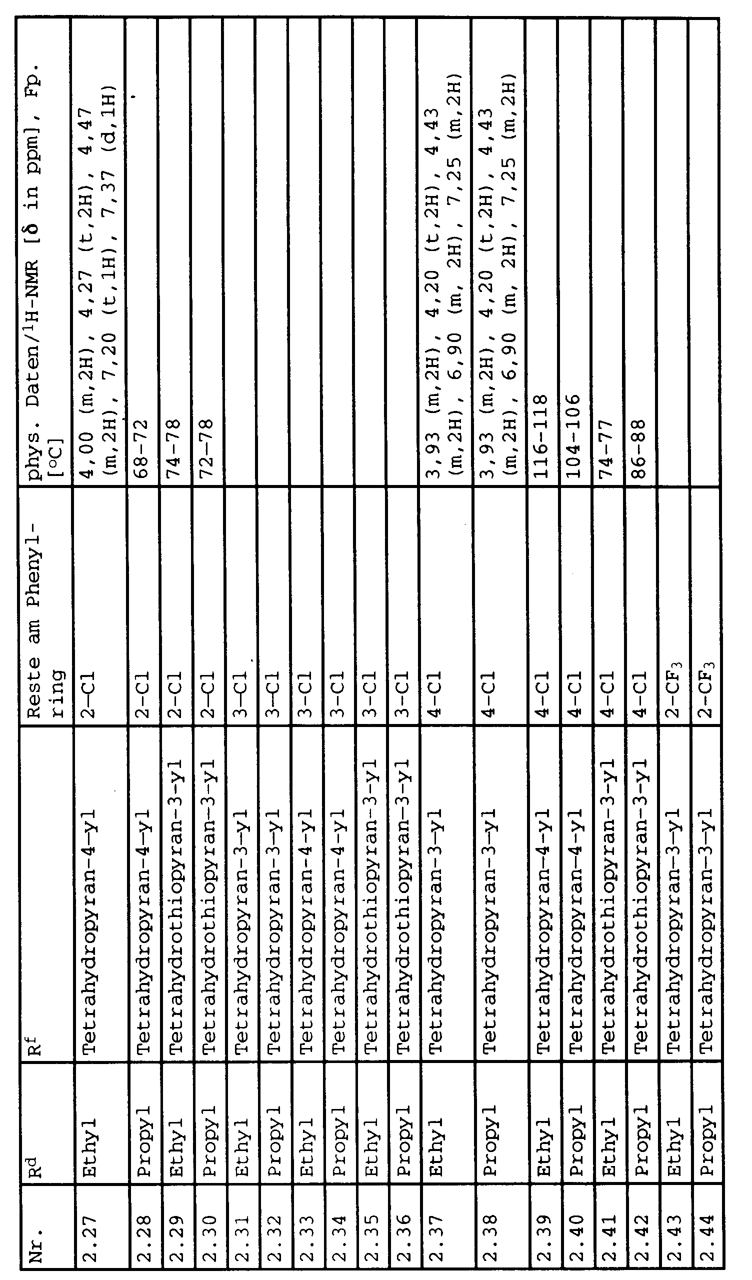

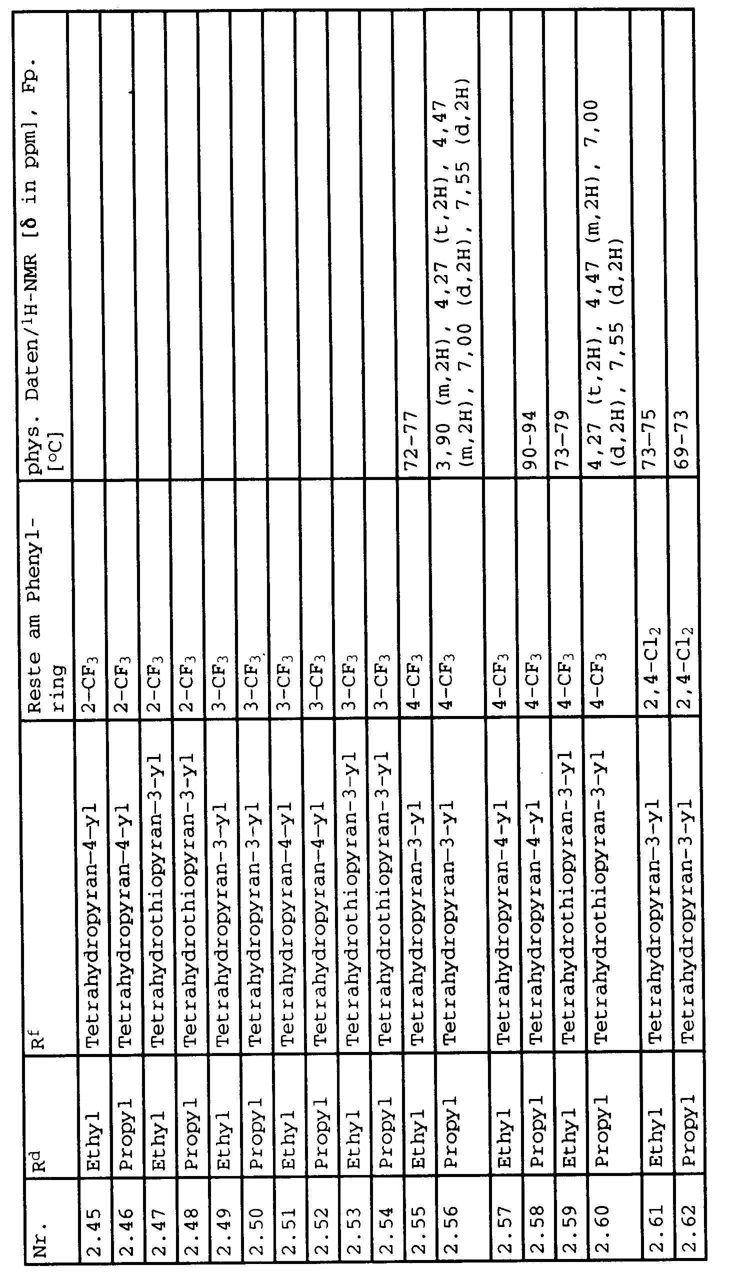

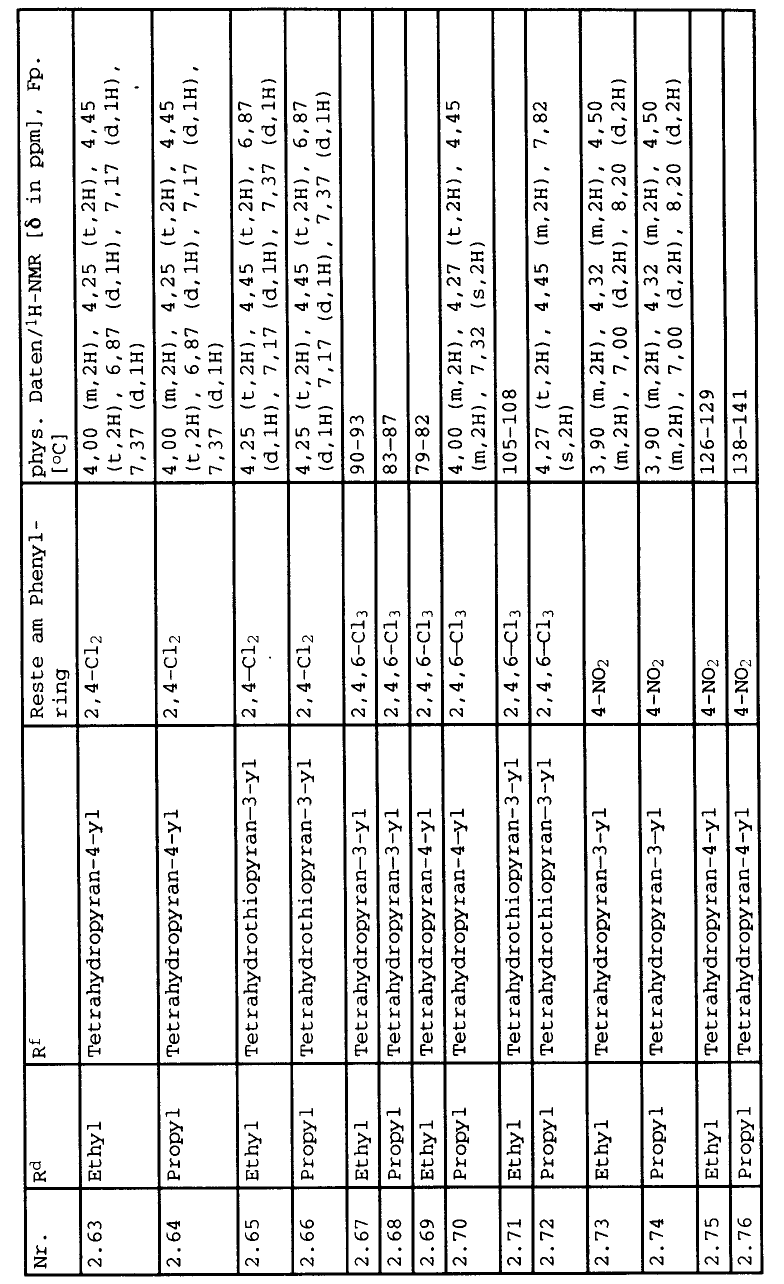

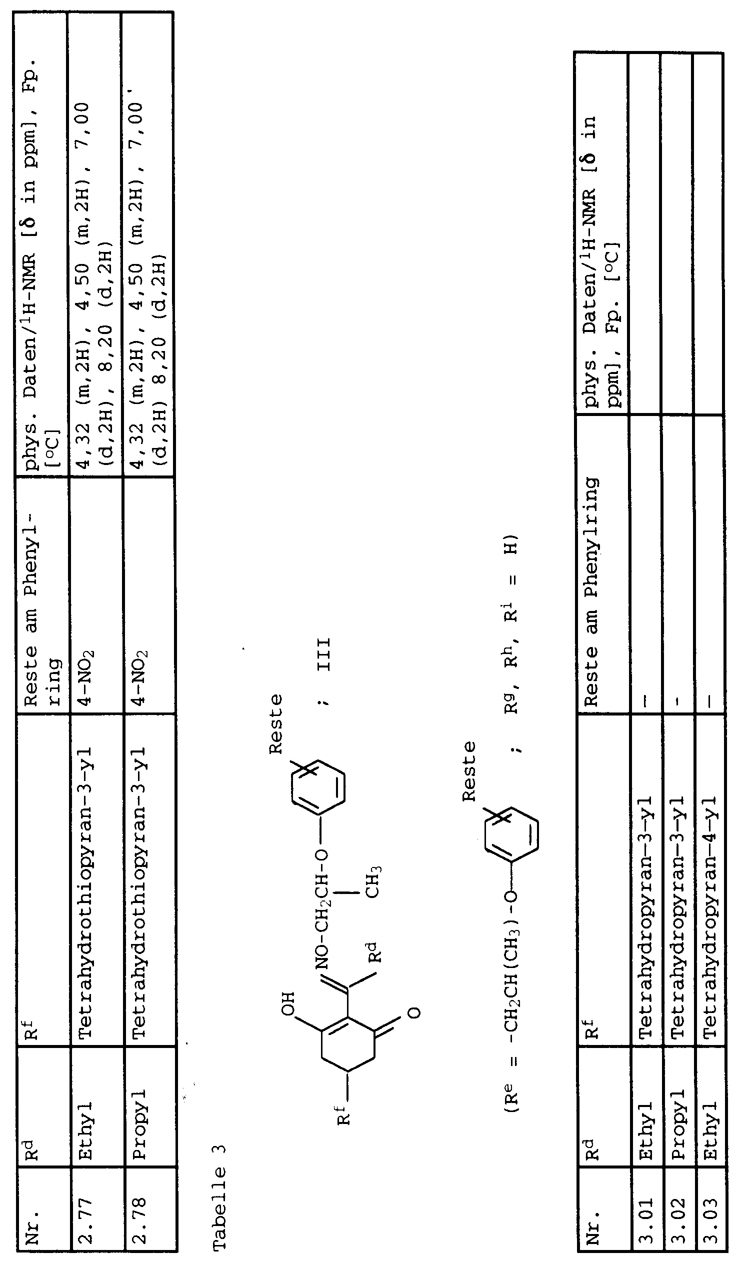

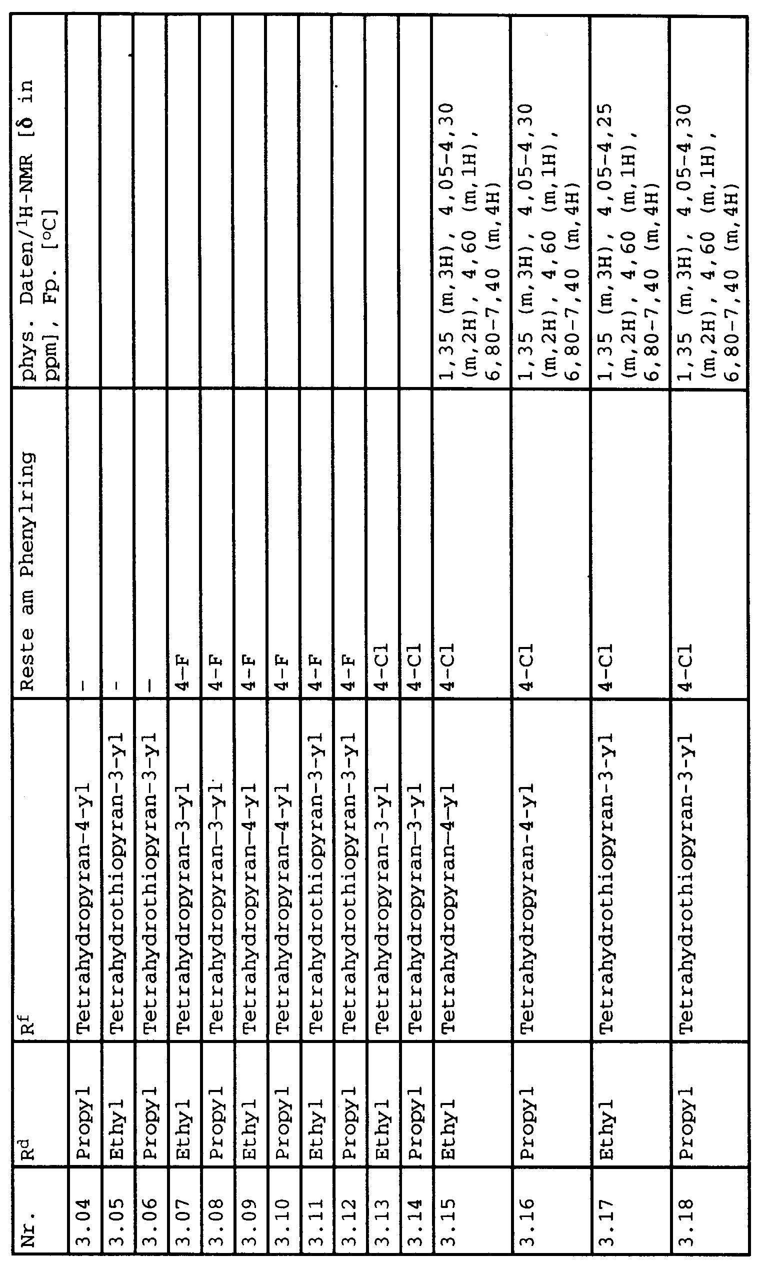

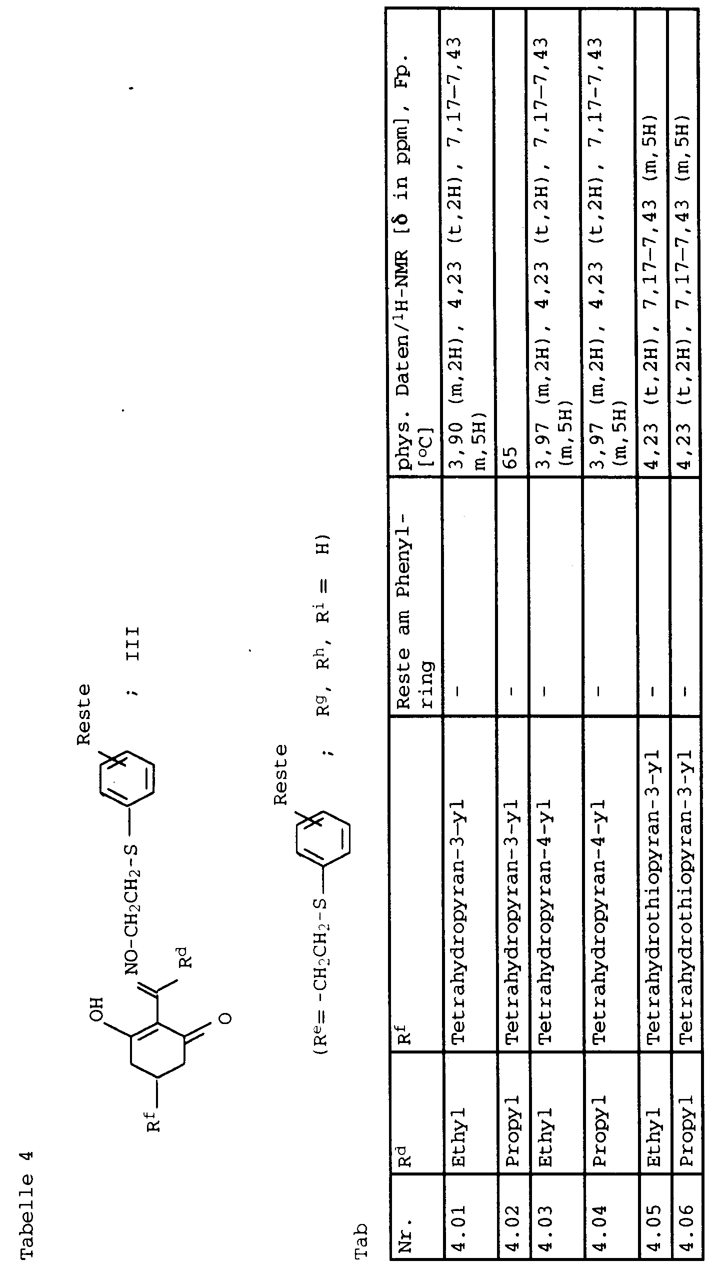

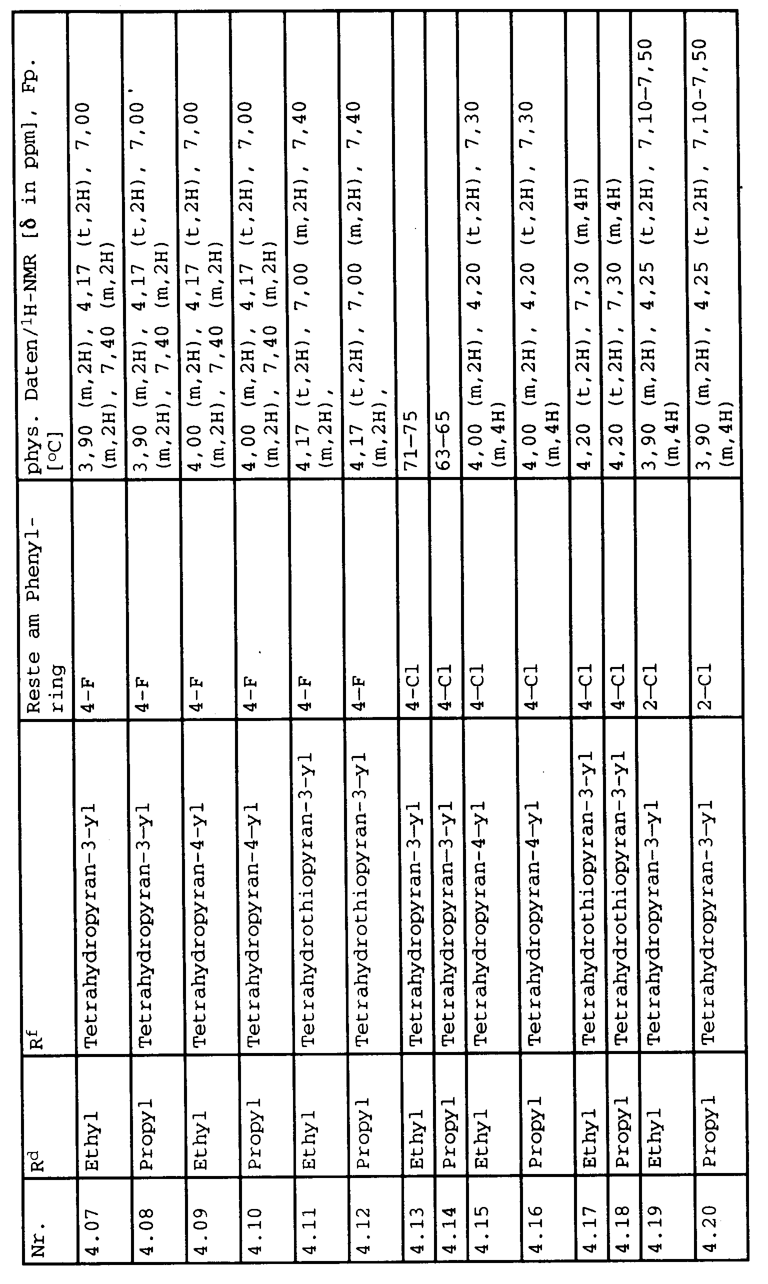

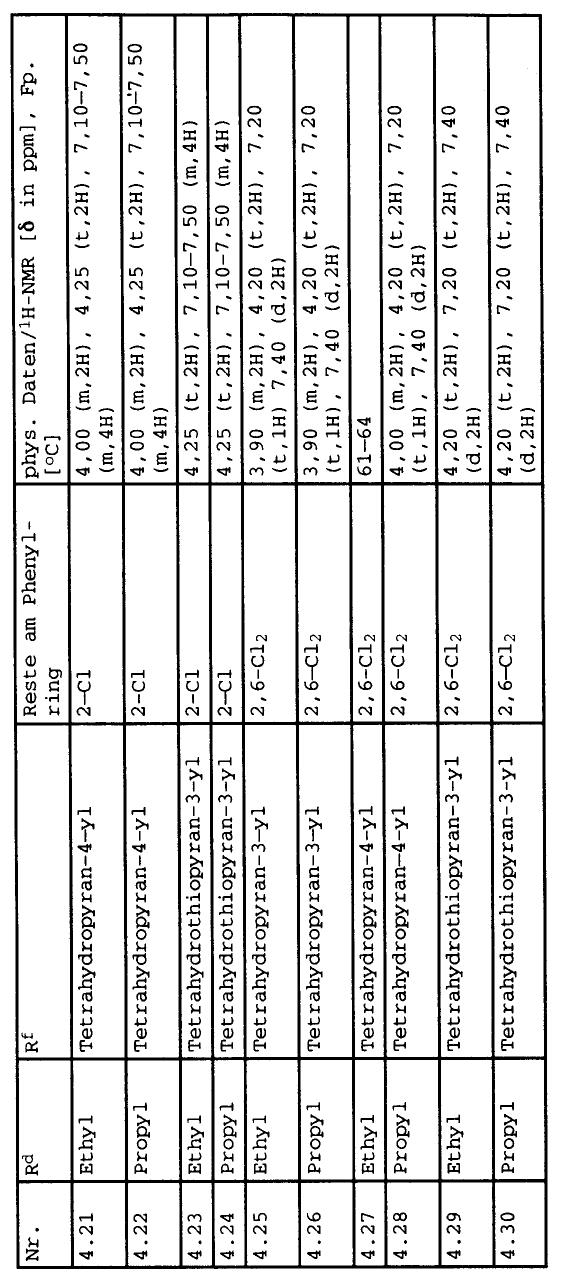

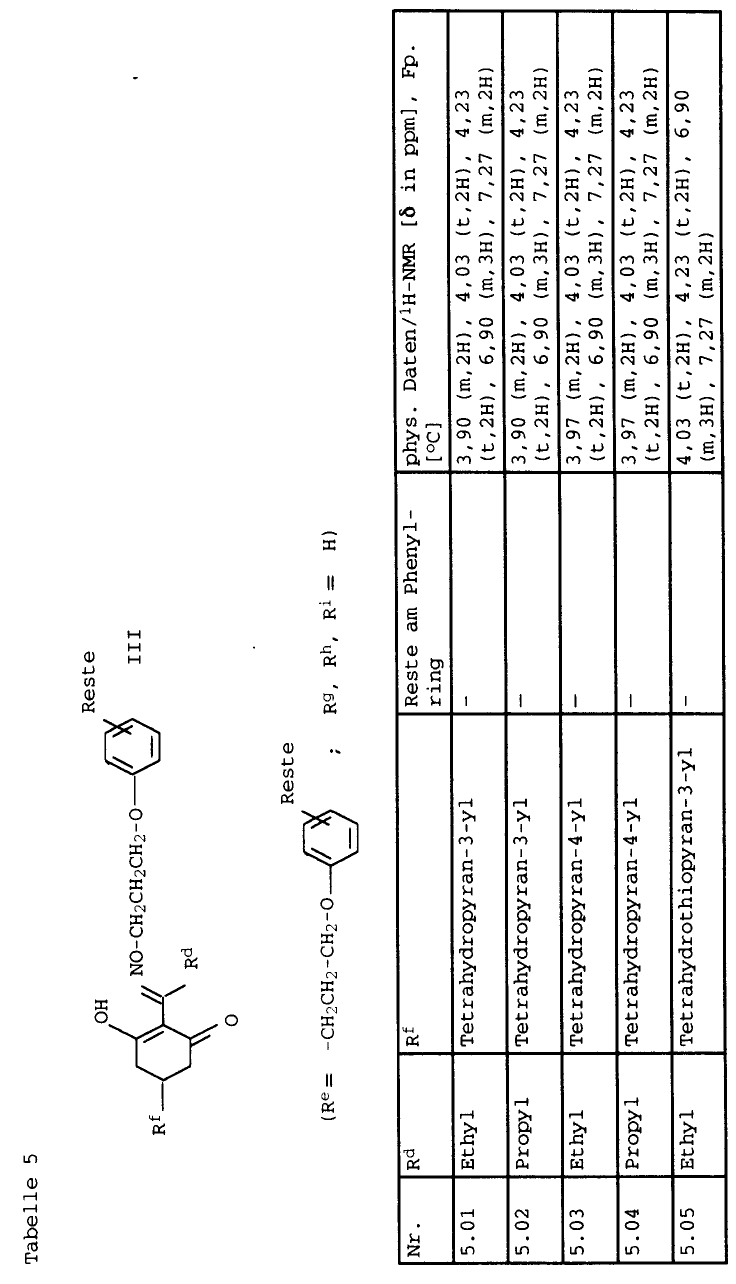

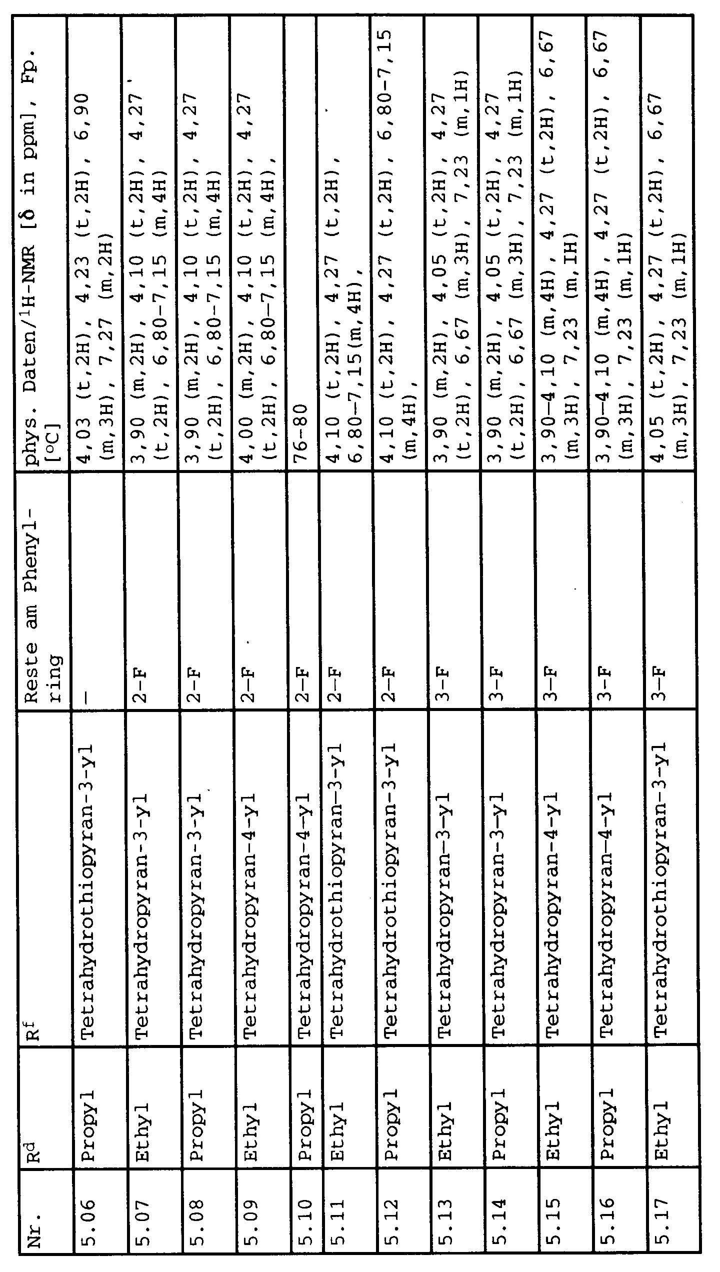

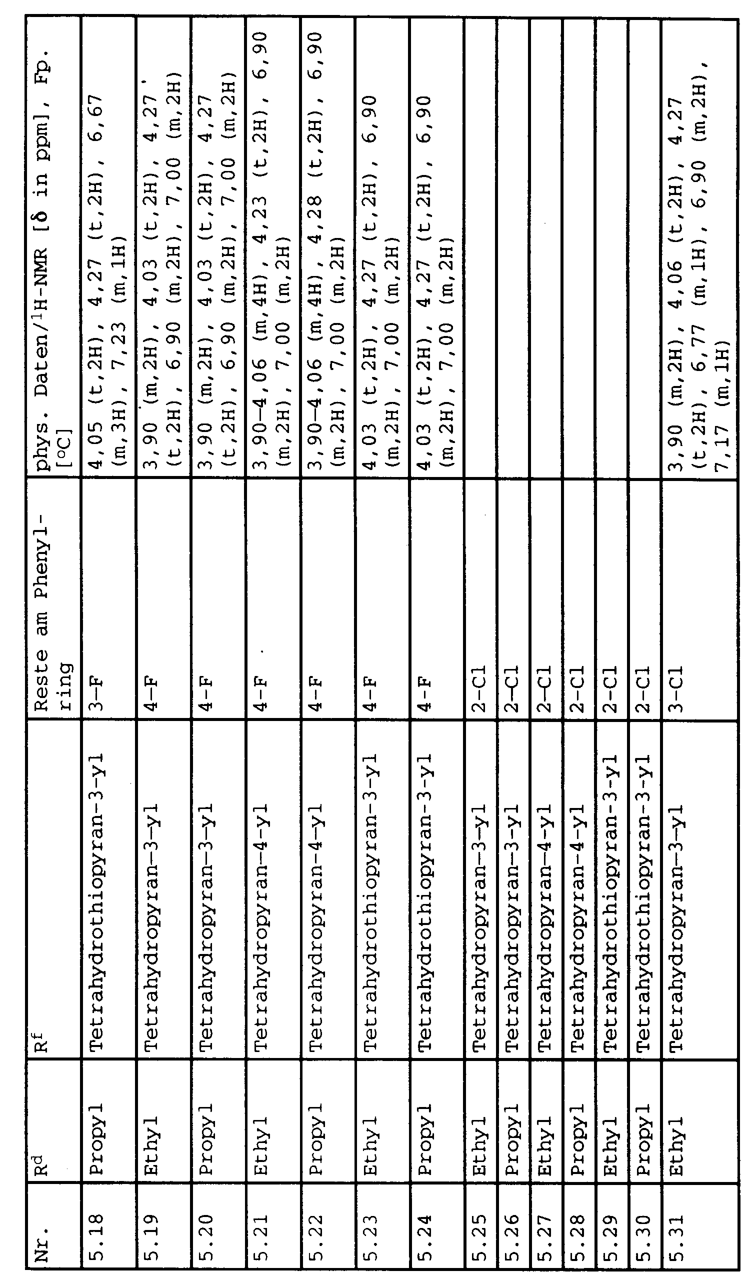

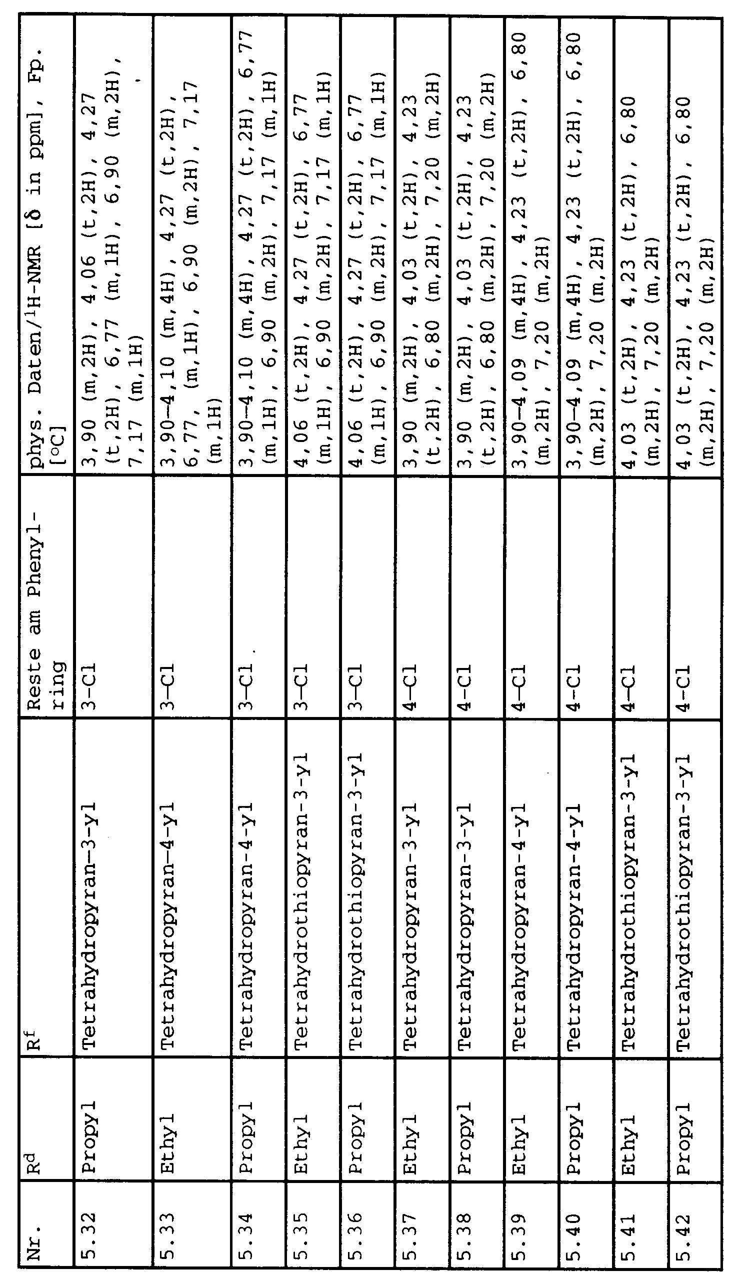

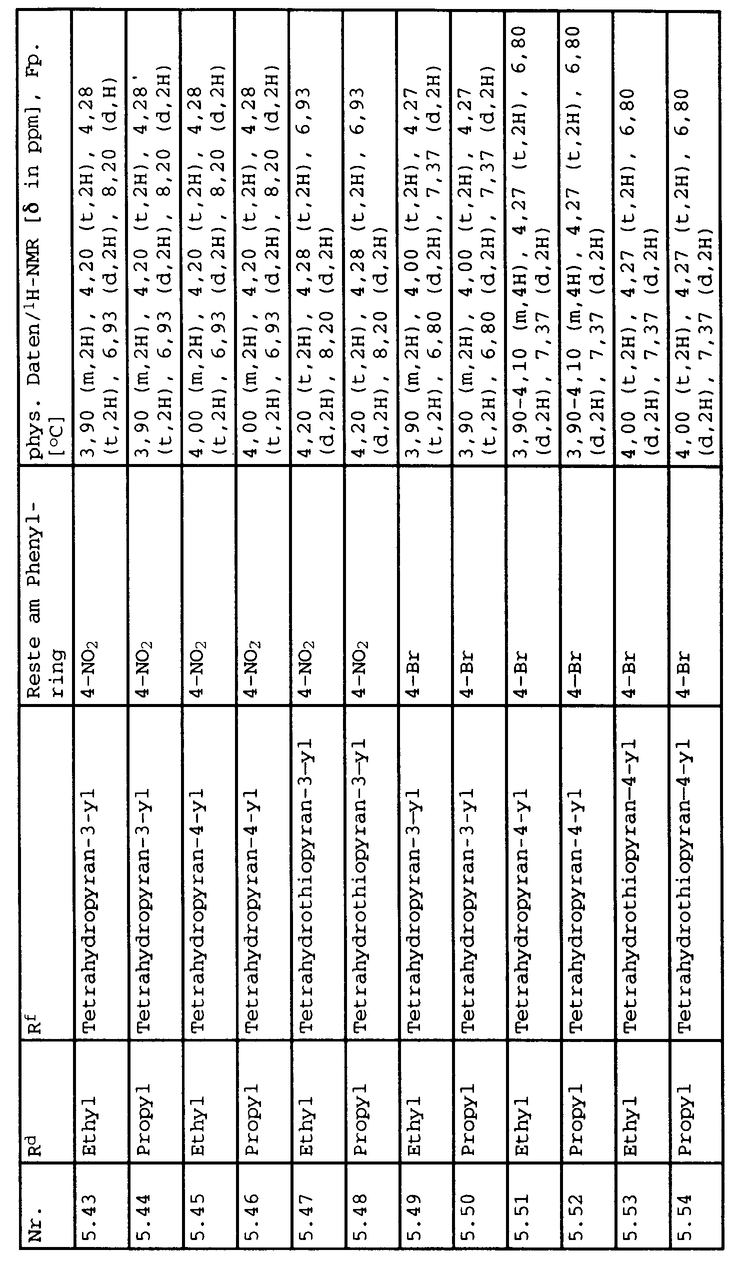

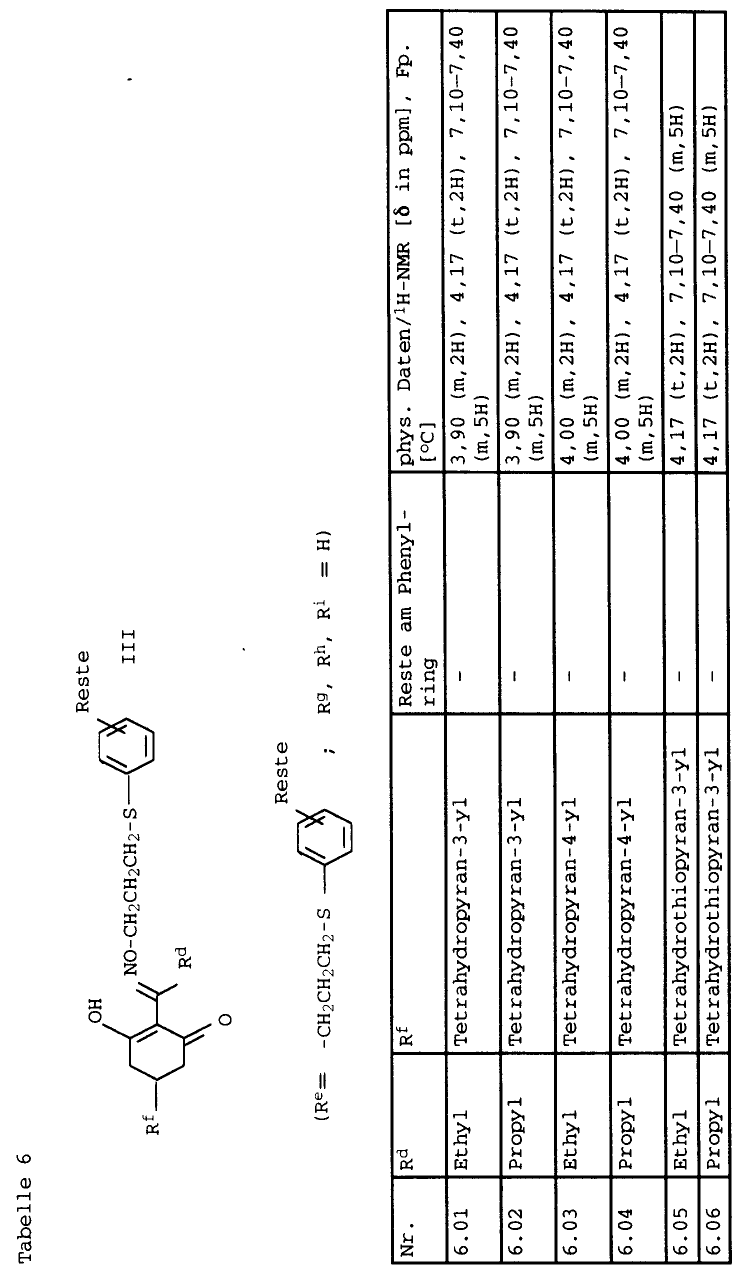

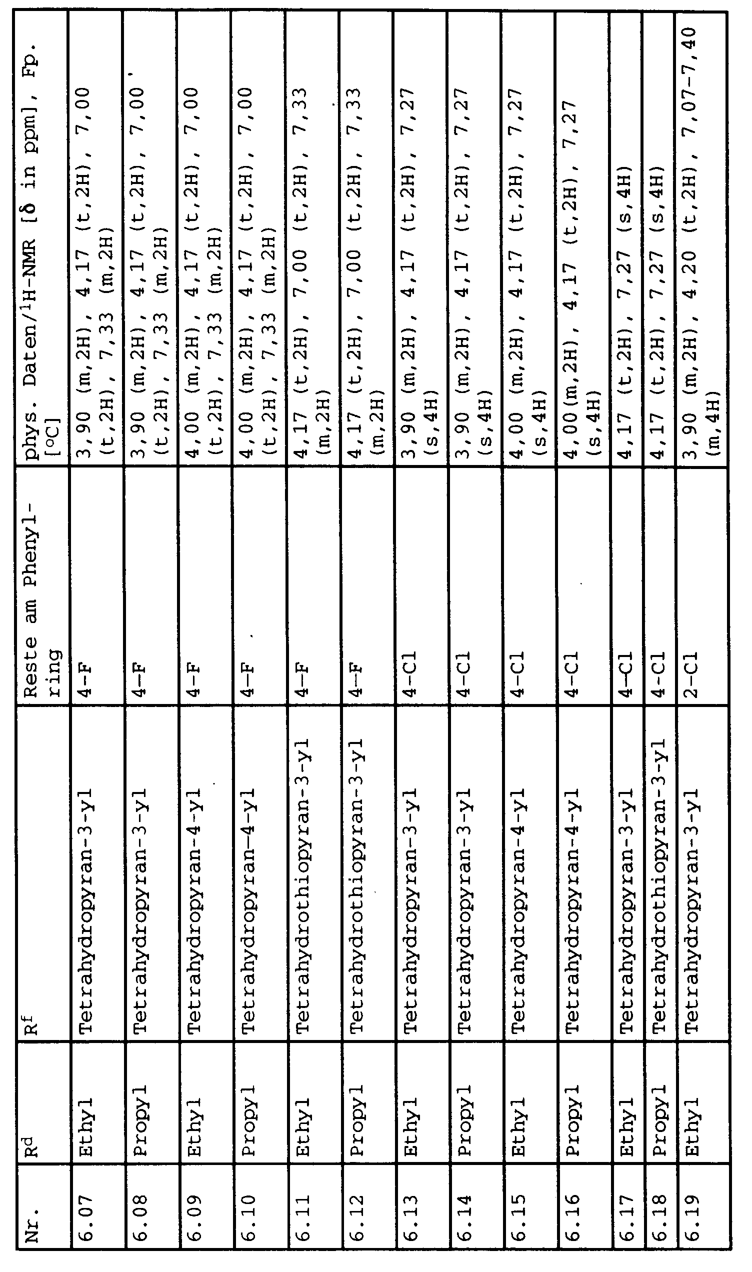

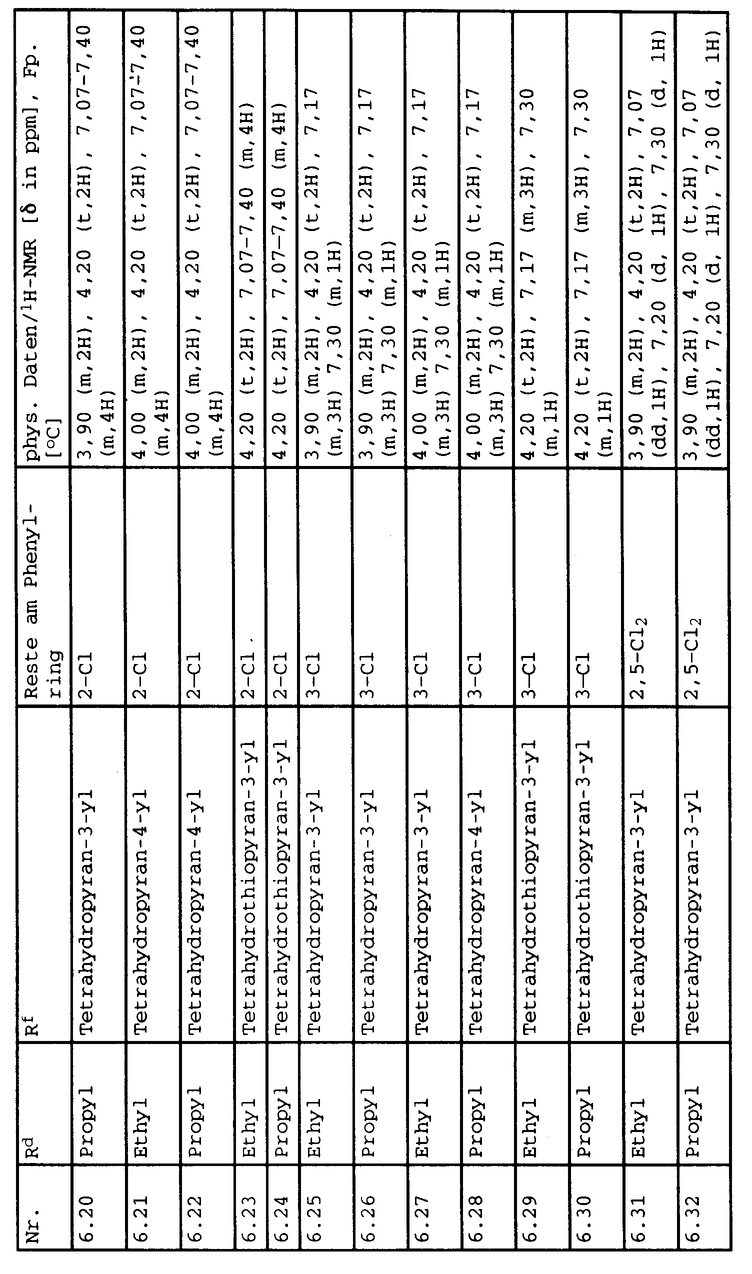

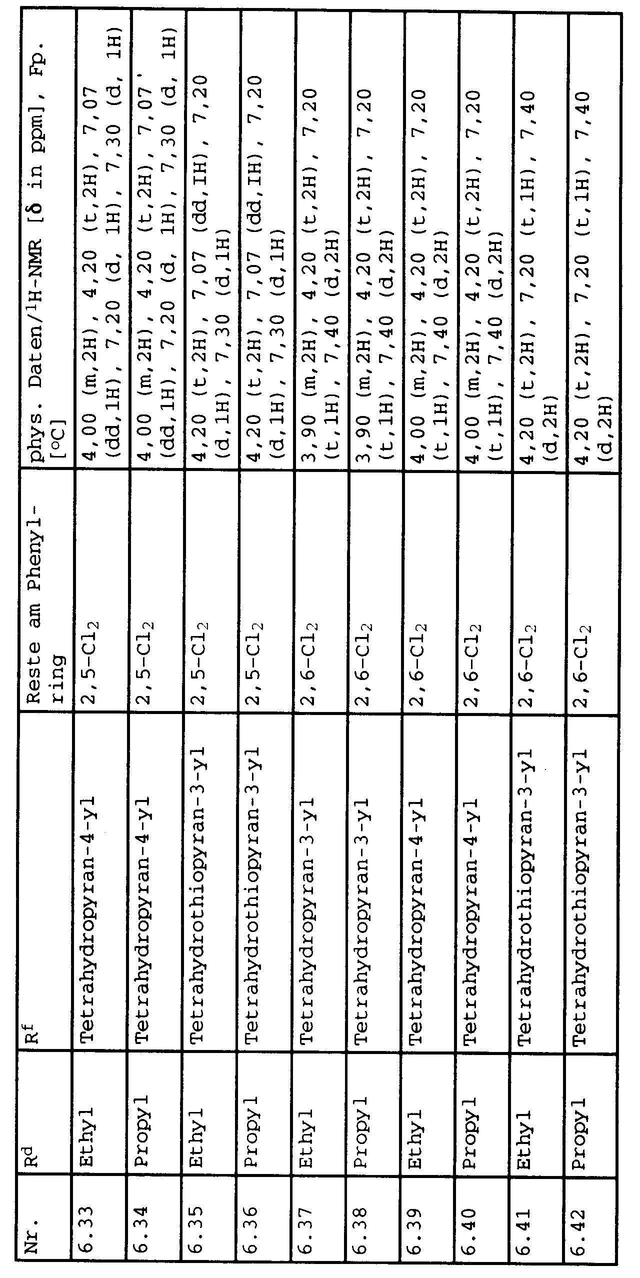

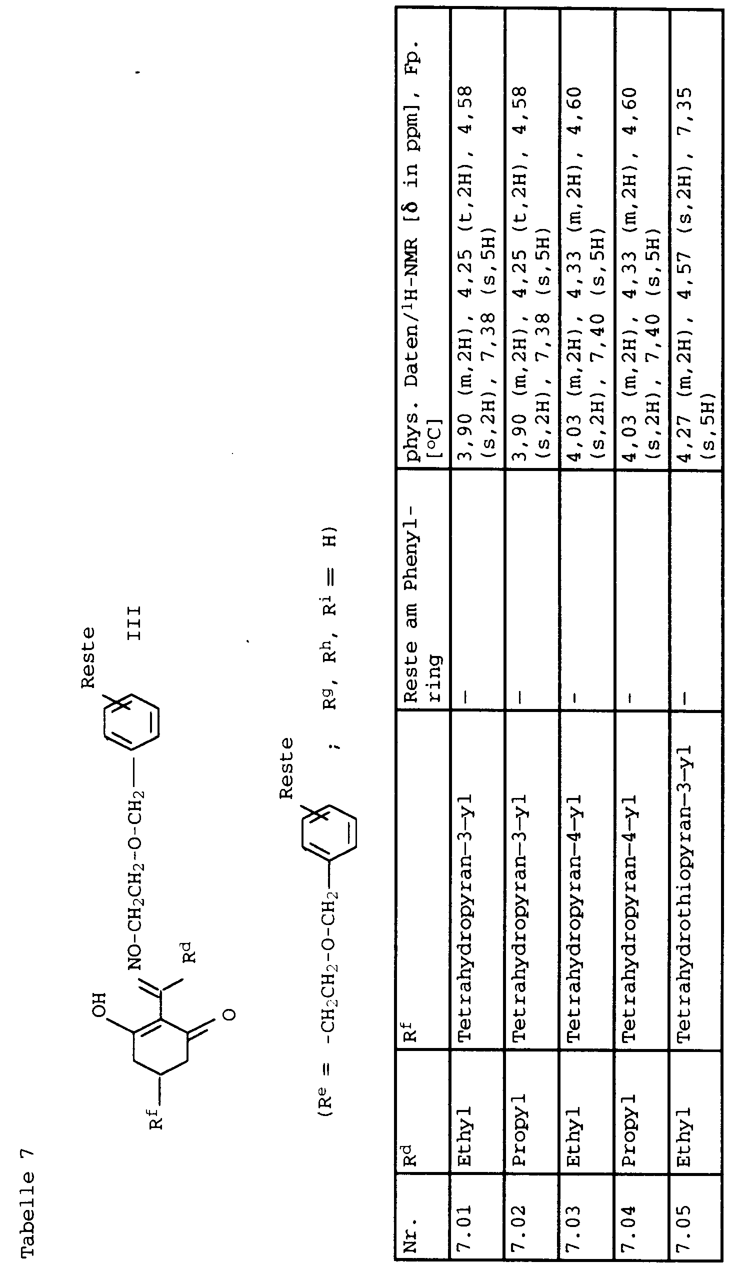

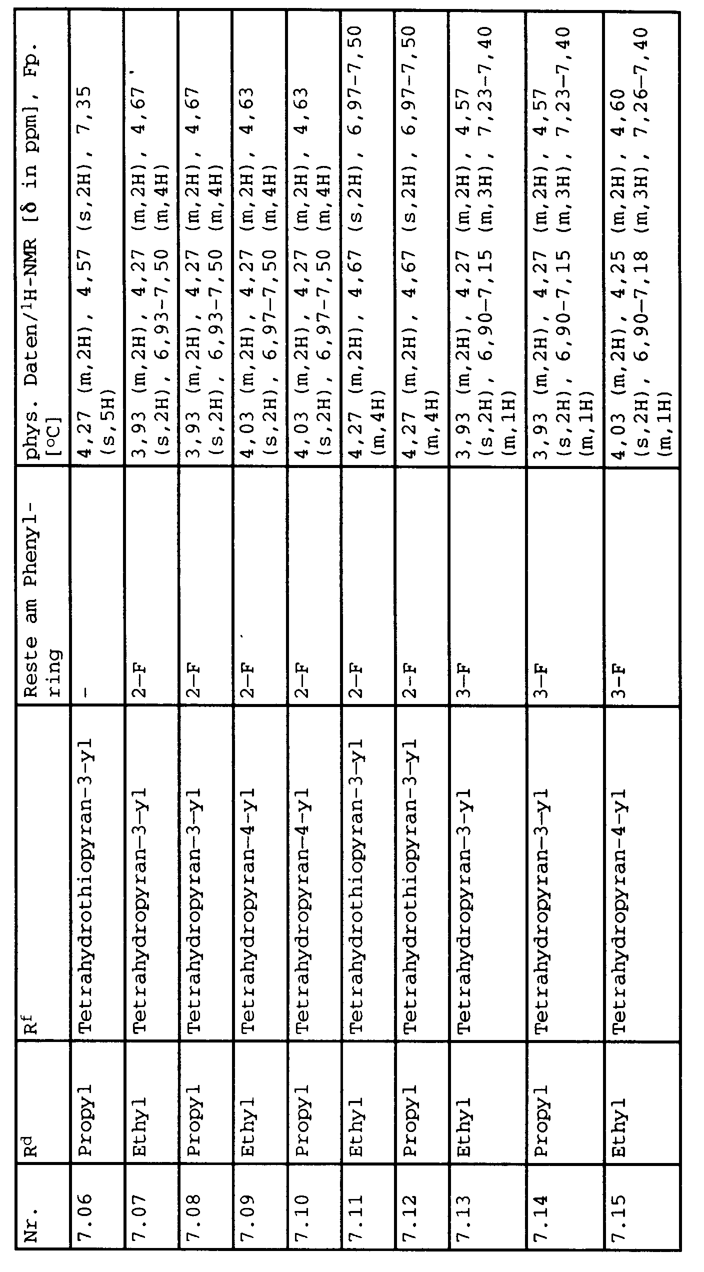

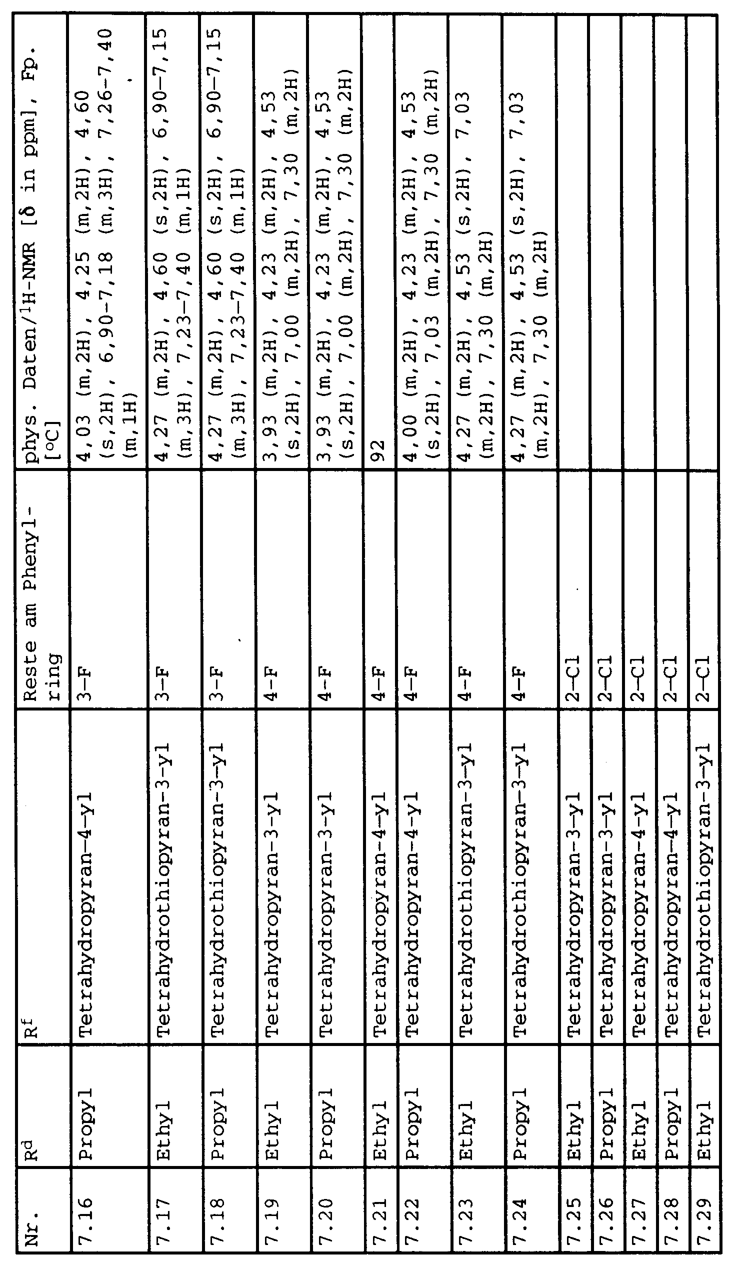

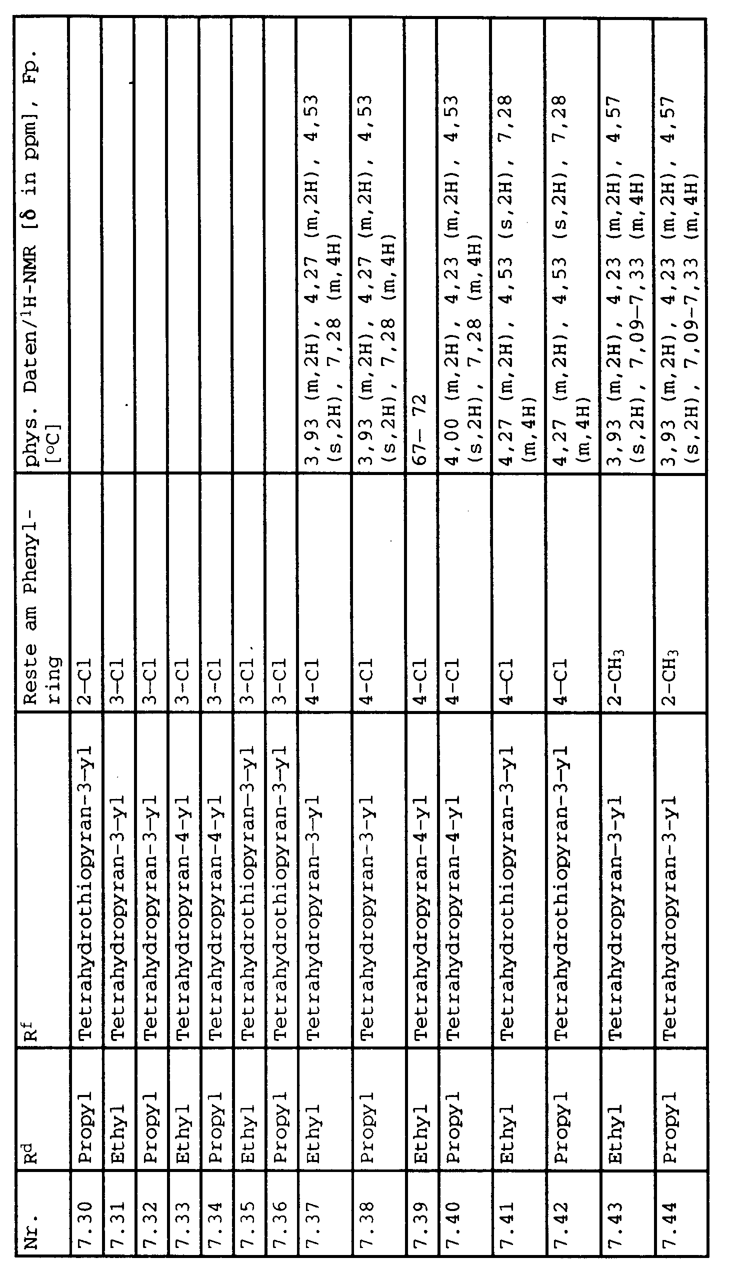

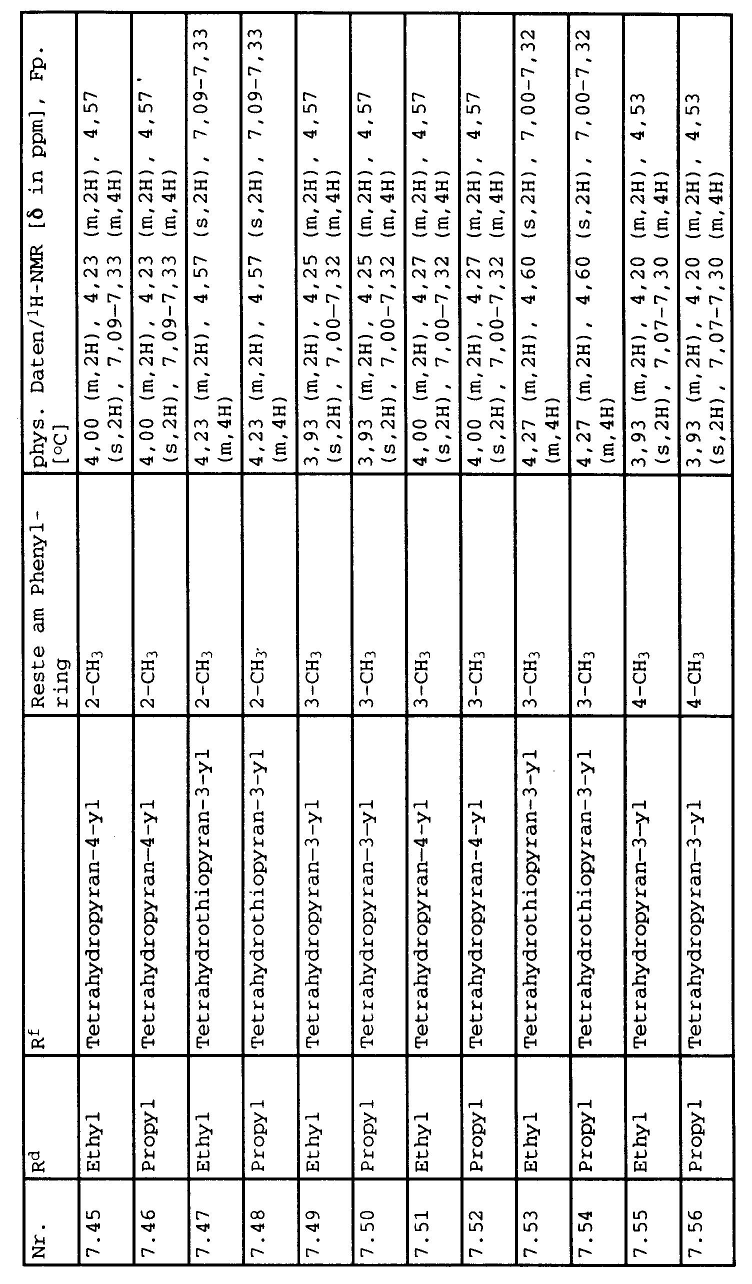

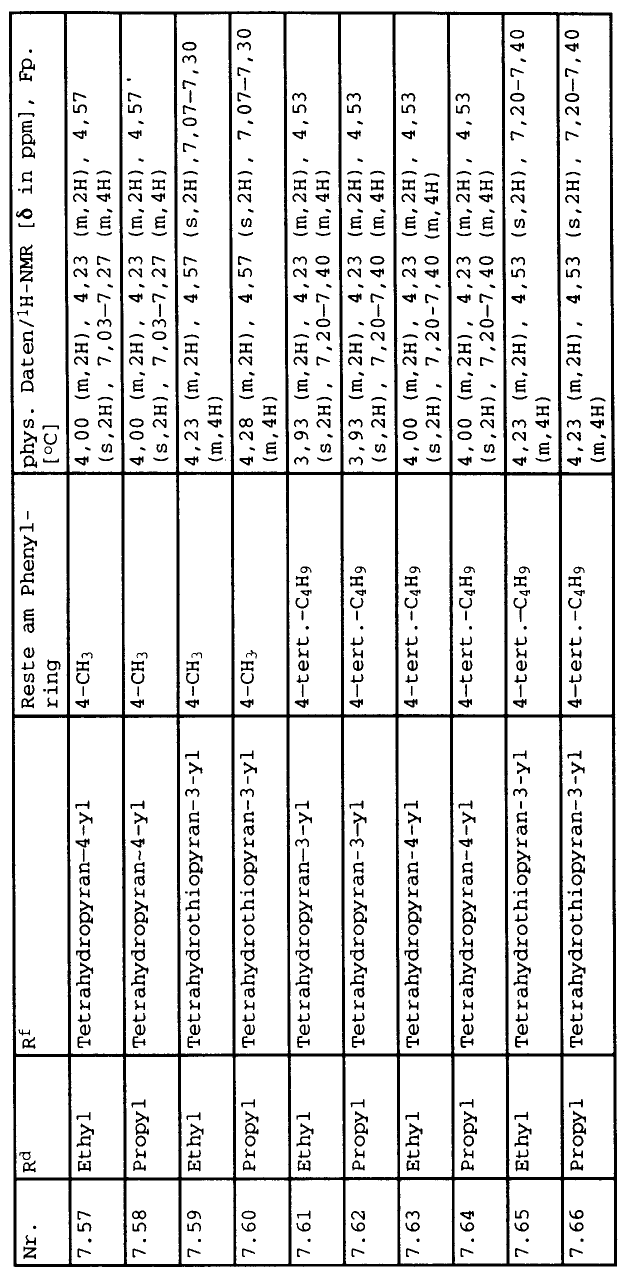

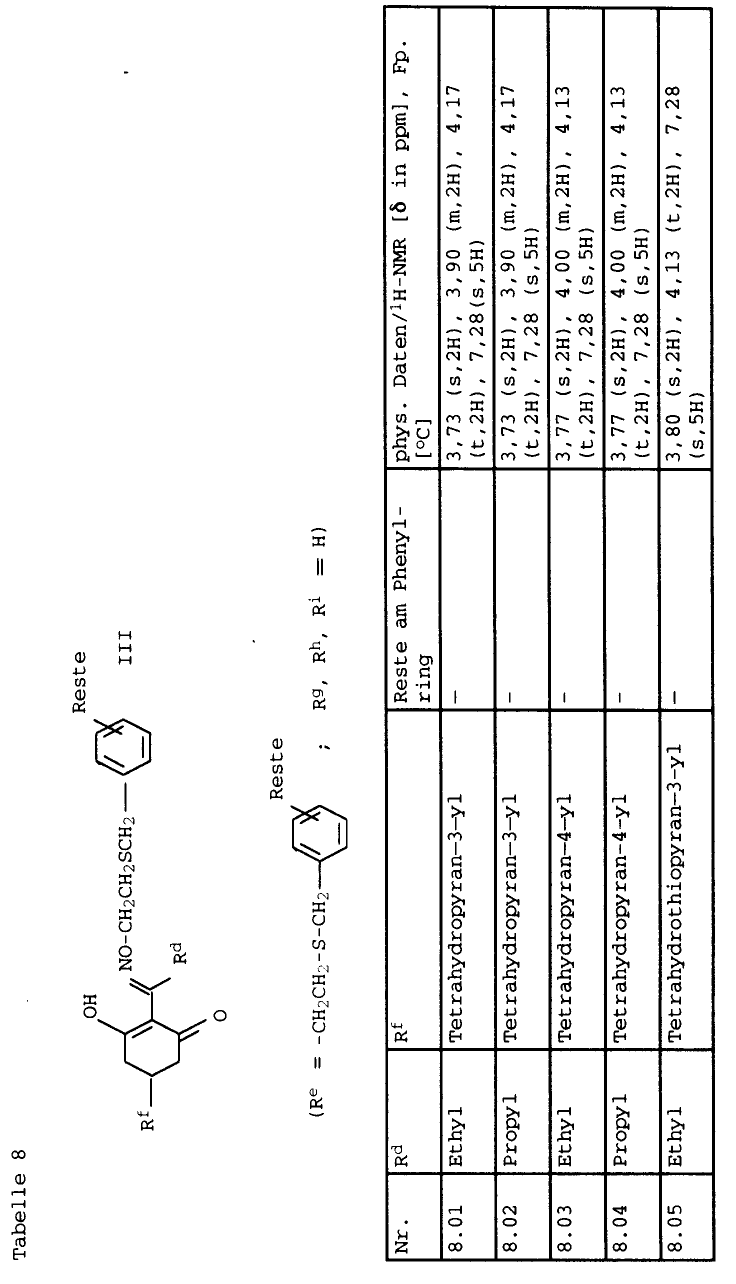

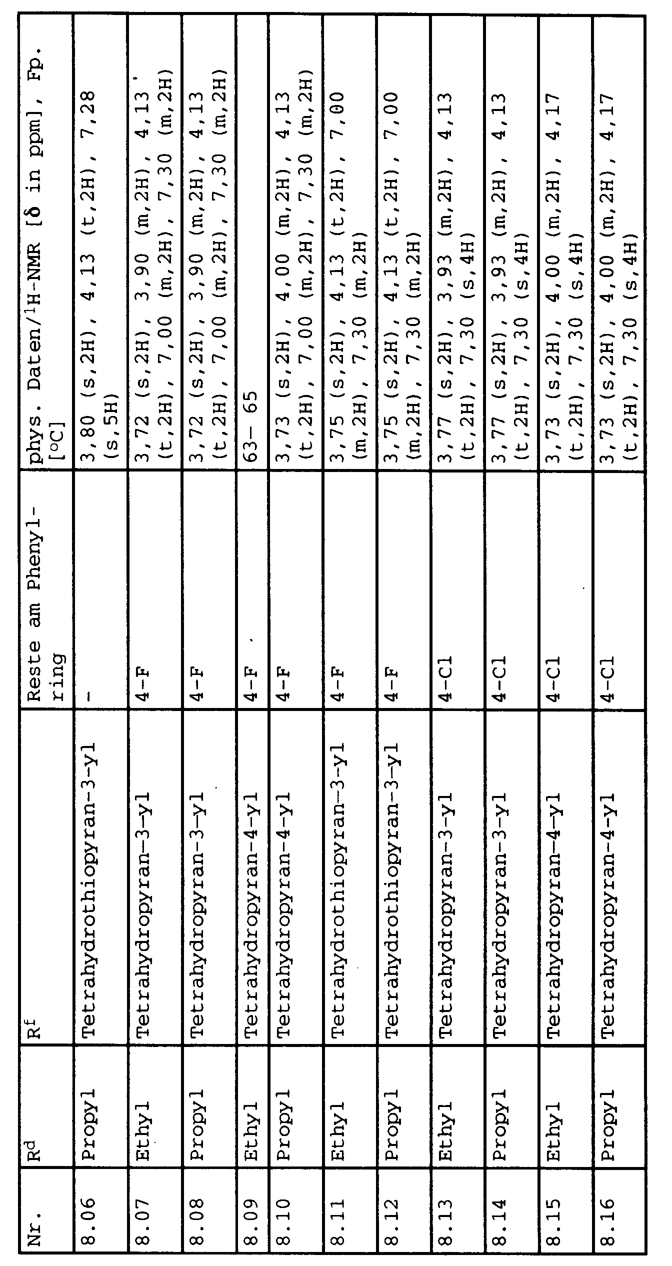

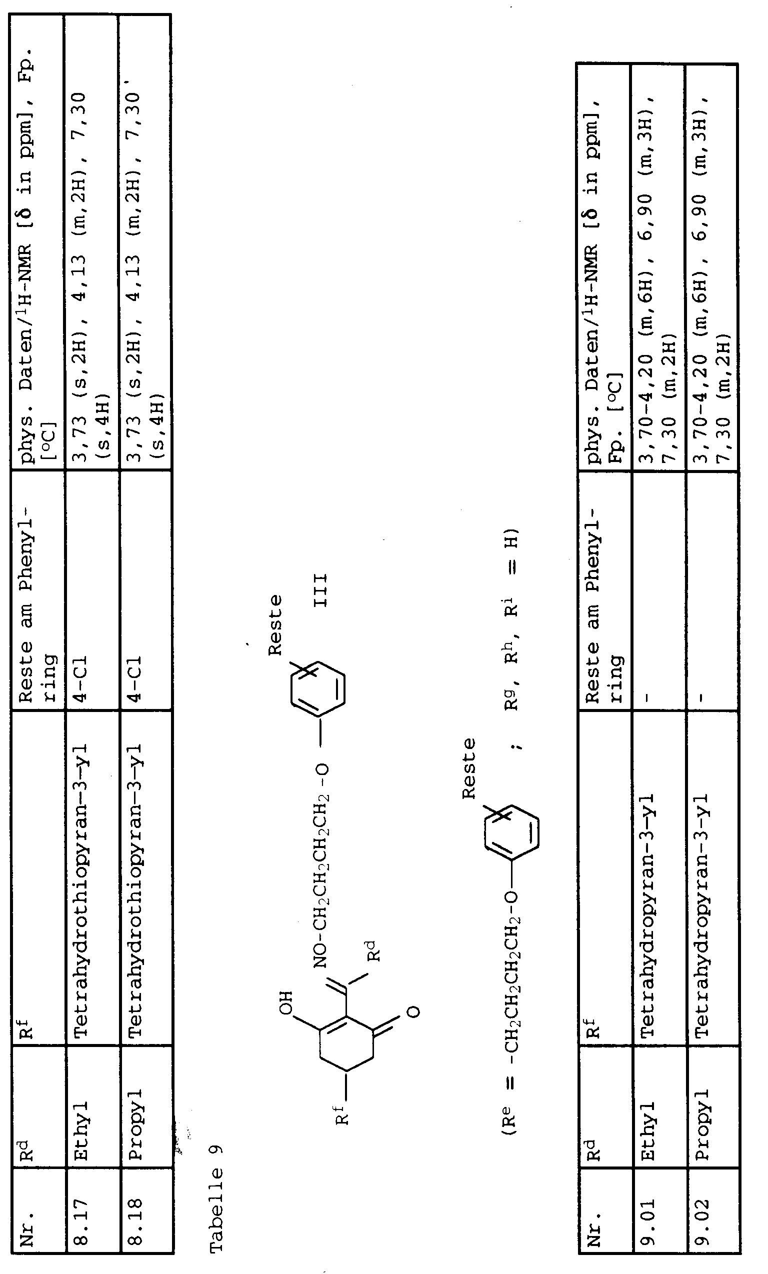

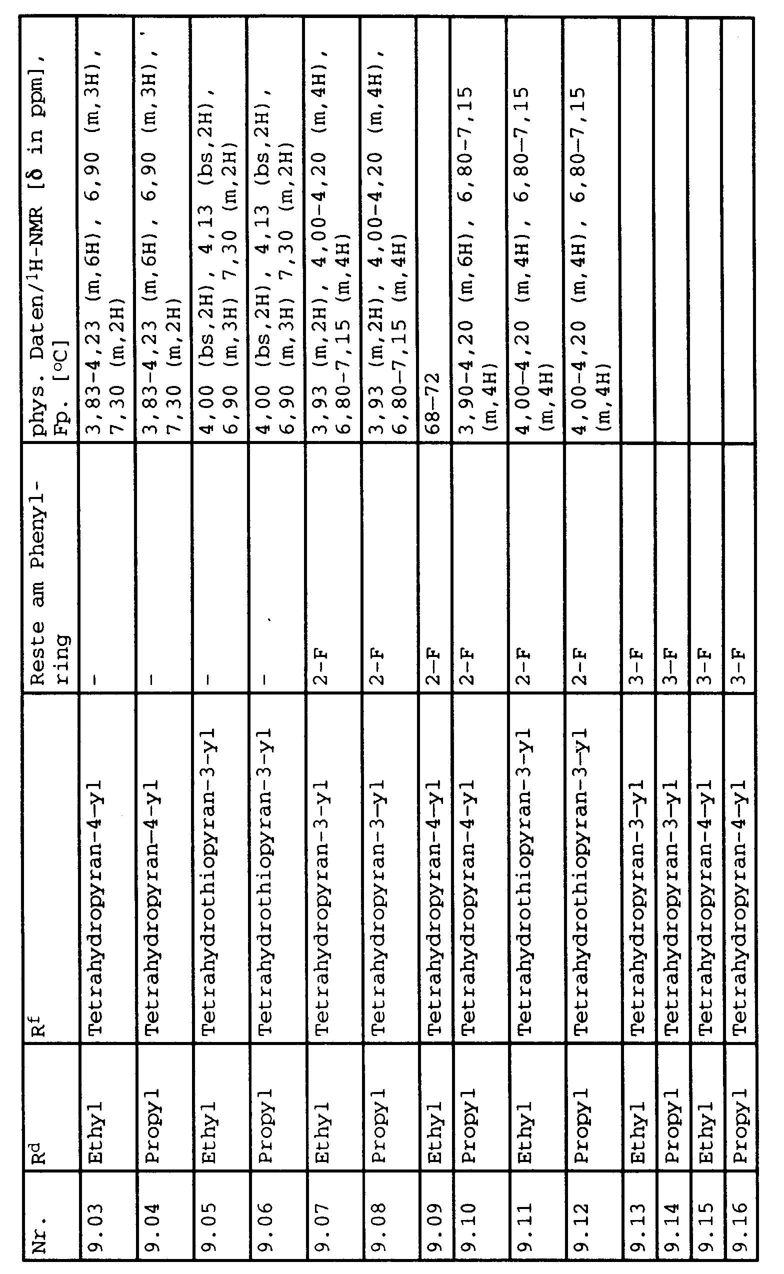

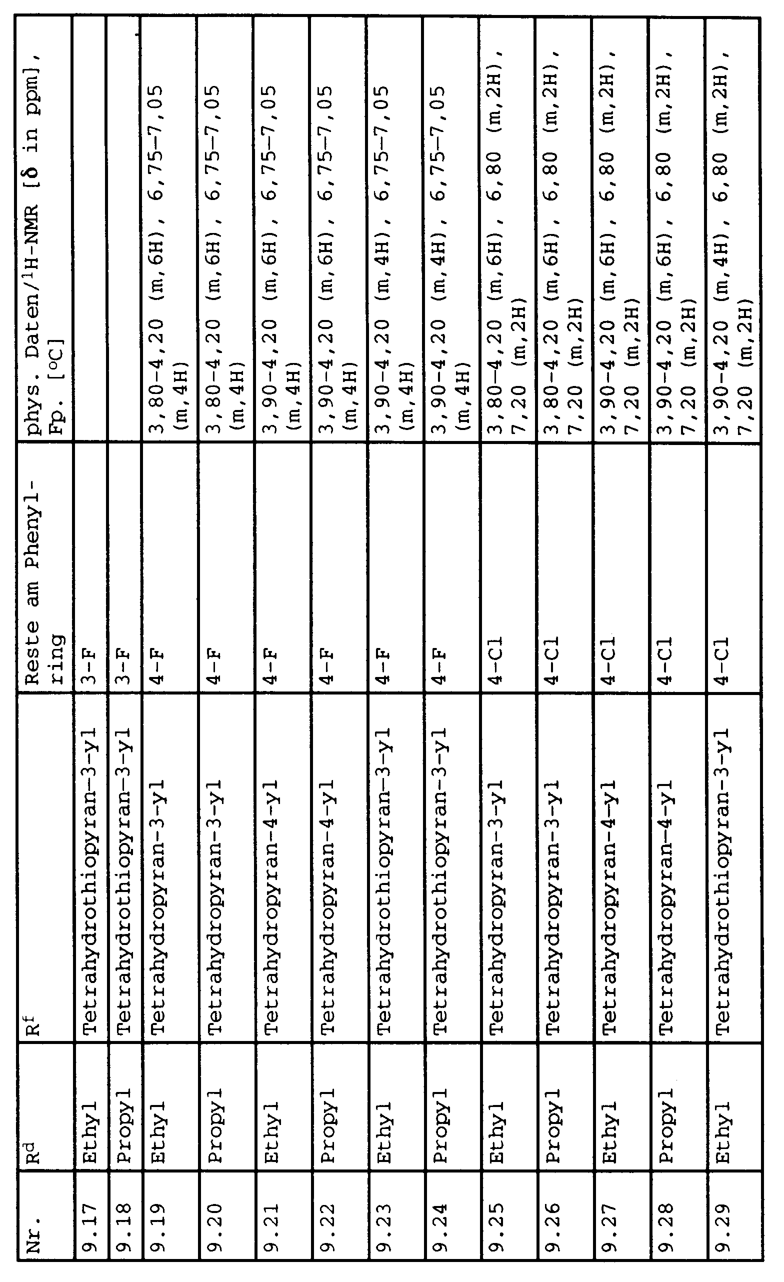

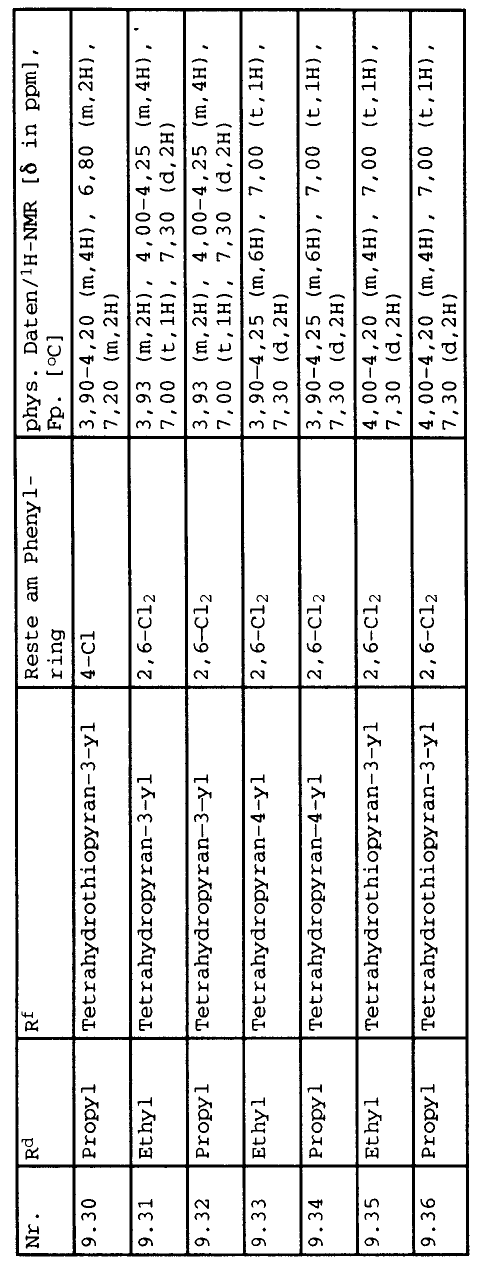

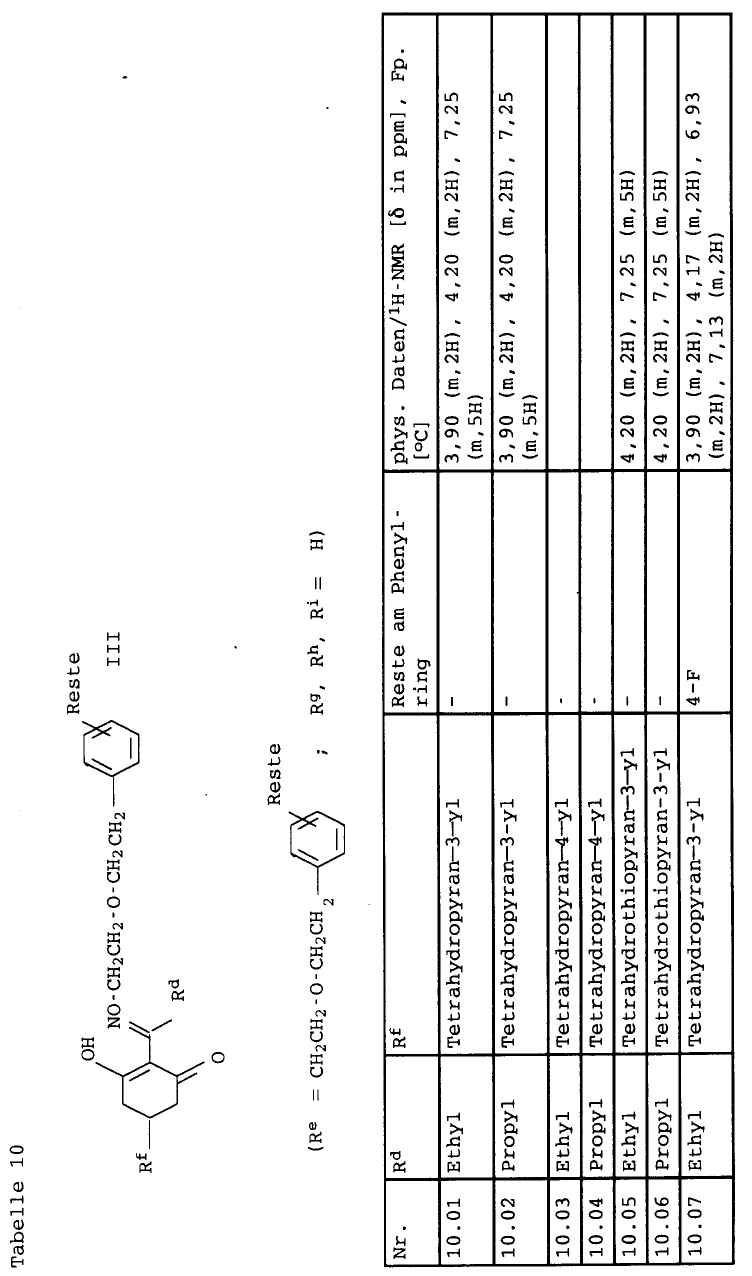

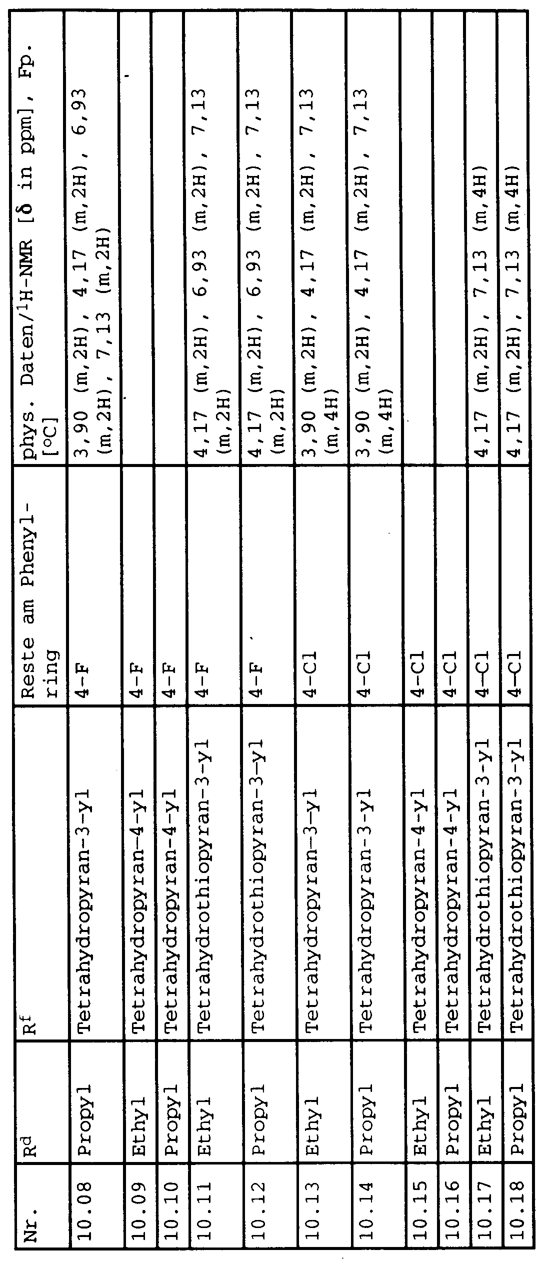

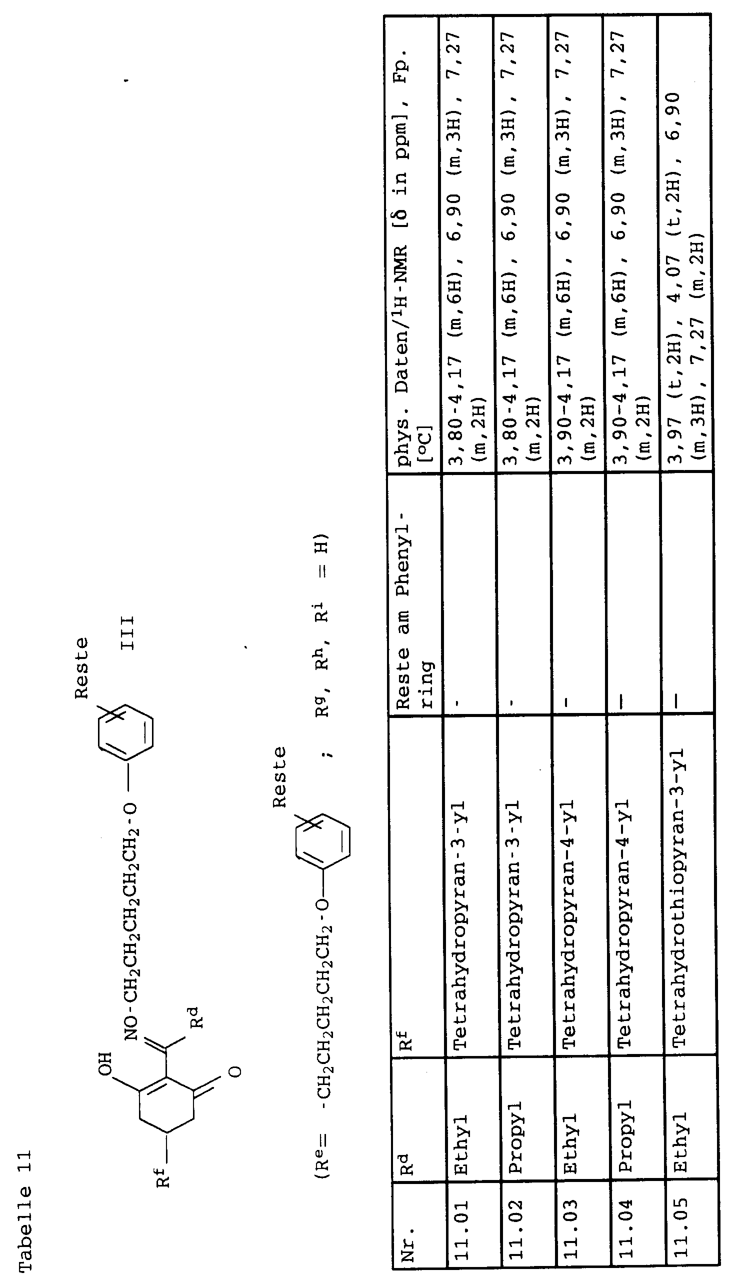

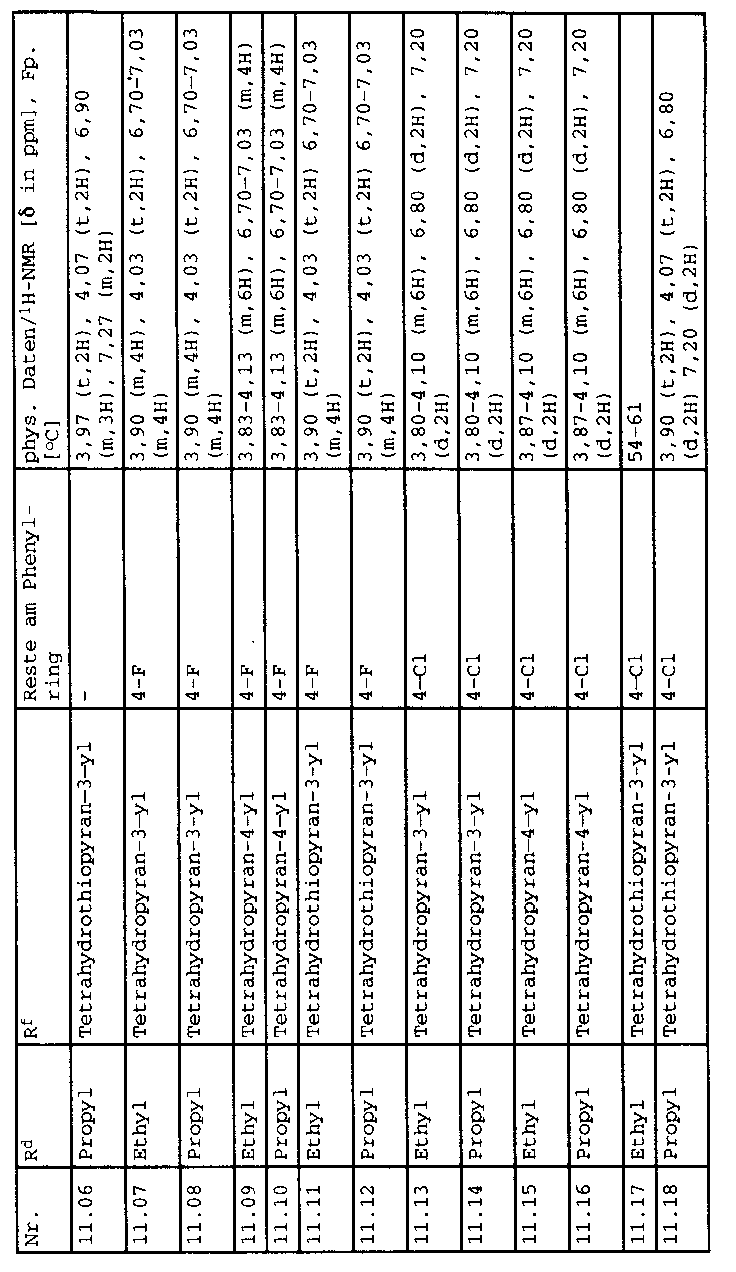

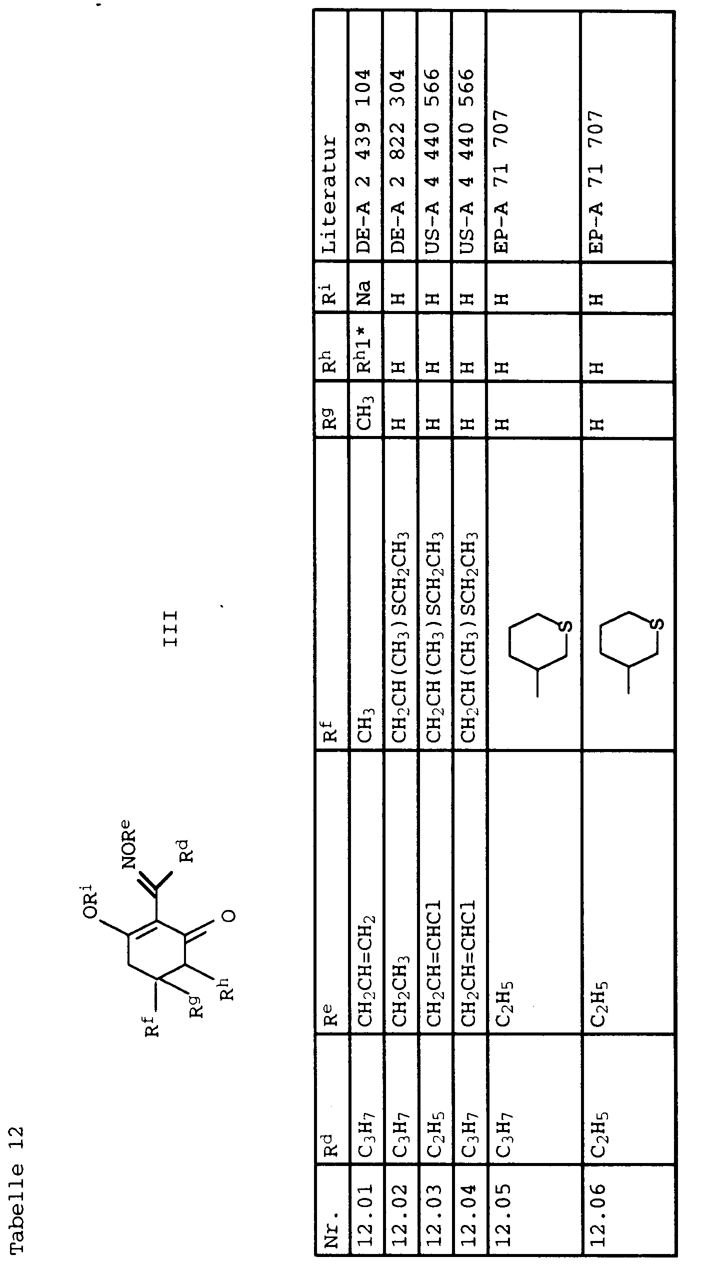

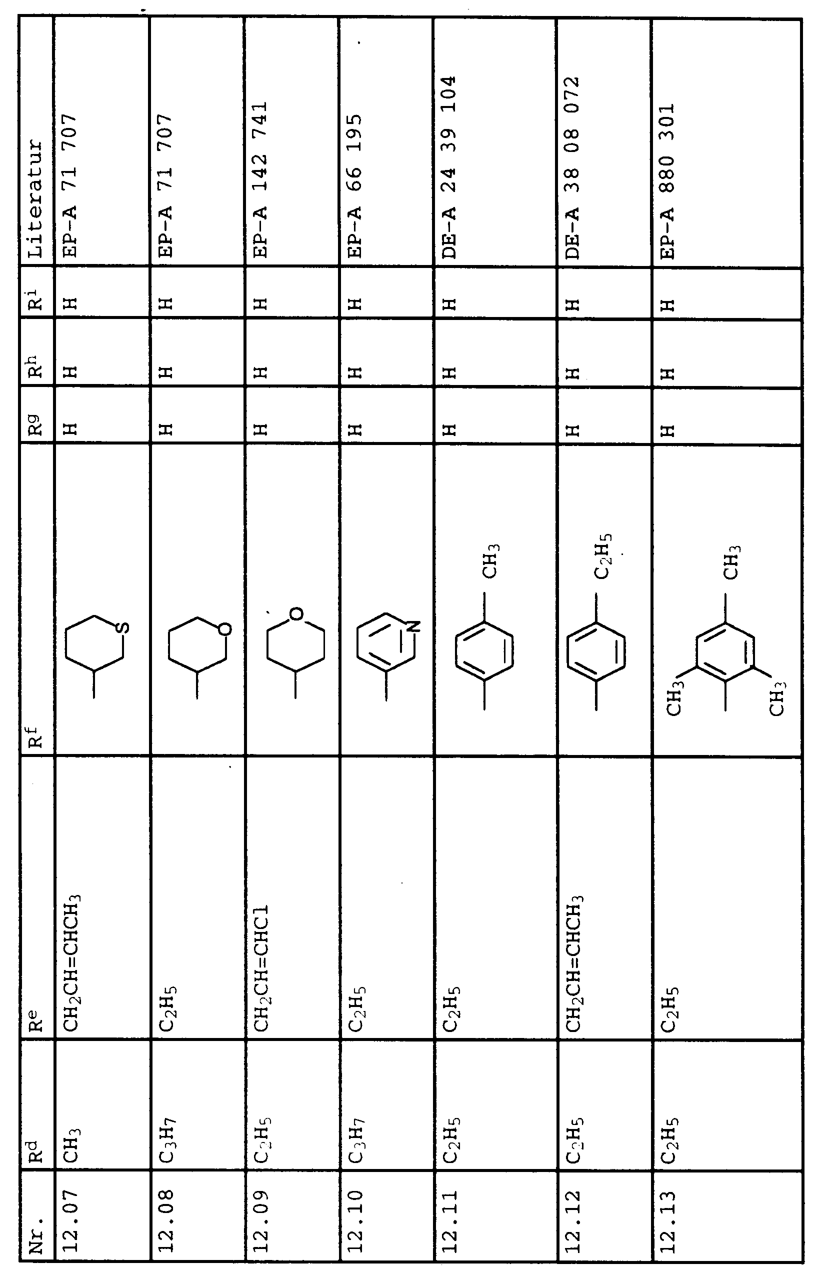

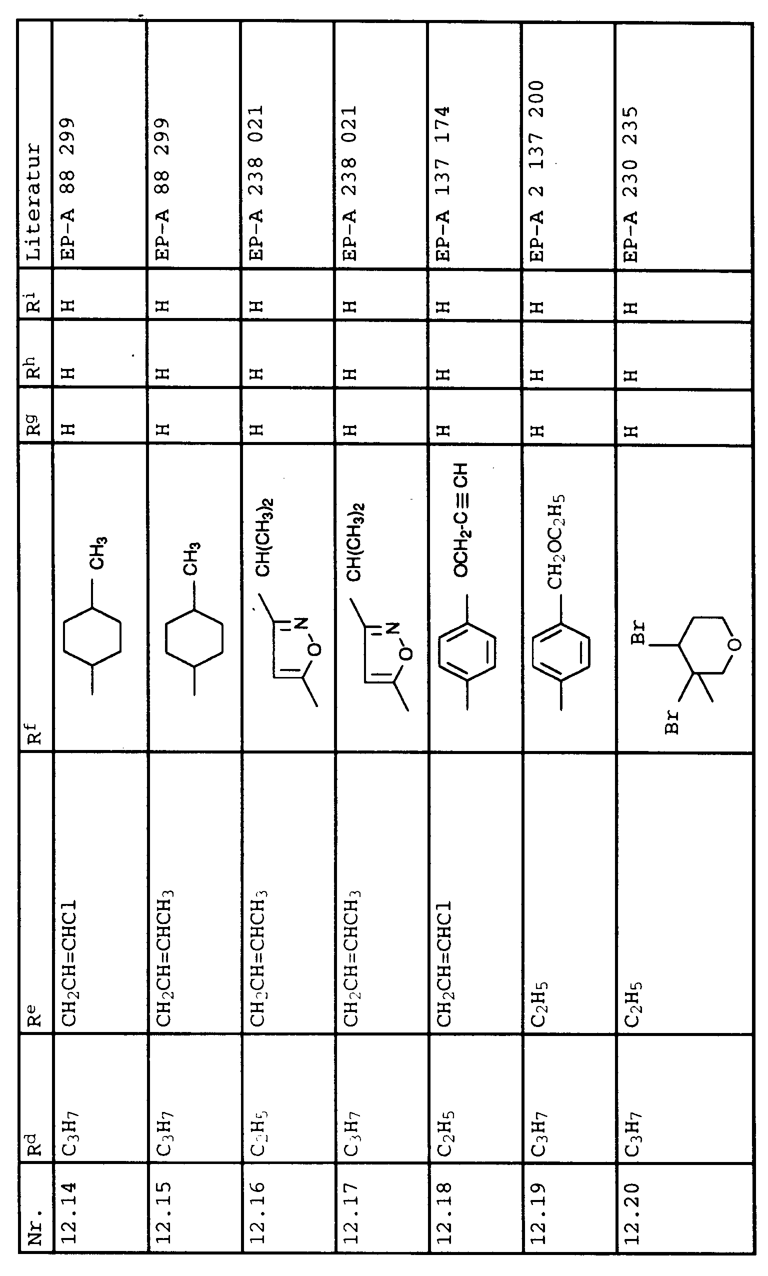

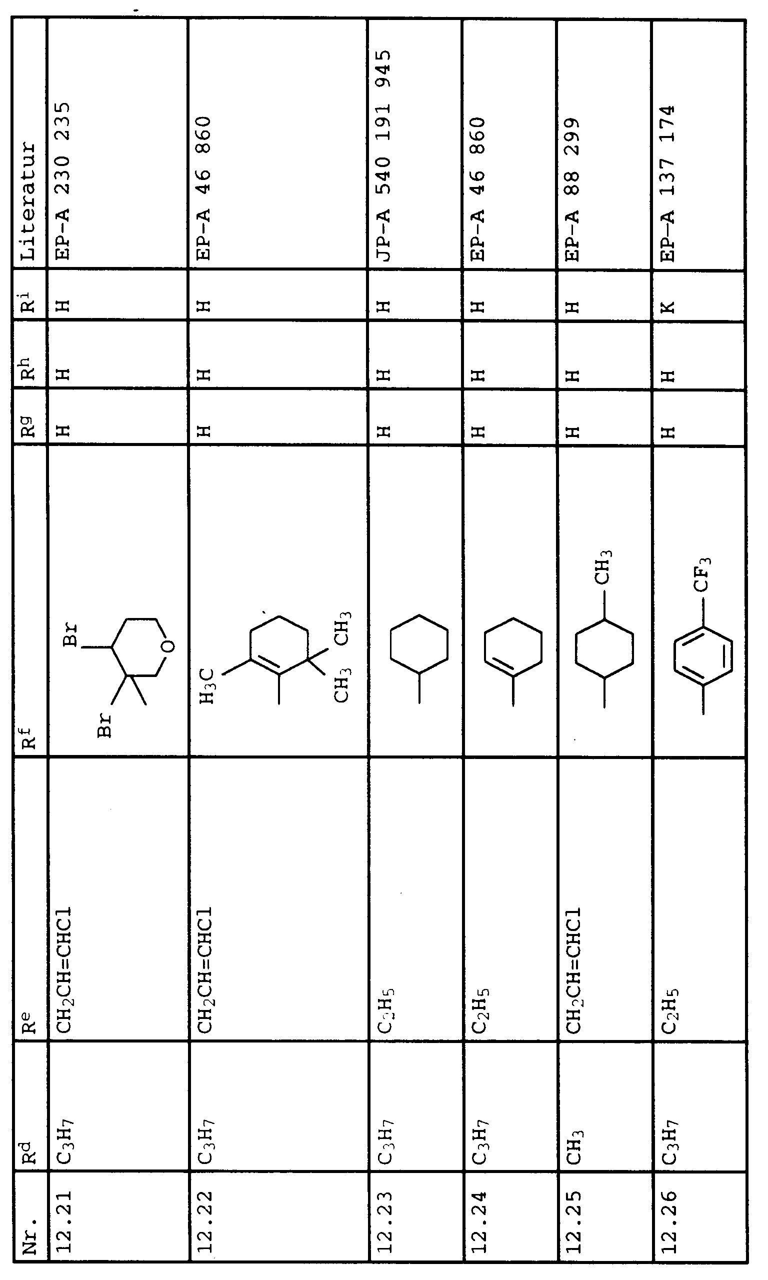

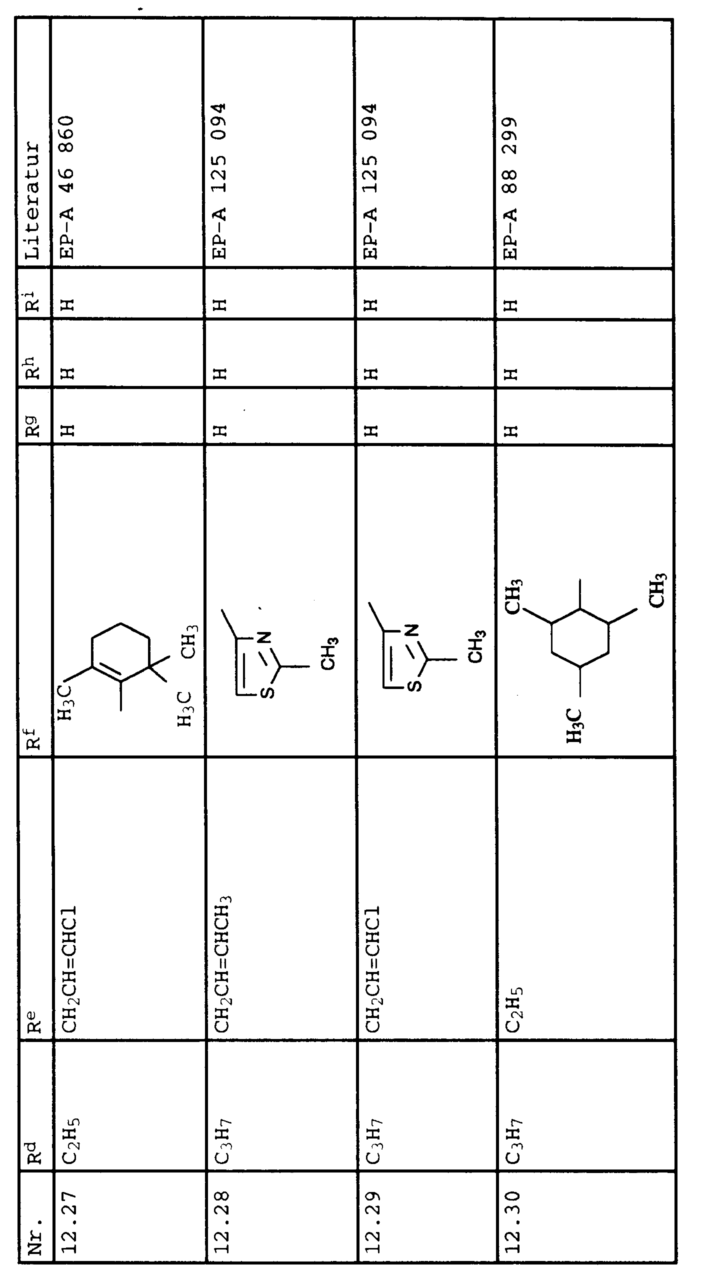

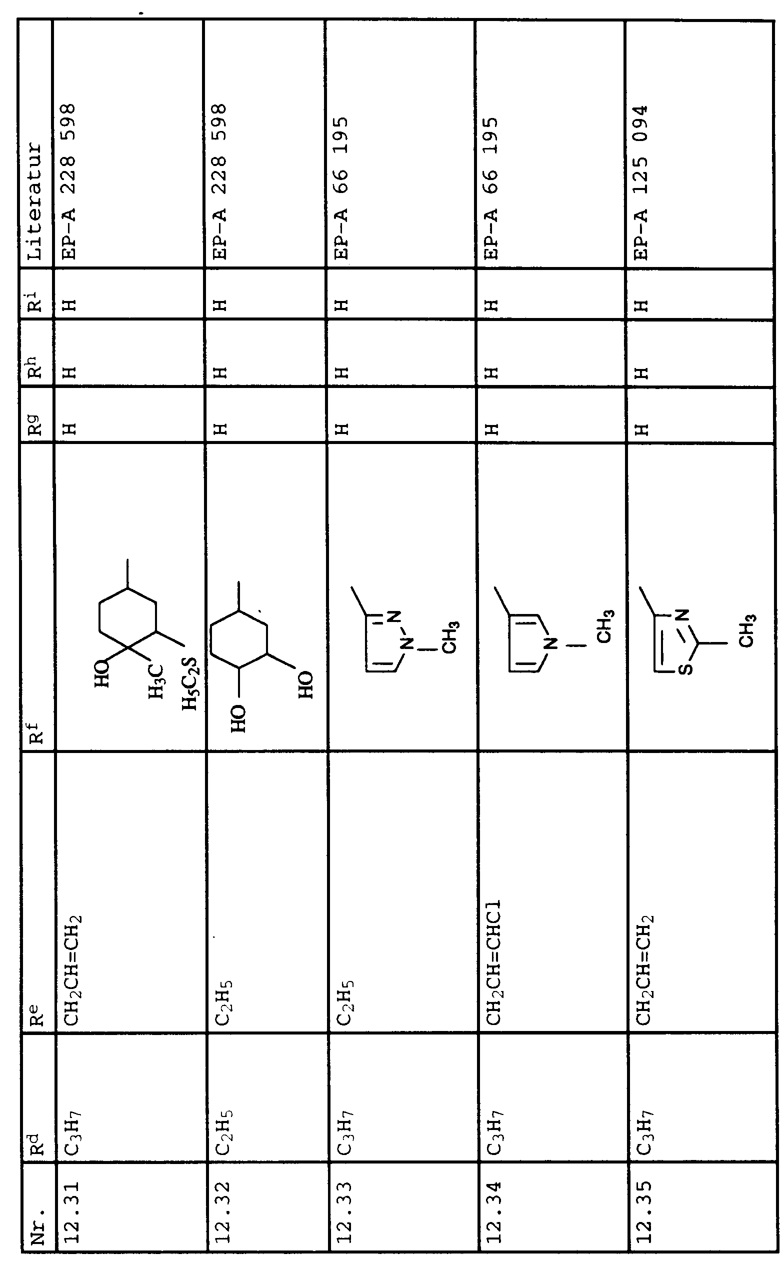

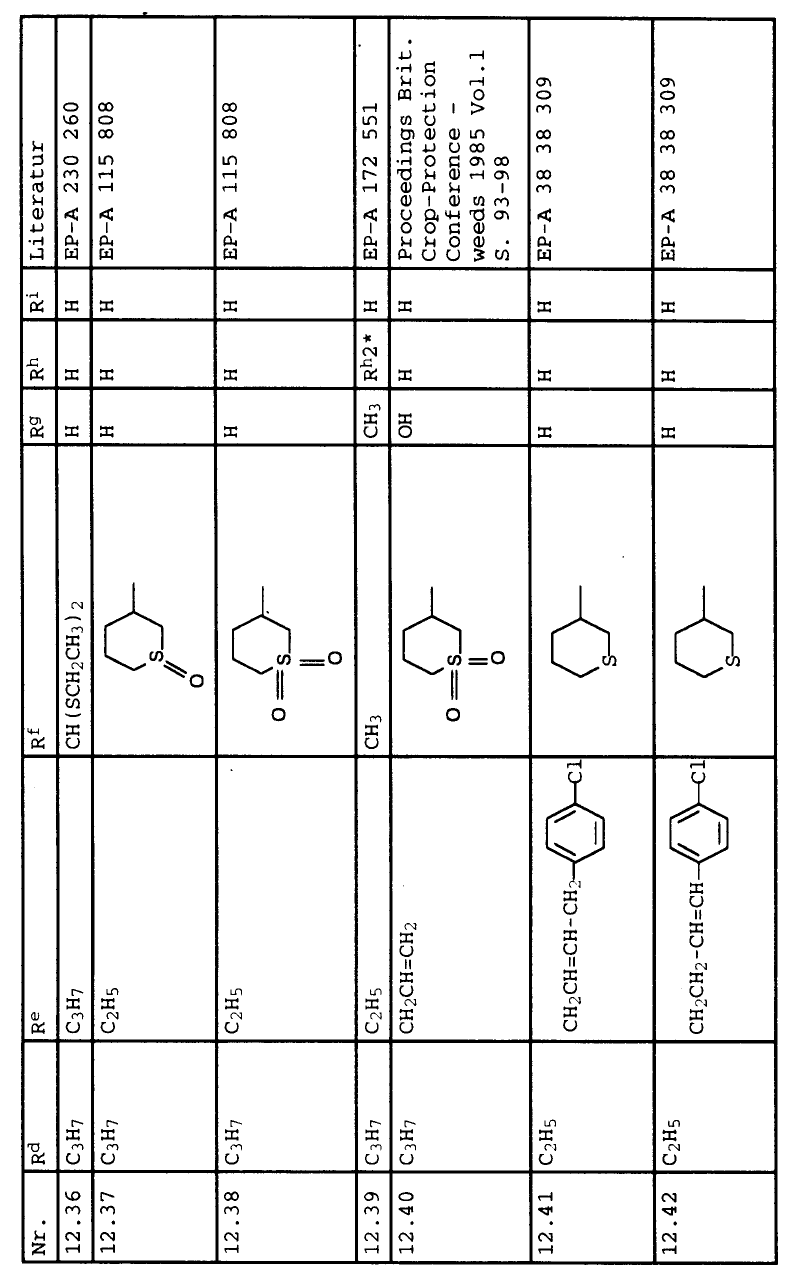

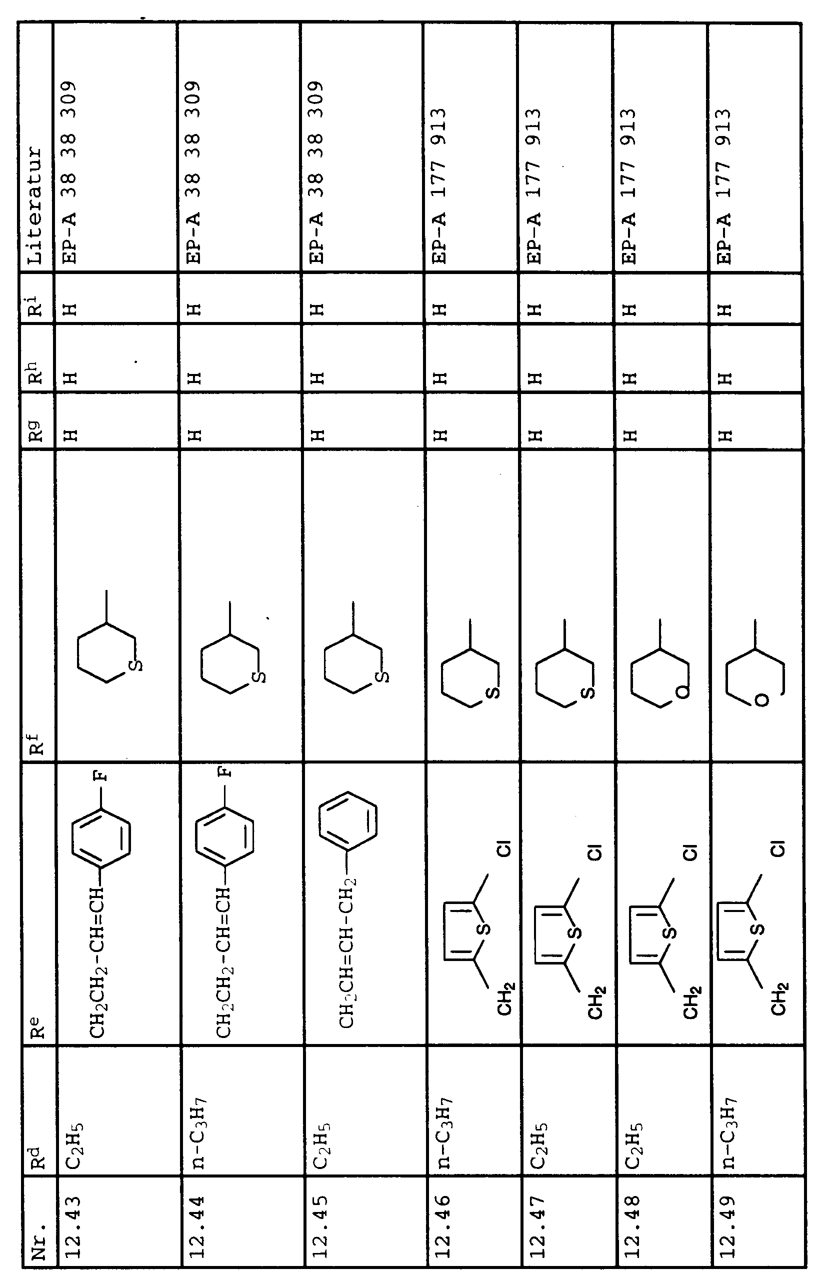

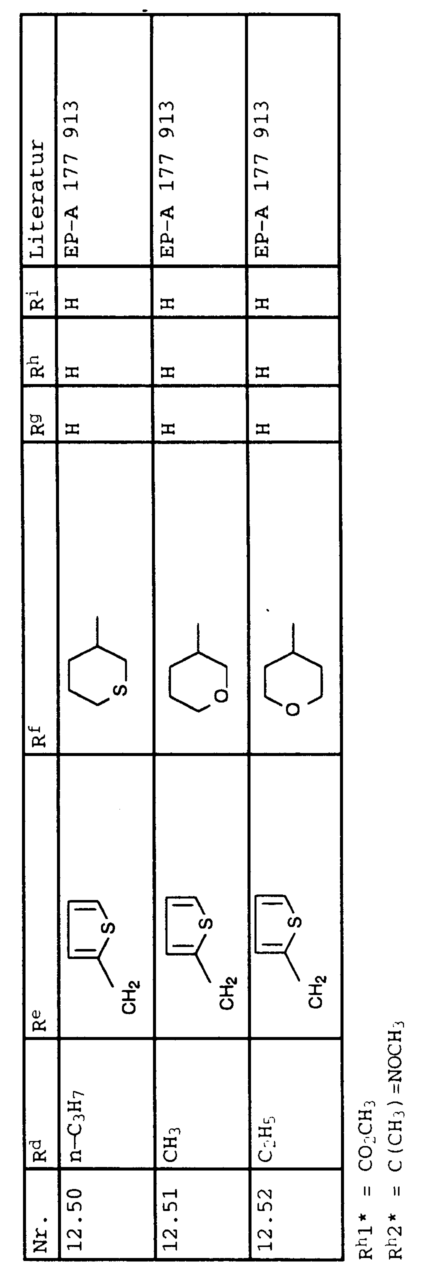

Herbizide Wirkstoffe und antidotisch wirkende Verbindungen können gemeinsam oder getrennt nach dem Auflaufen auf die Blätter und Sprosse der Kulturpflanzen und der unerwünschten Gräser ausgebracht werden. Bevorzugt wird das antidotisch wirkende Mittel gleichzeitig mit dem herbiziden Wirkstoff ausgebracht. Auch eine getrennte Ausbringung, wobei das Antidot zuerst und anschließend der herbizide Wirkstoff auf das Feld gebracht werden, ist möglich. Herbizider Wirkstoff und Antidot können hierbei als Spritzmittel in suspendierbarer, emulgierbarer oder löslicher Form gemeinsam oder getrennt formuliert vorliegen.

Antidotische Effekte werden auch durch Behandlung der Kulturpflanzensamen oder der Stecklinge mit dem Antidot vor der Aussaat bzw. vor dem Auspflanzen erzielt. Der herbizide Wirkstoff wird dann allein in der üblichen Weise appliziert.

Bei der Saatgutbehandlung werden im allgemeinen Wirkstoffmengen von 0,1 bis 10 g, vorzugsweise 1 bis 2 g, je Kilogramm Saatgut benötigt.

Bei der Applikation des Antidot durch Samenquellung oder bei der Stecklingsbehandlung werden bevorzugt Lösungen eingesetzt, die den antagonistischen Wirkstoff in einer Konzentration von 1 bis 10.000 ppm, insbesondere 100 bis 10.000 ppm, enthalten.

Für das gleiche Cyclohexenonderivat III werden unterschiedliche Mengen einer antidotisch wirkenden Verbindung benötigt, wenn das Cyclohexenonderivat in verschiedenen Kulturen eingesetzt wird. Die Mengenverhältnisse, in denen ein Cyclohexenonderivat und ein substituiertes 2-Aminothiophen I eingesetzt werden, sind in breiten Bereichen variabel. Sie sind abhängig von der Struktur des Cyclohexenonderivats, des substituierten 2-Aminothiophens I und der jeweiligen Kultur. Geeignete Anteilverhältnisse von herbizidem Wirkstoff zu antidotisch wirkender Verbindung liegen zwischen 1 : 10 und 1 : 0,01; vorzugsweise zwischen 1 : 4 und 1 : 0,25 Gew.-Teilen.

Die erfindungsgemäßen Mittel bzw. bei getrennter Ausbringung die herbiziden Wirkstoffe oder das Antidot werden beispielsweise in Form von direkt versprühbaren Lösungen, Pulvern, Suspensionen, auch hochprozentige wäßrige, ölige oder sonstige Suspensionen, oder Dispersionen, Emulsionen, Öldispersionen, Pasten, Stäubemitteln, Streumitteln oder Granulaten durch Versprühen, Vernebeln, Verstäuben, Verstreuen oder Gießen angewendet. Die Anwendungsformen richten sich ganz nach den Verwendungszwecken.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische oder aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie Dimethylformamid, Dimethylsulfoxid, N-Methylpyrrolidon und Wasser, in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können herbizider Wirkstoff und/oder Antidot als solche oder in einem Öl oder Lösungsmittel gelöst, mittels Netz-, Haft-, Dispergier- oder Emulgiermittel in Wasser homogenisiert werden. Es können aber auch aus herbizidem Wirkstoff und/oder Antidot Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatierten Fettalkoholglykolethern, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenylpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolylglykoletheracetal, Sorbitester, Lignin-Sulfitablaugen und Methylcellulose in Betracht.

Pulver, Streu- und Stäubmittel können durch Mischen oder gemeinsames Vermahlen von herbizidem Wirkstoff und/oder Antidot mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,01 und 95 Gew.-% an herbizidem Wirkstoff und Antidot, vorzugsweise zwischen 0,5 und 90 Gew.-%. Die Aufwandmengen an herbizidem Wirkstoff betragen 0,05 bis 5 kg/ha aktive Substanz (a.S.).

Die neuen herbiziden Mittel können neben dem substituierten 2-Aminothiophen I als Antidot und dem Herbizid aus der Gruppe der Cyclohexenone III weitere herbizide oder wachstumsregulierende Wirkstoffe anderer chemischer Struktur enthalten, wobei der antagonistische Effekt erhalten bleibt.

### Herstellungsbeispiele

### Beispiel 1

### 2-Benzoylamino-3-cyano-4,5,6,7-tetrahydro-benzo[b]thiophen

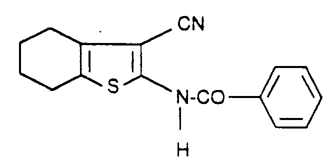

4,7 ml (40 mmol) Benzoylchlorid wurden zu einer Lösung aus 5,34 g (30 mmol) 2-Amino-3-cyano-4,5,6,7-tetrahydrobenzol-[b]thiophen in 50 ml Pyridin gegeben. Nach 2 Stunden Rühren bei 80°C versetzte man die Mischung mit 150 ml 10 mol-%iger wäßriger Salzsäure. Der gebildete Feststoff wurde wie üblich abgetrennt. Ausbeute: 97 %; Fp.: 168-170°C.

### Vorstufe 1α

### 2-Amino-3-cyano-4,5,6,7-tetrahydro-benzo[b]thiophen

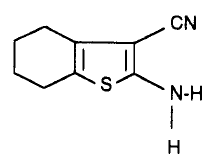

Zu einer Mischung aus 200 g (2,0 mol) Cyclohexanon, 132 g (2,0 mol) Malonsäuredinitril, 64 g (2,0 mol) Schwefel und 800 ml Ethanol wurden bei etwa 20°C langsam 200 ml (2,3 mol) Morpholin gegeben. Nach vierstündigem Rühren wurde der gebildete Feststoff wie üblich abgetrennt. Ausbeute: 51 %; Fp.: 140-141°C.

### Beispiel 2

### 2-(3,4-Dichlormaleinimino-1-yl)-3-cyano-4,5,6,7-tetrahydro-benzo[b]thiophen

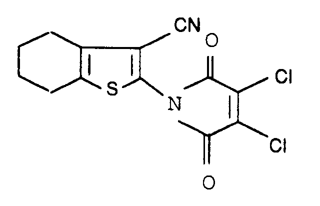

Eine Mischung aus 8,9 g (50 mmol) Amino-3-cyano-4,5,6,7-tetra∼ hydro-benzo[b]thiophen (hergestellt nach Vorstufe 1α), 8,4 g (50 mmol) 3,4-Dichlormaleinsäureanhydrid und 100 ml Eisessig wurde zwei Stunden auf Rückflugtemperatur erhitzt. Nach dem Abkühlen der Mischung trennte man das auskristallisierte Produkt wie üblich ab. Ausbeute: 85 %; Fp.: 155-160°C.

### Beispiel 3

### 2-(3-n-Butylureido)-3-cyano-4,5,6,7-tetrahydro-benzo[b]thiophen

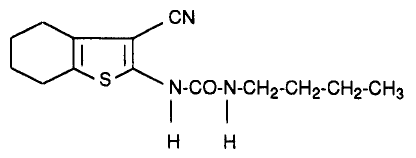

Eine Mischung aus 5,35 g (30 mmol) 2-Amino-3-cyano-4,5,6,7-tetrahydrobenzo[b]thiophen (hergestellt nach Vorstufe 1α), 2,0 ml (45 mmol) Triethylamin, 10 ml (90 mmol) n-Butylisocyanat und 100 ml Toluol wurde 16 Stunden bei 80°C gerührt. Anschließend wurde der gebildete Feststoff abgetrennt und aus Ethanol umkristalisiert. Ausbeute: 18 %; Fp.: 170-171°C.

### Beispiel 4

### 2-(2,4-Dichlorphenylmethanimino)-3-cyano-4,5,6,7-tetrahydrobenzo[b]thiophen

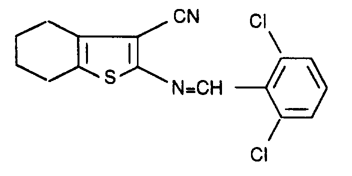

Eine Mischung aus 8,9 g (50 mmol) 2-Amino-3-cyano-4,5,6,7-tetrahydrobenzo[b]thiophen (hergestellt nach Vorstufe 1α), 8,8 g (50 mmol) 2,6-Dichlorbenzaldehyd, 0,2 g (1,1 mmol) p-Toluolsulfonsaure und 150 ml Toluol wurde unter laufender Entfernung des entstehenden Reaktionswassers 3 Stunden auf Siedetemperatur erhitzt. Nach Erkalten des Reaktionsgemisches trennte man das auskristallisierte Produkt wie üblich ab. Ausbeute: 65 %; Fp.: 182-183°C.

### Beispiel 5

### 2-Acylamino-3-cyano-4,5-benzo-6,7-dihydro-benzo(b]thiophen

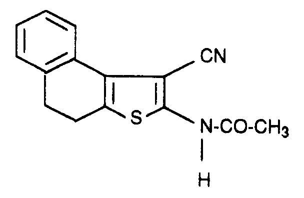

Eine Lösung aus 13,56 g (60 mmol) 2-Amino-3-cyano-4,5-benzo-6,7-dihydrobenzo[b]thiophen in 100 ml Essigsäureanhydrid wurde 4 Stunden auf Rückflußtemperatur erhitzt. Nach Abkühlung auf etwa 20°C goß man das Reaktionsgemisch in 150 ml Wasser und neutralisierte mit 80 ml konzentrierter Ammoniaklösung. Anschließend wurde das kristalline Produkt wie üblich abgetrennt. Ausbeute: 69 %; Fp.: 260-263°C.

### Vorstufe 5α

### 2-Amino-3-cyano-4,5-benzo-6,7-dihydro-benzo[b)thiophen

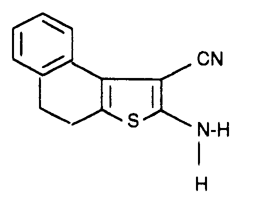

Eine Mischung aus 292 g (2,0 mol) α-Tetralon, 132 g (2,0 mol) Malonsäuredinitril, 16 g (0,2 mol) Ammoniumacetat, 50 ml Eisessig und 800 ml Toluol wurde unter laufender Entfernung des entstehenden Reaktionswassers 4 Stunden auf Siedetemperatur erhitzt und anschließend mit 200 ml Wasser ausgeschüttelt. Danach entfernte man das Lösungsmittel und versetzte den Rückstand mit 900 ml Ethanol, 49,5 g (1,5 mol) Schwefel und 155 ml (1,5 mol) Diethylamin. Das erhaltene Gemisch wurde 90 Minuten auf 60⁰C erwärmt, wonach beim Erkalten das Produkt auskristallisierte. Ausbeute: 64 %; Fp. 164-166°C.

In den folgenden Tabellen 13 und 14 sind noch weitere Verbindungen I aufgeführt, die auf die gleichen Weisen hergestellt wurden oder herstellbar sind.

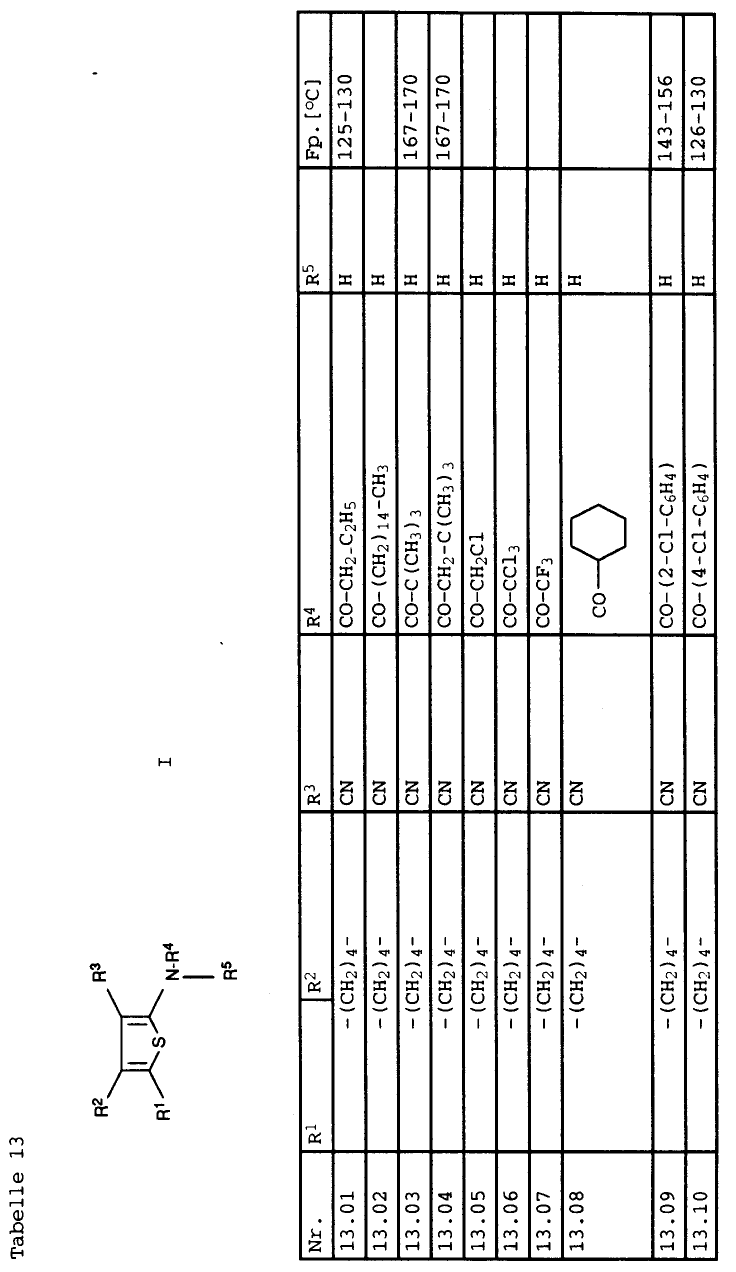

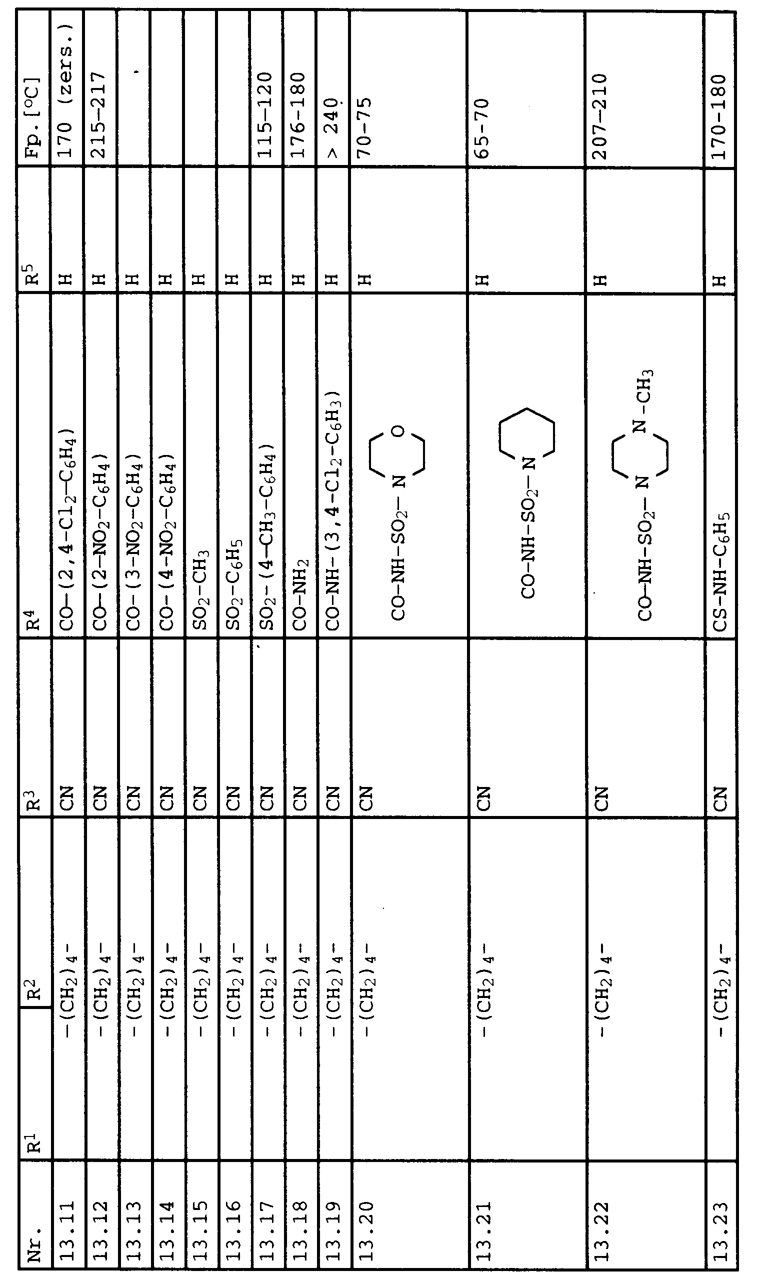

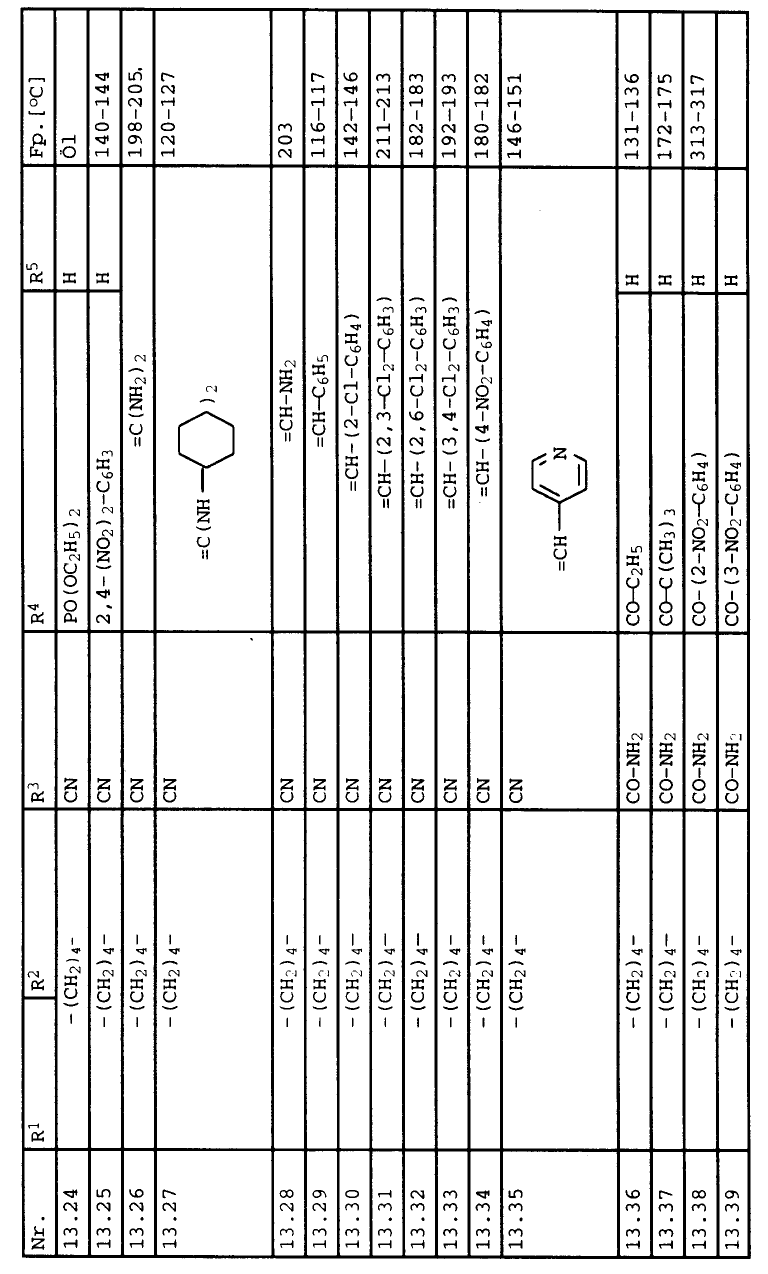

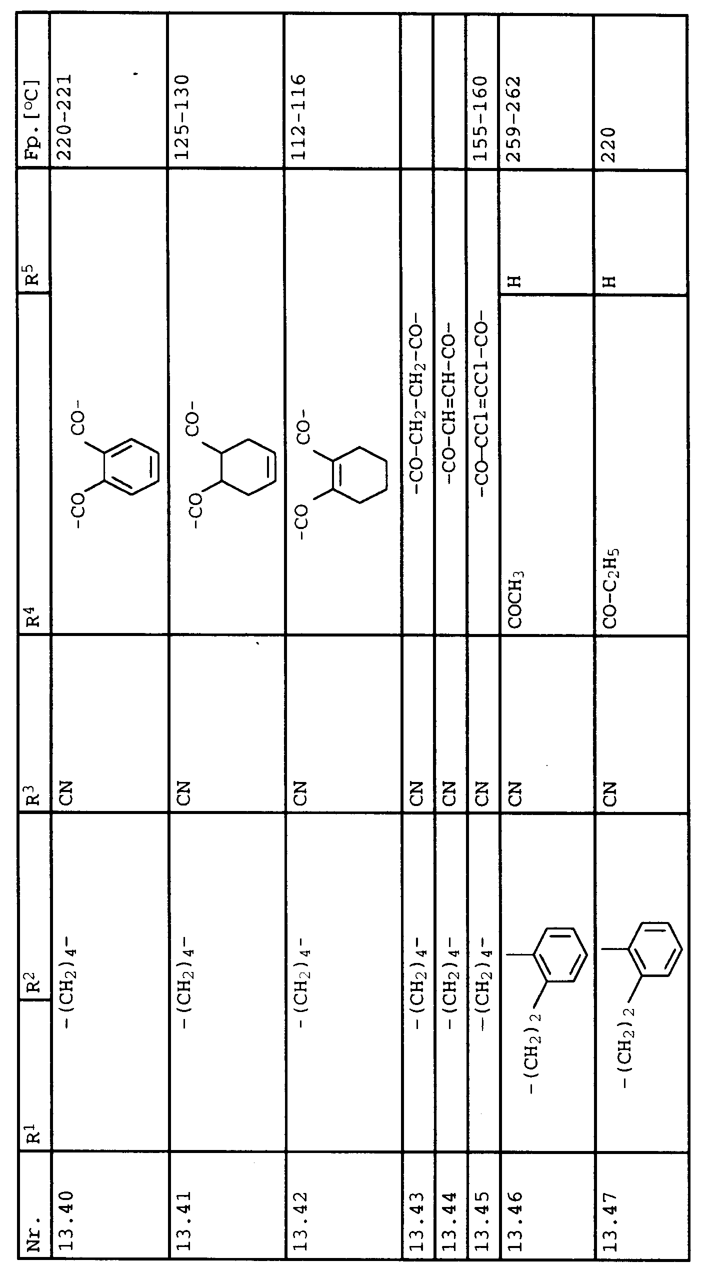

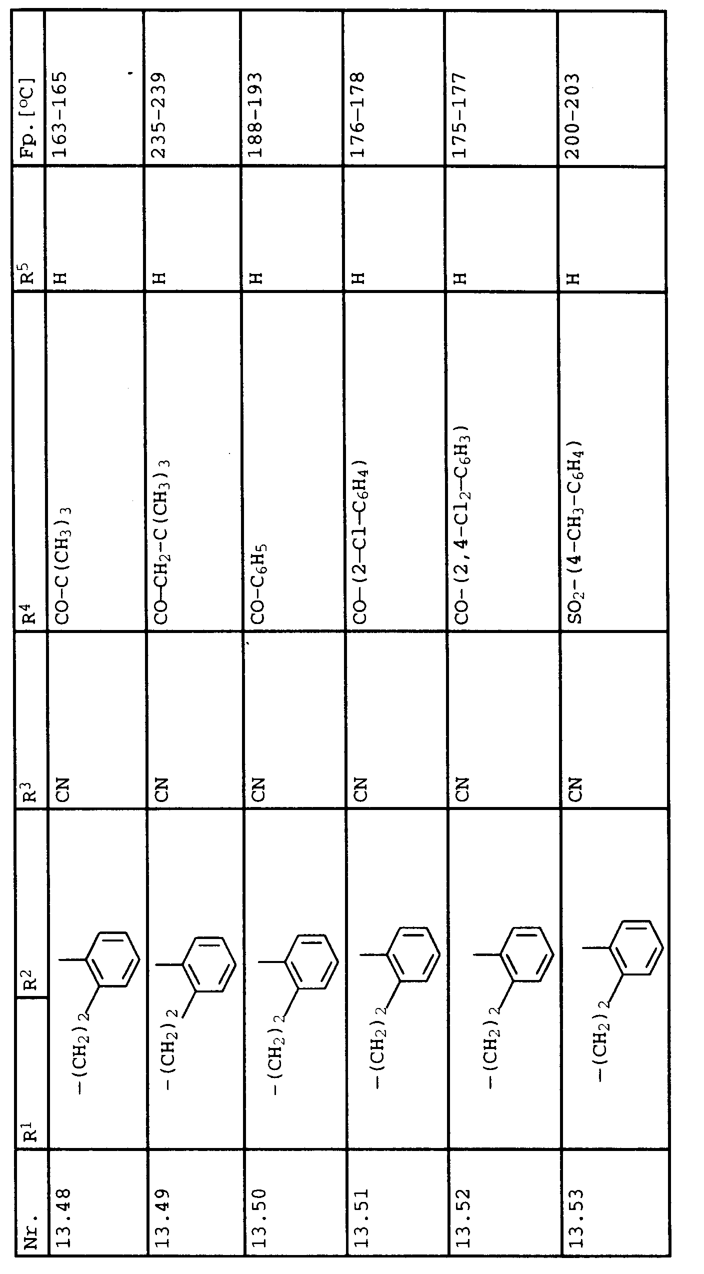

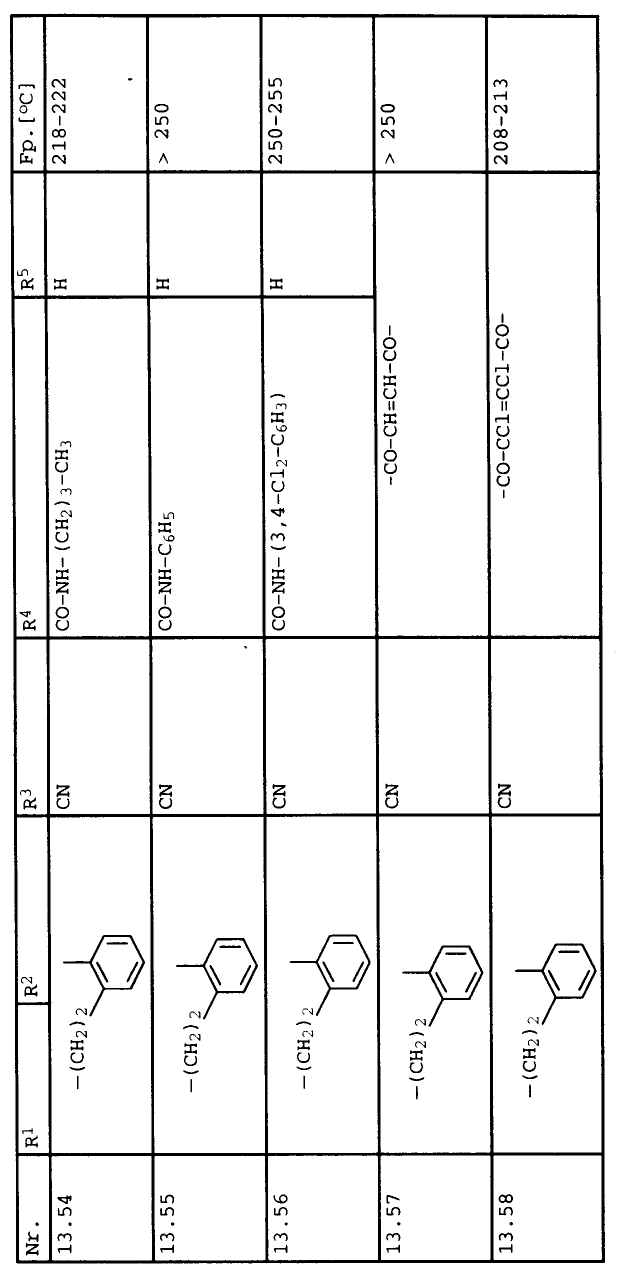

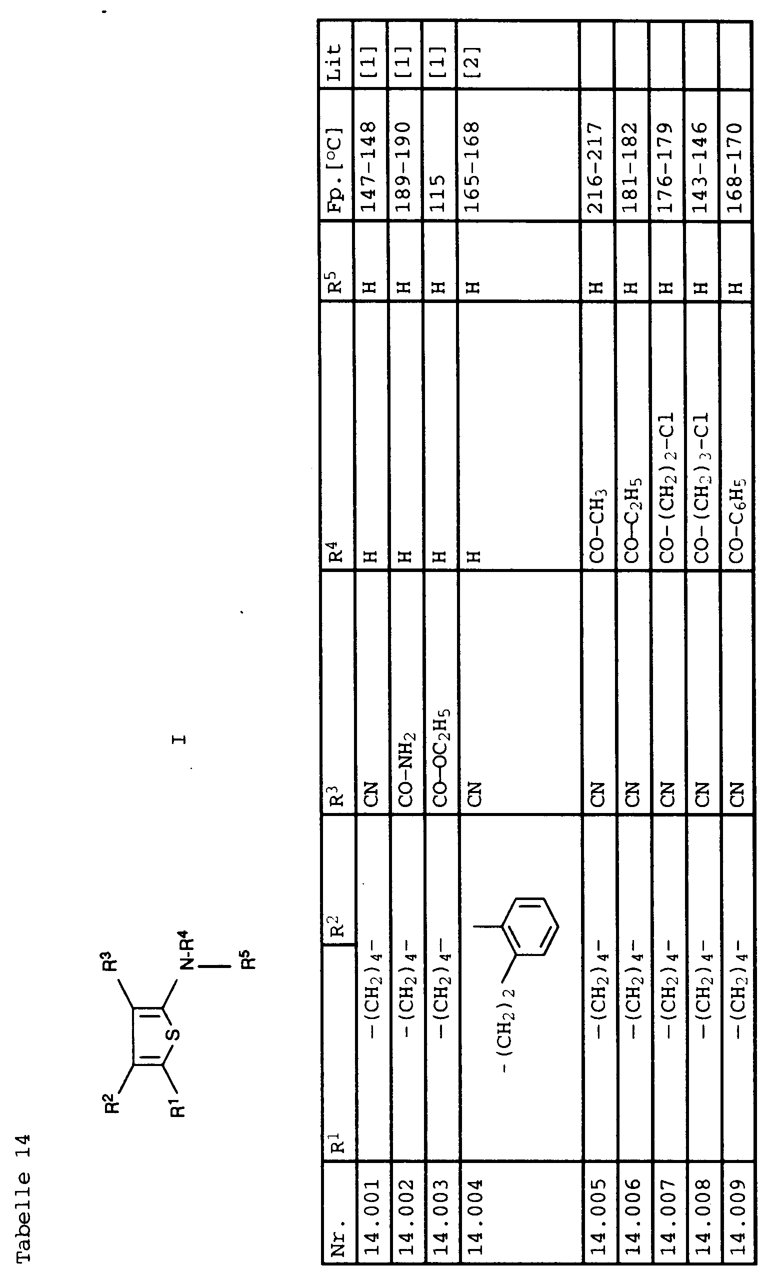

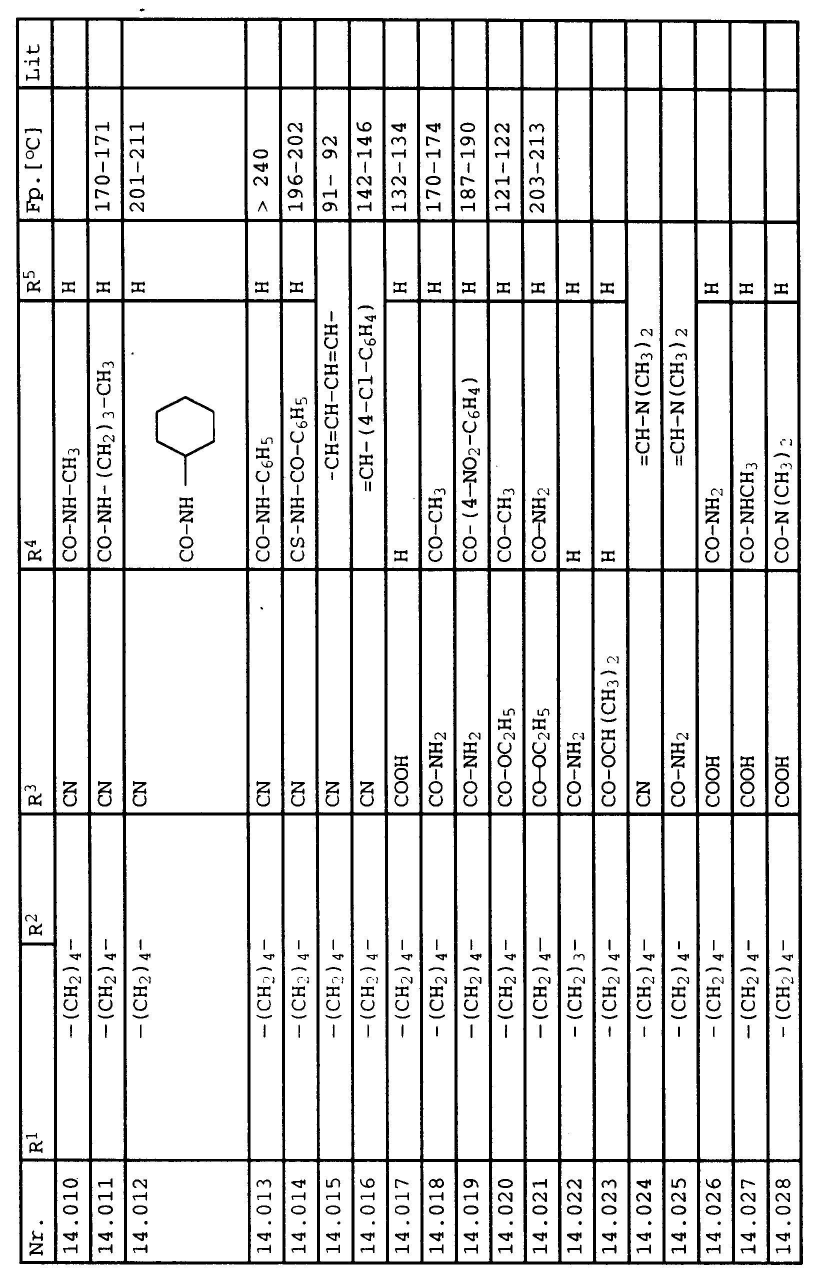

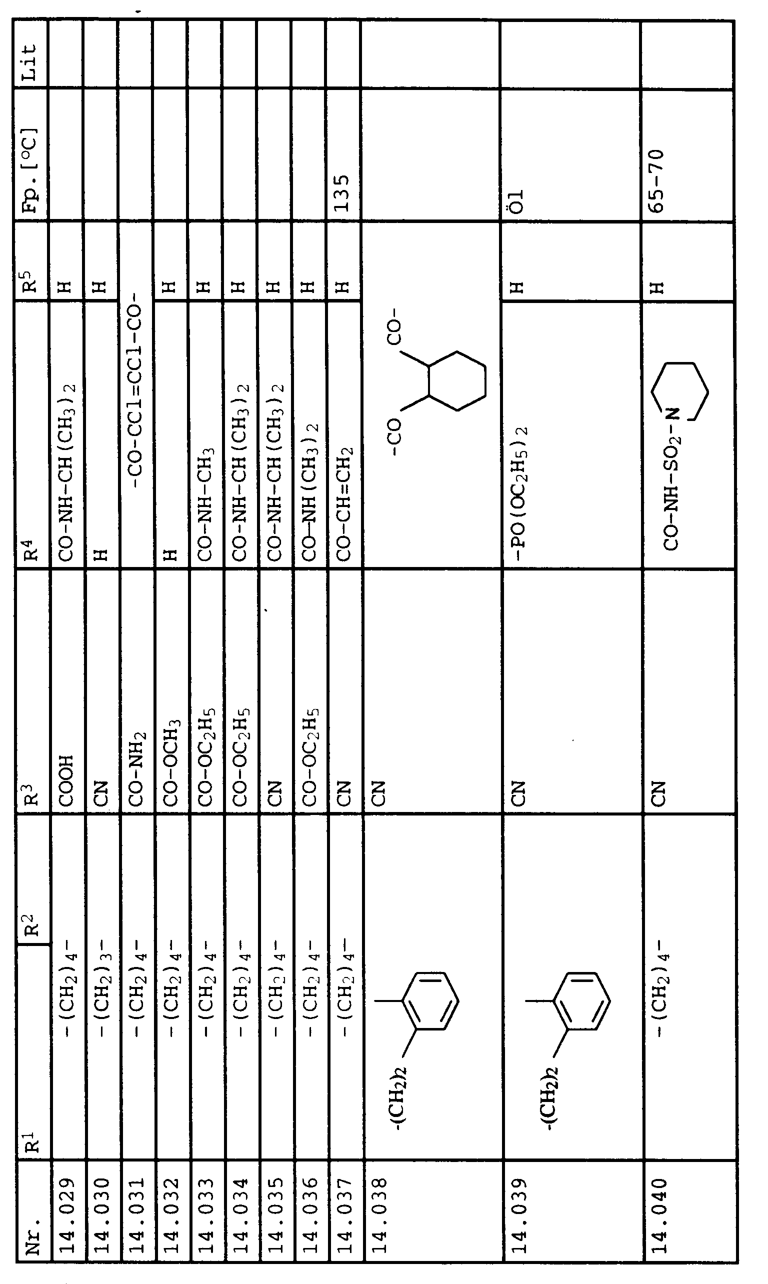

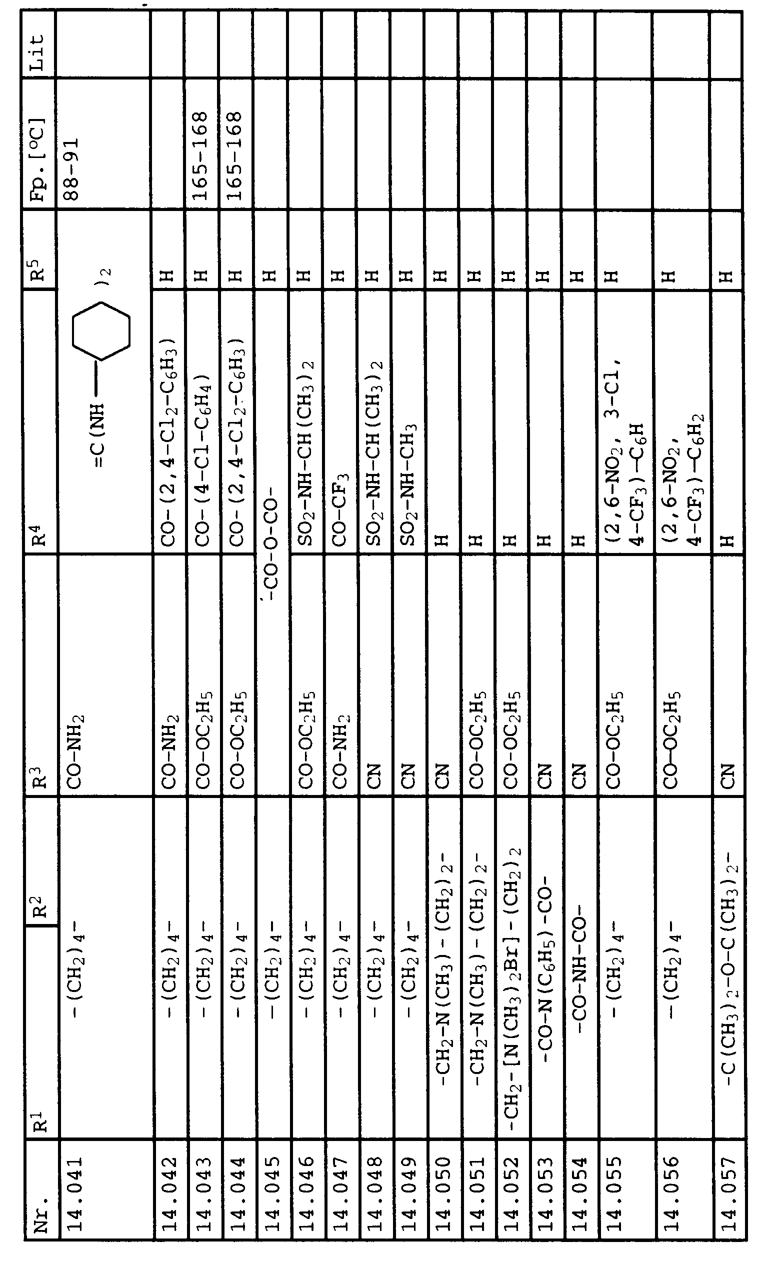

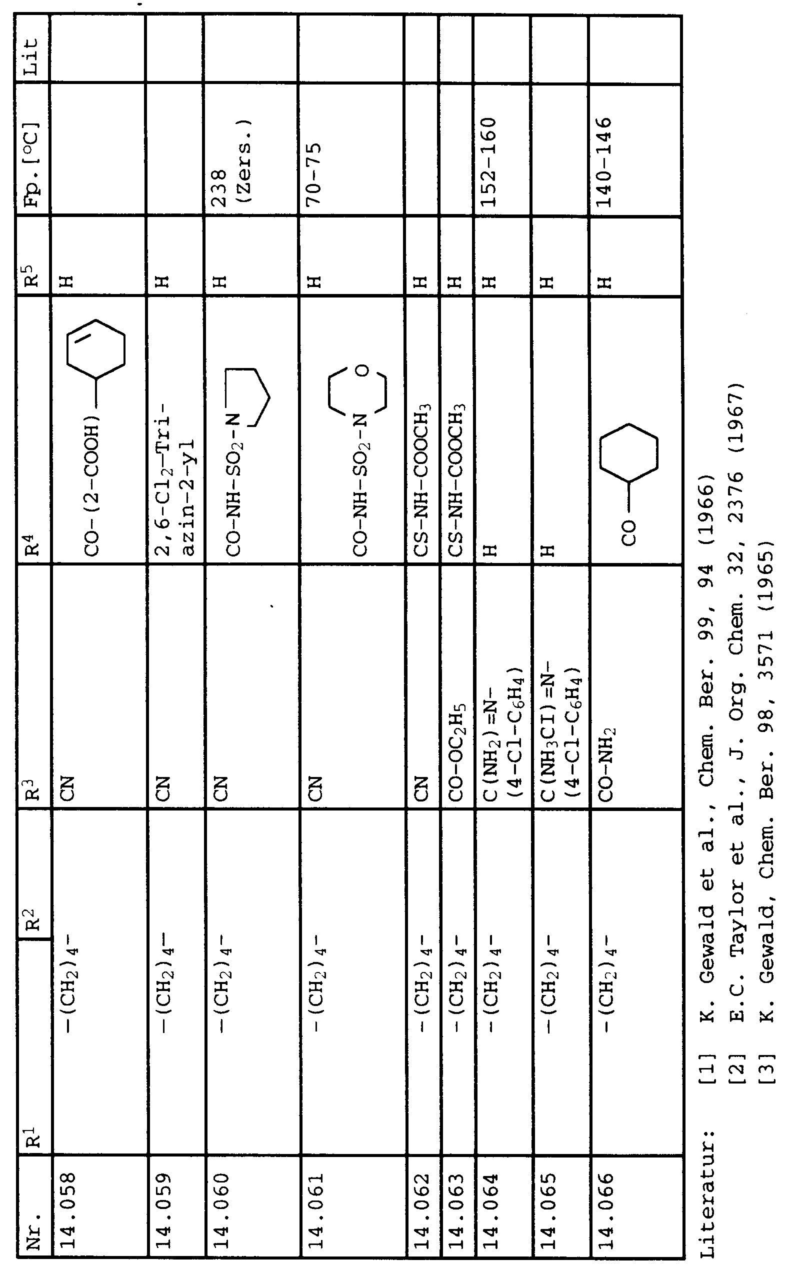

### Beispiele zur biologischen Wirkung

Der Einfluß verschiedener Vertreter der erfindungsgemäßen herbiziden Mittel bzw. Mittelkombinationen, bestehend aus Herbizid und antidotisch wirkender Verbindung, auf das Wachstum von erwünschten und unerwünschten Pflanzen im Vergleich zum herbiziden Wirkstoff allein wird durch die folgenden biologischen Beispiele aus Gewächshausversuchen belegt:

Bei Gewächshausversuchen dienten als Kulturgefäße Plastiktöpfe mit rund 300 cm³ Inhalt und lehmigem Sand mit etwa 3,0 % Humus als Substrat. Die Samen der Testpflanzen wurden nach Arten getrennt, flach eingesät und befeuchtet. Danach wurden die Gefäße mit durchsichtigen Plastikhauben abgedeckt, bis die Samen gleichmäßig gekeimt und die Pflanzen angewachsen waren.

### Liste der Testpflanzen

| Lateinischer Name | Deutscher Name | Englischer Name |
|---|---|---|
| Setaria viridis | Grüne Borstenhirse | green foxtail |
| Triticum aestivum | Weizen | wheat |
| Zea mays | Mais | Indian corn |

Für die Nachauflaufbehandlung zieht man die Testpflanzen je nach Wuchsform erst bis zu einer Wuchshöhe von 3 bis 20 cm und behandelt erst dann. Die herbiziden Mittel wurden hierbei in Wasser als Verteilungsmittel suspendiert oder emulgiert und mittels fein verteilender Düsen gespritzt.

Als Beispielherbizid der Cyclohexenonderivate der Formel III diente 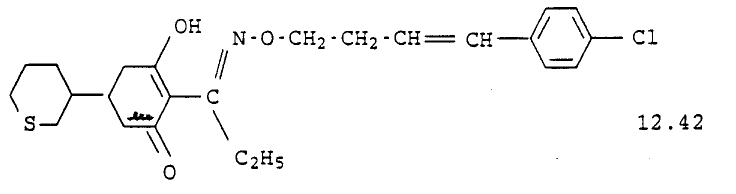 Sämtliche antidotisch wirkenden Verbindungen wurden für die Nachauflaufbehandlung in einem Gemisch, bestehend aus 80 Gew.-% Cyclohexanon als Lösungsmittel und 20 Gew.-% Tensid (Emulphor® EL*) mit 10 Gew.-% Wirkstoff aufbereitet.
*) ethoxyliertes Rizinusöl (castor oil)

Zum Vergleich wurde der herbizide Wirkstoff 12.42 als 10 bis 20 gew.-%iges Emulsionskonzentrat formuliert und jeweils unter Zugabe von derjenigen Menge an Lösungsmittelsystem in der Spritzbrühe eingesetzt, mit welcher die in den Tabellen angegebenen Aufwandmengen der antidotisch wirkenden Verbindung ausgebracht wurden. Die Herstellung der Lösung erfolgte durch Einmischen des Wirkstoffs in eine Lösung aus 93 Gew.-% Xylol und 7 Gew.-% Lutensol-AP-8 **).
**) nichtionisches oberflächenaktives Mittel auf Basis von Alkylphenolpolyethylenglykolethern

Nach Applikation der Wirkstoffmischung wurden die Testpflanzen im Gewächshaus kultiviert, und zwar wärmeliebende Arten bei etwa 18 bis 30°C, solche gemäßigterer Klimate bei ca. 10 bis 25°C.

Die Versuchsperiode erstreckte sich über 3 bis 5 Wochen. Während dieser Zeit wurden die Pflanzen gepflegt, und ihre Reaktion auf die einzelnen Behandlungen wurde erfaßt.

Bewertet wurde die Schädigung durch die herbiziden Mittel anhand einer Skala von 0 bis 100 % im Vergleich zu den unbehandelten Kontrollpflanzen. Dabei bedeutet 0 keine Schädigung und 100 eine völlige Zerstörung der Pflanzen.

Tabelle 15 dokumentiert die antidotische Wirkung der erfindungsgemäßen Beispielverbindungen Nr. 13.10, 13.25, 14.007, 14.012, 14.013, 14.015, 14.021 und 14.037.

**Tabelle 15**

| Verbesserung der Verträglichkeit des Herbizids 12.42 für Mais durch Zumischen einer antidotischen Beispielverbindung bei Nachauflaufanwendung; Gewächshausversuch | | | | |
|---|---|---|---|---|
| Antidot Nr. | Aufwandmenge [kg/ha a.S.] | | Testpflanzen und Schädigung [%] | |
| | | | Kulturpflanze | unerwünschte Pflanze |
| | Herbizid | Antidot | Mais | Setaria viridis |
| - | 0,125 | - | 85 | 100 |
| 14.012 | 0,125 | 0,125 | 25 | 95 |
| 14.013 | 0,125 | 0,125 | 20 | 80 |
| 14.037 | 0,125 | 0,125 | 25 | 90 |
| 13.10 | 0,125 | 0,125 | 30 | 100 |
| 14.007 | 0,125 | 0,125 | 30 | 90 |
| - | 0,06 | - | 60 | 95 |
| 14.021 | 0,06 | 0,06 | 30 | 80 |
| 14.015 | 0,06 | 0,06 | 25 | 70 |
| 13.25 | 0,06 | 0,06 | 15 | 70 |

Aus Tabelle 15 ist zu erkennen, daß die substituierten 2-Aminothiophene die Verträglichkeit des Herbizids 12.42 für Kulturpflanzen aus der Familie der Gramineen (Gräser) deutlich erhöhen.

## Patentansprüche

1. Herbizide Mittel, enthaltend mindestens ein substituiertes 2-Aminothiophen der allgemeinen Formel I 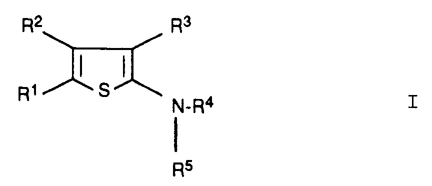 in der die Variablen die folgende Bedeutung haben:
R¹ u. R² zusammen eine C₃-C₆-Alkylenkette, an die ein Benzolring anelliert sein kann, wobei dieser Benzolring noch einen bis drei der folgenden Reste tragen kann: Halogen, C₁-C₄-Alkyl, partiell oder vollständig halogeniertes C₁-C₄-Alkyl, C₁-C₄-Alkoxy, partiell oder vollständig halogeniertes C₁-C₄-Alkoxy oder C₁-C₄-Alkylthio und der zusätzlich noch ein weiteres Halogenatom tragen kann, so daß die Gesamtzahl der Reste 4 beträgt;
eine durch Sauerstoff oder eine Gruppe-N(R⁷)- unterbrochene C₂-C₃-Alkylenkette, die noch einen bis vier C₁-C₄-Alkylreste tragen kann, wobei
R⁷ Wasserstoff, Phenyl oder C₁-C₄-Alkyl bedeutet;
eine Gruppe -CO-N(R⁷)-CO-;
R³ die Cyanogruppe oder eine Gruppe CX-R⁶, wobei X Sauerstoff oder Schwefel bedeutet und wobei R⁶ Hydroxyl, C₁-C₄-Alkyl, C₁-C₄-Alkoxy, C₁-C₄-Alkoxy-C₁-C₄-alkoxy, Amino, C₁-C₄-Alkylamino, Di-(C₁-C₄)-alkylamino, C₃-C₆-Cycloalkylamino oder Phenylamino, das noch einen bis drei Halogen-, C₁-C₄-Alkyl und/oder C₁-C₄-Alkoxyreste tragen kann, bedeutet;
R4 Wasserstoff, eine C₁-C₄-Alkylgruppe, eine C₂-C₄-Alkenylgruppe, die Phenylgruppe, die noch einen bis drei der folgenden Reste tragen kann: Nitro, Halogen, C₁-C₄-Alkyl, partiell oder vollständig halogeniertes C₁-C₄-Alkyl, C₁-C₄-Alkoxy, partiell oder vollständig halogeniertes C₁-C₄-Alkoxy oder C₁-C₄-Alkylthio und wobei die Phenylgruppe noch so viele Halogenatome tragen kann, daß die Gesamtzahl der Reste 4 oder 5 beträgt;
einen über ein C-Atom gebundenen Triazinring, der an jedem der beiden anderen C-Atome noch ein Halogenatom tragen kann;
oder eine Gruppe -PX(OR⁸)₂, -CX-R⁹, -SO₂R¹⁰, -SO₂-NHR¹⁰, CX-N(R⁷)-CY-R⁸ oder -CX-N(R⁷)-SO₂-R¹¹, wobei die Variablen die folgende Bedeutung haben:
Y Sauerstoff oder Schwefel;
R⁸ Wasserstoff, eine C₁-C₄-Alkylgruppe, die einen bis drei der folgenden Reste tragen kann: C₁-C₄-Alkyl, partiell oder vollständig halogeniertes C₁-C₄-Alkyl, C₁-C₄-Alkoxy, partiell oder vollständig halogeniertes C₁-C₄-Alkoxy oder C₁-C₄-Alkylthio;
R⁹ C₁-C₂₀-Alkyl, partiell oder vollständig halogeniertes C₁-C₆-Alkyl, C₂-C₆-Alkenyl, partiell oder vollständig halogeniertes C₂-C₆-Alkenyl, C₃-C₆-Cycloalkyl, C₅-C₆-Cycloalkenyl, wobei die cyclischen Reste noch eine Hydroxycarbonylgruppe tragen können, Amino, C₁-C₄-Alkylamino, Di-(C₁-C₄-)-alkylamino, C₃-C₈-Cycloalkylamino, Phenyl oder Phenylamino, wobei der Aromat jeweils noch einen bis drei der folgenden Substituenten tragen kann: Nitro, Halogen, C₁-C₄-Alkyl, partiell oder vollständig halogeniertes C₁-C₄-Alkyl, C₁-C₄-Alkoxy, partiell oder vollständig halogeniertes C₁-C₄-Alkoxy oder C₁-C₄-Alkylthio, und wobei der Aromat jeweils noch so viele Halogenatome zusätzlich tragen kann, daß die Gesamtzahl der Reste 4 oder 5 beträgt;
R¹⁰ C₁-C₄-Alkyl oder Phenyl, das einen bis drei der folgenden Reste tragen kann: Halogen, C₁-C₄-Alkyl, partiell oder vollständig halogeniertes C₁-C₄-Alkyl, C₁-C₄-Alkoxy, partiell oder vollständig halogeniertes C₁-C₄-Alkoxy oder C₁-C₄-Alkylthio, und das zusätzlich noch so viele Halogenatome tragen kann, daß die Gesamtzahl der Reste 4 oder 5 betragt;
R¹¹ Amino, C₁-C₄-Alkylamino, Di-(C₁-C₄)-alkyl- amino, Pyrrolidin-1-yl, Piperidin-1-yl, Morpholin-4-yl oder Piperazin-1-yl, wobei die Heterocyclen noch eine oder zwei C₁-C₄-Alkylgruppen tragen können;
oder
R³ u. R4 zusammen eine Gruppe -CO-O-CO-;
R⁵ Wasserstoff, eine C₁-C₄-Alkylgruppe oder eine Gruppe -CX-R⁹ oder -SO₂R¹⁰
oder
R⁴ u. R⁵ zusammen eine Gruppe =CR¹²R¹³ oder -CO-W-CO-, wobei die Variablen die folgende Bedeutung haben:
R¹² Wasserstoff, Amino, C₁-C₄-Alkylamino oder C₃-C₈-Cycloalkylamino;
R¹³ Amino, C₁-C₄-Alkylamino, Di-(C₁-C₄)-alkylamino, C₃-C₈-Cycloalkylamino, Thienylamino, wobei der Heteroaromat noch einen Cyano- oder Carbonylaminorest und/oder einen anellierten Benzolring tragen kann, Pyrrolidin-1-yl, Piperidin-1-yl, Morpholin-4-yl, Phenyl oder Pyridyl, wobei die beiden letztgenannten Substituenten noch einen bis drei der folgenden Reste tragen können: Halogen, Nitro, C₁-C₄-Alkyl, partiell oder vollständig halogeniertes C₁-C₄-Alkyl, C₁-C₄-Alkoxy, partiell oder vollständig halogeniertes C₁-C₄-Alkoxy oder C₁-C₄-Alkylthio;
W eine Ethylen- oder Ethenylenbrücke, eine 5- oder 6-gliedrige 1,2-C-verknüpfte aromatische oder heteroaromatische Brücke mit einem Stickstoff-, Sauerstoff oder Schwefelatom als Heteroatom, wobei diese Brückenglieder an jedem substituierbaren C-Atom noch einen Rest tragen können, ausgewählt aus einer Gruppe von bis zu 2 der folgenden Reste: Halogen, C₁-C₄-Alkyl, partiell oder vollständig halogeniertes C₁-C₄-Alkyl, C₁-C₄-Alkoxy, partiell oder vollständig halogeniertes C₁-C₄-Alkoxy, C₁-C₄-Alkylthio oder partiell oder vollständig halogeniertes C₁-C₄-Alkylthio, und wobei die Brückenglieder zusätzlich noch so viele Halogenatome tragen können, wie weitere substituierbare C-Atome vorhanden sind; oder
eine 5- oder 6-gliedrige 1,2-C-verknüpfte Cycloalkylen- oder Cycloalkenylenbrücke, wobei diese Substituenten noch einen bis vier der folgenden Reste tragen können: Halogen oder C₁-C₄-Alkyl,
sowie die basischen Salze der Verbindungen I, bei denen R³ eine Hydroxycarbonyl- oder Hydroxythiocarbonylgruppe bedeutet und die sauren Salze derjenigen Verbindungen I, die ein basisches Stickstoffatom enthalten, als antagonistisch wirksame Verbindung
und mindestens einen herbiziden Wirkstoff aus der Gruppe der Cyclohexenonderivate der Formel III 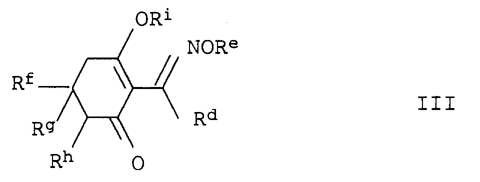 wobei die Substituenten folgende Bedeutung haben:
R^{d} eine C₁-C₄-Alkylgruppe;
R^{e} eine C₁-C₄-Alkylgruppe, eine C₃-C₄-Alkenylgruppe, eine C₃-C₄-Alkinylgruppe oder eine partiell oder vollständig halogenierte C₃-C₄-Alkenylgruppe;
eine C₁-C₄-Alkylen- oder C₂-C₄-Alkenylenkette, die beide noch ein bis drei C₁-C₃-Alkylreste und/oder Halogenatome tragen können, oder eine gewünschtenfalls durch C₁-C₃-Alkyl substituierte 3- bis 6-gliedrige Alkylen- oder 4- bis 6-gliedrige Alkenylenkette, die jeweils ein dem Oximetherteil nicht direkt benachbartes Sauerstoff- oder Schwefelatom als Kettenglied enthält, wobei alle vorstehend genannten Ketten endständig den Phenylring tragen, der seinerseits durch ein bis drei Reste substituiert sein kann, ausgewählt aus einer Gruppe bestehend aus einem Benzyloxycarbonyl- oder Phenylrest und jeweils einem bis drei der folgenden Reste:
Nitro, Cyano, C₁-C₄-Alkyl, C₁-C₄-Alkoxy, C₁-C₄- Alkylthio, partiell oder vollständig halogeniertes C₁-C₄-Alkyl, partiell oder vollständig halogeniertes C₁-C₄-Alkoxy, Carboxyl und C₁-C₄-Alkoxycarbonyl, und wobei der Phenylring noch zusätzlich so viele Halogenatome tragen kann, daß die Gesamtzahl der Reste 4 oder 5 beträgt;
eine Thienylmethylgruppe, die noch ein Halogenatom tragen kann;
R^{f} eine C₁-C₄-Alkylgruppe, welche einfach oder zweifach durch C₁-C₄-Alkylthio oder C₁-C₄-Alkoxy substituiert sein kann;
ein 5- oder 6-gliedriges gesättigtes oder einfach ungesättigtes Ringsystem, welches neben Kohlenstoffgliedern ein Sauerstoff-, ein Schwefelatom oder eine Sulfoxid- oder Sulfongruppe enthalten kann, wobei dieser Ring bis zu drei der folgenden Reste tragen kann: Hydroxy, Halogen, C₁-C₄-Alkyl, partiell oder vollständig halogeniertes C₁-C₄- Alkyl, C₁-C₄-Alkoxy und/oder C₁-C₄-Alkylthio;
einen 10-gliedrigen gesättigten oder einfach ungesättigten Heterocyclus, welcher zwei Sauerstoffatome oder Schwefelatome enthält und durch ein bis drei C₁-C₄-Alkylgruppen und/oder Methoxygruppen substituiert sein kann;
die Phenylgruppe, die Pyridylgruppe, die Pyrrolylgruppe, die Pyrazolyl-, Thiazolyl- oder Isoxazolylgruppe, wobei diese Gruppen gewünschtenfalls einen bis drei der folgenden Reste tragen können: C₁-C₄-Alkyl, partiell oder vollständig halogeniertes C₁-C₄-Alkyl, C₁-C₄-Alkoxy, C₁-C₄-Alkylthio, C₃-C₆-Alkenyloxy, C₃-C₆-Alkinyloxy, C₁-C₄-Alkoxy-C₁-C₃-alkyl, C₁-C₄-Alkylthio-C₁-C₃-alkyl, C₁-C₄-Dialkoxy-C₁-C₃-alkyl, Formyl, Halogen und/oder Benzoylamino;
R^{g} Wasserstoff, Hydroxy oder, wenn R^{f} die Bedeutung C₁-C₆-Alkylgruppe hat, eine C₁-C₆-Alkylgruppe;
R^{h} Wasserstoff, die Cyanogruppe, ein Halogenatom, eine C₁-C₄-Alkoxycarbonylgruppe oder eine Gruppe 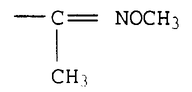 und
Rⁱ Wasserstoff oder das Äquivalent eines landwirtschaftlich brauchbaren Kations.

2. Herbizide Mittel nach Anspruch 1, enthaltend ein substituiertes 2-Aminothiophen I als antagonistisch wirksame Verbindung und ein Herbizid III im Gewichtsverhältnis 0,01 : 1 bis 10 : 1.

3. Verfahren zur selektiven Bekämpfung von unerwünschtem Pflanzenwuchs, dadurch gekennzeichnet, daß man ein substituiertes 2-Aminothiophen I als antagonistisch wirksame Verbindung und ein Cyclohexenonderivat der Formel III gemäß Anspruch 1 vor, bei oder nach der Aussaat der Kulturpflanzen, vor oder während des Auflaufens der Kulturpflanzen gleichzeitig oder nacheinander ausbringt.

4. Verfahren zur selektiven Bekämpfung von unerwünschtem Pflanzenwuchs, dadurch gekennzeichnet, daß man die Blätter der Kulturpflanzen und der unerwünschten Pflanzen im Nachauflaufverfahren mit einem substituiertem 2-Aminothiophen I als antagonistisch wirksame Verbindung und mit einem Cyclohexenonderivat der Formel III gemäß Anspruch 1 gleichzeitig oder nacheinander behandelt.

5. Verfahren zur Verhinderung von Schädigungen von Kulturpflanzen durch herbizide Cyclohexenonderivate der Formel III gemäß Anspruch 1, dadurch gekennzeichnet, daß man das Saatgut der Kulturpflanzen mit einer antagonistisch wirksamen Menge eines substituierten 2-Aminothiophen der Formel I gemäß Anspruch 1 behandelt.

6. Verfahren gemäß den Ansprüchen 3 bis 5, dadurch gekennzeichnet, daß die Kulturpflanzen Gerste, Weizen, Mais, Kultursorghum und Reis sind.

## Claims

1. A herbicide containing one or more substituted 2-aminothiophenes of the formula I 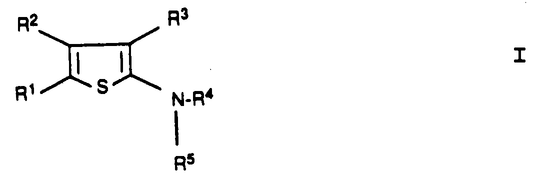 where
R¹ and R² together form a C₃-C₆-alkylene chain to which a benzene ring may be fused, and this benzene ring may carry from one to three of the following radicals: halogen, C₁-C₄-alkyl, partially or completely halogenated C₁-C₄-alkyl, C₁-C₄-alkoxy, partially or completely halogenated C₁-C₄-alkoxy or C₁-C₄-alkylthio, and may additionally carry a further halogen atom so that the total number of radicals is 4;
a C₂- or C₃-alkylene chain which is interrupted by oxygen or by a group -N(R⁷)- and which may carry from one to four C₁-C₄-alkyl radicals, where
R⁷ is hydrogen, phenyl or C₁-C₄-alkyl;
-CO-N(R⁷)-CO-;
R³ is cyano or CX-R⁶, where X is oxygen or sulfur and where R⁶ is hydroxyl, C₁-C₄-alkyl, C₁-C₄-alkoxy, C₁-C₄-alkoxy-C₁-C₄-alkoxy, amino, C₁-C₄-alkylamino, di-C₁-C₄-alkylamino, C₃-C₆-cycloalkylamino or phenylamino, which may furthermore carry from one to three halogen, C₁-C₄-alkyl and/or C₁-C₄-alkoxy radicals;
R⁴ is hydrogen, C₁-C₄-alkyl, C₂-C₄-alkenyl or a phenyl group which may carry from one to three of the following radicals: nitro, halogen, C₁-C₄-alkyl, partially or completely halogenated C₁-C₄-alkyl, C₁-C₄-alkoxy, partially or completely halogenated C₁-C₄-alkoxy or C₁-C₄-alkylthio, and which may carry a number of halogen atoms so that the total number of radicals is 4 or 5;
a triazine ring which is bonded via a carbon atom and may carry a halogen atom on each of the other two carbon atoms, or
-PX(OR⁸)₂, -CX-R⁹, -SO₂R¹⁰, -SO₂-NHR¹⁰, -CX-N(R⁷)-CY-R⁸ or -CX-N(R⁷)-SO₂-R¹¹, where
Y is oxygen or sulfur;
R⁸ is hydrogen or a C₁-C₄-alkyl which may carry from one to three of the following radicals: C₁-C₄-alkyl, partially or completely halogenated C₁-C₄-alkyl, C₁-C₄-alkoxy, partially or completely halogenated C₁-C₄-alkoxy or C₁-C₄-alkylthio;
R⁹ is C₁-C₂₀-alkyl, partially or completely halogenated C₁-C₆-alkyl, C₂-C₆-alkenyl, partially or completely halogenated C₂-C₆-alkenyl, C₃-C₆-cycloalkyl, C₅- or C₆-cycloalkenyl, where the cyclic radicals may carry a hydroxycarbonyl group, or amino, C₁-C₄-alkylamino, di-C₁-C₄-alkylamino, C₃-C₈-cycloalkylamino, phenyl or phenylamino, where the aromatic moieties may each carry from one to three of the following substituents: nitro, halogen, C₁-C₄-alkyl, partially or completely halogenated C₁-C₄-alkyl, C₁-C₄-alkoxy, partially or completely halogenated C₁-C₄-alkoxy or C₁-C₄-alkylthio, and where the aromatic moieties may each additionally carry a number of halogen atoms so that the total number of radicals is 4 or 5;
R¹⁰ is C₁-C₄-alkyl or phenyl, which may carry from one to three of the following radicals: halogen, C₁-C₄-alkyl, partially or completely halogenated C₁-C₄-alkyl, C₁-C₄-alkoxy, partially or completely halogenated C₁-C₄-alkoxy or C₁-C₄-alkylthio, and which may additionally carry a number of halogen atoms so that the total number of radicals is 4 or 5;
R¹¹ is amino, C₁-C₄-alkylamino, di-C₁-C₄-alkylamino, pyrrolidin-1-yl, piperidin-1-yl, morpholin-4-yl or piperazin-1-yl, where the heterocyclic structures may carry one or two C₁-C₄-alkyl groups,
or
R³ and R⁴ together form a group -CO-O-CO-;
R⁵ is hydrogen, C₁-C₄-alkyl, -CX-R⁹ or -SO₂R¹⁰, or
R⁴ and R⁵ together form a group =CR¹²R¹³ or -CO-W-CO-,
where
R¹² is hydrogen, amino, C₁-C₄-alkylamino or C₃-C₈-cycloalkylamino;
R¹³ is amino, C₁-C₄-alkylamino, di-C₁-C₄-alkylamino, C₃-C₈-cycloalkylamino, thienylamino, where the heteroaromatic may carry a cyano or carbonylamino radical and/or a fused benzene ring, or pyrrolidin-1-yl, piperidin-1-yl, morpholin-4-yl, phenyl or pyridyl, where the two last-mentioned substituents may carry from one to three of the following radicals: halogen, nitro, C₁-C₄-alkyl, partially or completely halogenated C₁-C₄-alkyl, C₁-C₄-alkoxy, partially or completely halogenated C₁-C₄-alkoxy or C₁-C₄-alkylthio;
W is an ethylene or ethenylene bridge, a 5-membered or 6-membered 1,2-C-bonded aromatic or heteroaromatic bridge having a nitrogen, oxygen or sulfur atom as a hetero atom, where these bridge members may carry, on each substitutable carbon atom, a radical selected from up to 2 of the following: halogen, C₁-C₄-alkyl, partially or completely halogenated C₁-C₄-alkyl, C₁-C₄-alkoxy, partially or completely halogenated C₁-C₄-alkoxy, C₁-C₄-alkylthio or partially or completely halogenated C₁-C₄-alkylthio, and where the bridge members may additionally carry a number of halogen atoms corresponding to the number of further substitutable carbon atoms, or
a 5-membered or 6-membered 1,2-C-bonded cycloalkylene or cycloalkenylene bridge, where these substituents may carry from one to four of the following radicals: halogen or C₁-C₄-alkyl,
and the basic salts of the compounds I in which R³ is hydroxycarbonyl or hydroxythiocarbonyl, and the acidic salts of the compounds I which contain a basic nitrogen atom, as antagonistic compounds
and one or more herbicidal active ingredients from the group consisting of the cyclohexenone derivatives of the formula III 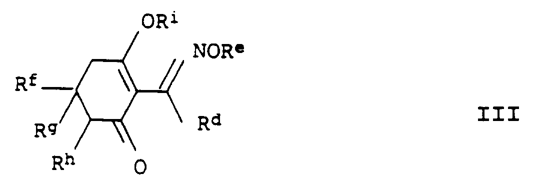 where
R^{d} is C₁-C₄-alkyl;
R^{e} is C₁-C₄-alkyl, C₃- or C₄-alkenyl, C₃- or C₄-alkynyl or partially or completely halogenated C₃- or C₄-alkenyl;
a C₁-C₄-alkylene or C₂-C₄-alkenylene chain, both of which may furthermore carry from one to three C₁-C₃-alkyl radicals and/or halogen atoms, or a 3-membered to 6-membered alkylene or 4-membered to 6-membered alkenylene chain which is, if desired, substituted by C₁-C₃-alkyl and each of which contains, as a chain member, an oxygen or sulfur atom which is not directly adjacent to the oxime ether moiety, all abovementioned chains carrying the terminal phenyl ring which in turn may be substituted by from one to three radicals selected from a group consisting of a benzyloxycarbonyl or phenyl radical and from one to three of the following radicals:
nitro, cyano, C₁-C₄-alkyl, C₁-C₄-alkoxy, C₁-C₄-alkylthio, partially or completely halogenated C₁-C₄-alkyl, partially or completely halogenated C₁-C₄-alkoxy, carboxyl and C₁-C₄-alkoxycarbonyl, and it being possible for the phenyl ring additionally to carry a number of halogen atoms such that the total number of radicals is 4 or 5;
thienylmethyl which may carry a halogen atom;
R^{f} is C₁-C₄-alkyl which may be monosubstituted or disubstituted by C₁-C₄-alkylthio or by C₁-C₄-alkoxy;
a 5-membered or 6-membered saturated or monounsaturated ring system which, in addition to carbon members, may contain an oxygen or sulfur atom or a sulfoxide or sulfone group, where this ring may carry up to 3 of the following radicals: hydroxyl, halogen, C₁-C₄-alkyl, partially or completely halogenated C₁-C₄-alkyl, C₁-C₄-alkoxy and/or C₁-C₄-alkylthio;
a 10-membered saturated or monounsaturated heterocyclic structure which contains two oxygen atoms or sulfur atoms and may be substituted by one to three C₁-C₄-alkyl groups and/or methoxy groups;
phenyl, pyridyl, pyrrolyl, pyrazolyl, thiazolyl or isoxazolyl, where these groups may, if desired, carry from one to three of the following radicals: C₁-C₄-alkyl, partially or completely halogenated C₁-C₄-alkyl, C₁-C₄-alkoxy, C₁-C₄-alkylthio, C₃-C₆-alkenyloxy, C₃-C₆-alkynyloxy, C₁-C₄-alkoxy-C₁-C₃-alkyl, C₁-C₄-alkylthio-C₁-C₃-alkyl, C₁-C₄-dialkoxy-C₁-C₃-alkyl, formyl, halogen and/or benzoylamino;
R^{g} is hydrogen or hydroxyl or, when R^{f} is C₁-C₆-alkyl, is C₁-C₆-alkyl;
R^{h} is hydrogen, cyano, halogen, C₁-C₄-alkoxycarbonyl or a group 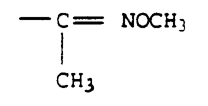 and
Rⁱ is hydrogen or one equivalent of an agriculturally useful cation.

2. A herbicide as claimed in claim 1, containing a substituted 2-aminothiophene I as an antagonistic compound and a herbicide III in a weight ratio of from 0.01 : 1 to 10 : 1.

3. A method for selectively controlling undesirable plant growth, wherein a substituted 2-aminothiophene I, as an antagonistic compound, and a cyclohexenone derivative of the formula III as claimed in claim 1 are applied, simultaneously or in succession, before, during or after sowing of the crops or before or during emergence of the crops.

4. A method for selectively controlling undesirable plant growth, wherein the leaves of the crops and of the undesirable plants are treated simultaneously or in succession, by the postemergence method, with a substituted 2-aminothiophene I, as an antagonistic compound, and with a cyclohexenone derivative of the formula III as claimed in claim 1.

5. A method for preventing damage to crops by herbicidal cyclohexenone derivatives of the formula III as claimed in claim 1, wherein the seed of the crops is treated with an antagonistic amount of a substituted 2-aminothiophene of the formula I as claimed in claim 1.

6. A method as claimed in claim 3 or 4 or 5, wherein the crops are barley, wheat, corn, sorghum and rice.

## Revendications

1. Produit herbicide contenant au moins un 2-aminothiophène substitué répondant à la formule générale I 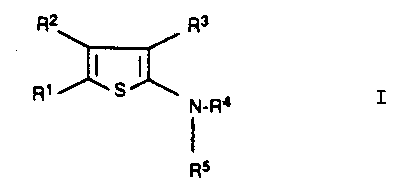
dans laquelle les symboles ont les significations suivantes :
R¹ et R² forment ensemble une chaîne alkylène en C3-C6 sur laquelle un cycle benzénique peut être condensé, ce cycle benzénique pouvant encore porter un à trois des substituants suivants : halogéno, alkyle en C1-C4, alkyle en C1-C4 partiellement ou totalement halogéné, alcoxy en C1-C4, alcoxy en C1-C4 partiellement ou totalement halogéné ou alkylthio en C1-C4, et encore un autre atome d'halogène, en sorte que le nombre total des substituants soit de 4 ;
une chaîne alkylène en C2-C3 interrompue par l'oxygène ou par un groupe -N(R⁷)- et qui peut encore porter un à quatre groupes alkyle en C1-C4,
R⁷ représentant l'hydrogène, un groupe phényle ou alkyle en C1-C4 ;
un groupe -CO-N(R⁷)-CO- ;
R³ : le groupe cyano ou un groupe CX-R⁶ dans lequel X représente l'oxygène ou le soufre et R⁶ un groupe hydroxy, alkyle en C1-C4, alcoxy en C1-C4, (alcoxy en C1-C4)-alcoxy en C1-C4, amino, alkylamino en C1-C4, di-(alkyle en C1-C4)amino, cycloalkylamino en C3-C6 ou phénylamino qui peut encore porter un à trois substituants halogéno, alkyle en C1-C4 et/ou alcoxy en C1-C4 ;
R⁴ : l'hydrogène, un groupe alkyle en C1-C4, un groupe alcényle en C2-C4, le groupe phényle qui peut encore porter un à trois des substituants suivants : nitro, halogéno, alkyle en C1-C4, alkyle en C1-C4 partiellement ou totalement halogéné, alcoxy en C1-C4, alcoxy en C1-C4 partiellement ou totalement halogéné ou alkylthio en C1-C4, le groupe phényle pouvant encore porter des atomes d'halogènes en nombre tel que le nombre total des substituants soit de 4 ou 5 ;
un cycle triazine relié par l'intermédiaire d'un atome de carbone et qui peut encore porter un atome d'halogène sur chacun des deux autres atomes de carbone ;
ou bien un groupe -PX(OR⁸)₂, -CX-R⁹, -SO₂R¹⁰, -SO₂-NHR¹⁰, -CX-N(R⁷)-CY-R⁸ ou -CX-N(R⁷)-SO₂-R¹¹, dans lequel les symboles ont les significations suivantes :
Y : l'oxygène ou le soufre ;
R⁸ : l'hydrogène, un groupe alkyle en C1-C4 qui peut porter un à trois des substituants suivants : alkyle en C1-C4, alkyle en C1-C4 partiellement ou totalement halogéné, alcoxy en C1-C4, alcoxy en C1-C4 partiellement ou totalement halogéné ou alkylthio en C1-C4 ;
R⁹ : un groupe alkyle en C1-C20, alkyle en C1-C6 partiellement ou totalement halogéné, alcényle en C2-C6, alcényle en C2-C6 partiellement ou totalement halogéné, cycloalkyle en C3-C6, cycloalcényle en C5-C6, les radicaux cycliques pouvant encore porter un groupe hydroxycarbonyle, amino, alkylamino en C1-C4, di-(alkyle en C1-C4)amino, cycloalkylamino en C3-C8, phényle ou phénylamino, la partie aromatique pouvant encore, dans chaque cas, porter un à trois des substituants suivants : nitro, halogéno, alkyle en C1-C4, alkyle en C1-C4 partiellement ou totalement halogéné alkoxy en C1-C4, alkoxy en C1-C4 partiellement ou totalement halogéné ou alkylthio en C1-C4, la partie aromatique pouvant encore, dans chaque cas, porter des atomes d'halogènes en nombre tel que le nombre total des substituants soit de 4 ou 5 ;
R¹⁰ : un groupe alkyle en C1-C4 ou phényle qui peut porter un à trois des substituants suivants : halogéno, alkyle en C1-C4, alkyle en C1-C4 partiellement ou totalement halogéné, alcoxy en C1-C4, alcoxy en C1-C4 partiellement ou totalement halogéné ou alkylthio en C1-C4, et qui peut encore porter des atomes d'halogènes en nombre tel que le nombre total des substituants soit de 4 ou 5 ;
R¹¹ : un groupe amino, alkylamino en C1-C4, di-(alkyle en C1-C4)amino, pyrrolidin-1-yle, pipéridin-1-yle, morpholin-4-yle ou pipérazin-1-yle, les hétérocycles pouvant encore porter un ou deux groupes alkyle en C1-C4 ;
ou bien
R³ et R⁴ forment ensemble un groupe -CO-O-CO- ;
R⁵ représente l'hydrogène, un groupe alkyle en C1-C4 ou un groupe -CX-R⁹ ou -SO₂R¹⁰,
ou bien
R⁴ et R⁵ forment ensemble un groupe =CR¹²R¹³ ou -CO-W-CO- dans lequel les symboles ont les significations suivantes :
R¹² : l'hydrogène, un groupe amino, alkylamino en C1-C4 ou cycloalkylamino en C3-C8 ;
R¹³ : un groupe amino, alkylamino en C1-C4, di-(alkyle en C1-C4)amino, cycloalkylamino en C3-C8, thiénylamino, la partie hétéroaromatique pouvant encore porter un groupe cyano ou carbonylamino et/ou un cycle benzénique condensé, un groupe pyrrolidin-1-yle, pipéridin-1-yle, morpholin-4-yle, phényle ou pyridyle, ces deux derniers substituants pouvant encore porter un à trois des substituants suivants : halogéno, nitro, alkyle en C1-C4, alkyle en C1-C4 partiellement ou totalement halogéné, alcoxy en C1-C4, alcoxy en C1-C4 partiellement ou totalement halogéné ou alkylthio en C1-C4 ;
W : un pont éthylène ou éthénylène, un pont aromatique ou hétéroaromatique à cinq ou six chaînons relié en position 1,2-C, contenant en tant qu'hétéroatome l'azote, l'oxygène ou le soufre, ces ponts pouvant encore porter, sur chaque atome de carbone substituable, un substituant choisi dans un groupe allant jusqu'a deux des substituants suivants : halogéno, alkyle en C1-C4, alkyle en C1-C4 partiellement ou totalement halogéné, alcoxy en C1-C4, alcoxy en C1-C4 partiellement ou totalement halogéné, alkylthio en C1-C4 ou alkylthio en C1-C4 partiellement ou totalement halogéné, les ponts pouvant encore porter des atomes d'halogènes dans la mesure où il subsiste d'autres atomes de carbone substituables ; ou bien
un pont cycloalkylène ou cycloalcénylène à cinq ou six chaînons, relié en position 1,2-C, ce pont pouvant encore porter un à quatre des substituants suivants halogéno ou alkyle en C1-C4,
ainsi que les sels basiques des composés I pour lesquels R³ représente un groupe hydroxy-carbonyle ou hydroxythiocarbonyle, et les sels acides des composés I qui contiennent un atome d'azote basique, en tant que composé à effet antagoniste,
et au moins une substance active herbicide prise dans le groupe des dérivés de cyclohexénones de formule III 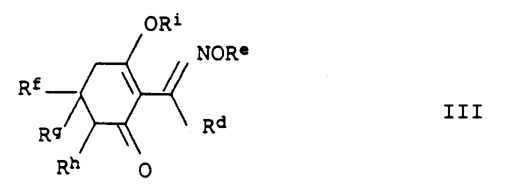
dans laquelle les symboles ont les significations suivantes :
R^{d} : un groupe alkyle en C1-C4 ;
R^{e} : un groupe alkyle en C1-C4, alcényle en C3-C4, alcynyle en C3-C4 ou un groupe alcényle en C3-C4 partiellement ou totalement halogéné ;
une chaîne alkylène en C1-C4 ou alcénylène en C2-C4 qui peut porter un à trois groupes alkyle en C1-C3 et/ou atomes d'halogènes, ou bien une chaîne alkylène de trois à six chaînons ou alcénylène de quatre à six chaînons éventuellement substituée par un groupe alkyle en C1-C3 et qui contient, en tant que chaînon, un atome d'oxygène ou de soufre non voisin directement de la partie éther d'oxime, toutes les chaînes mentionnées ci-dessus portant en position terminale le cycle phényle qui peut lui-même porter un à trois substituants choisis dans un groupe consistant en un groupe benzyloxycarbonyle ou phényle et dans chaque cas, un à trois des substituants suivants :
nitro, cyano, alkyle en C1-C4, alcoxy en C1-C4, alkylthio en C1-C4, alkyle en C1-C4 partiellement ou totalement halogéné, alcoxy en C1-C4 partiellement ou totalement halogéné, carboxyle et (alcoxy en C1-C4)carbonyle, le cycle phényle pouvant encore porter des atomes d'halogènes en nombre tel que le nombre total des substituants soit de 4 ou 5 ;
un groupe thiénylméthyle qui peut encore porter un atome d'halogène ;
R^{f} : un groupe alkyle en C1-C4 qui peut porter un ou deux substituants alkylthio en C1-C4 ou alcoxy en C1-C4 ;
un système cyclique à cinq ou six chaînons, saturé ou monoinsaturé qui, en plus des chaînons carbonés, peut contenir un atome d'oxygène, un atome de soufre, un groupe sulfoxyde ou un groupe sulfone, ce cycle pouvant porter jusqu'à trois des substituants suivants : hydroxy, halogéno, alkyle en C1-C4, alkyle en C1-C4 partiellement ou totalement halogéné, alcoxy en C1-C4 et/ou alkylthio en C1-C4 ;
un hétérocycle à dix chaînons, saturé ou mono-insaturé, qui contient deux atomes d'oxygène ou de soufre et qui peut porter un à trois substituants alkyle en C1-C4 et/ou méthoxy ;
le groupe phényle, le groupe pyridyle, le groupe pyrrolyle, le groupe pyrazolyle, thiazolyle ou isoxazolyle, ces groupes pouvant porter si on le désire un à trois des substituants suivants : alkyle en C1-C4, alkyle en C1-C4 partiellement ou totalement halogéné, alcoxy en C1-C4, alkylthio en C1-C4, alcényloxy en C3-C6, alcynyloxy en C3-C6, (alcoxy en C1-C4)alkyle en C1-C3, (alkylthio en C1-C4)alkyle en C1-C3, di-(alcoxy en C1-C4)alkyle en C1-C3, formyle, halogéno et/ou benzoylamino ;
R^{g} : l'hydrogène, un groupe hydroxy ou bien un groupe alkyle en C1-C6 lorsque R^{f} représente un groupe alkyle en C1-C6 ;
R^{h} : l'hydrogène, le groupe cyano, un atome d'halogène, un groupe (alcoxy en C1-C4)-carbonyle ou un groupe 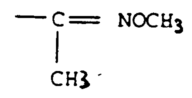 et
Rⁱ représente l'hydrogène ou un équivalent d'un cation utilisable dans des applications agricoles.

2. Produit herbicide selon revendication 1, contenant un 2-aminothiophène substitué I en tant que composé a effet antagoniste et un produit herbicide III dans des proportions relatives en poids de 0,01 : 1 à 10 : 1.

3. Procédé pour combattre sélectivement les croissances de végétaux indésirables, caractérisé par le fait que, avant ou après les semailles des végétaux cultivés avant ou durant la sortie de terre des végétaux cultivés, on applique simultanément ou successivement un 2-aminothiophène substitué I en tant qu'antagoniste et un dérivé de cyclohexénone de formule III selon la revendication 1.

4. Procédé pour combattre sélectivement les croissances de végétaux indésirables, caractérisé par le fait que l'on traite simultanément ou successivement le feuillage des végétaux cultivés et des végétaux indésirables, en post-levée, par un 2-aminothiophène substitué I en tant qu'antagoniste et un dérivé de cyclohexénone de formule III selon la revendication 1.

5. Procédé pour protéger les végétaux cultivés contre les dommages provoqués par les dérivés de cyclohexénones herbicides de formule III selon la revendication 1, caractérisé par le fait que l'on traite les semences de la plante cultivée par une quantité antagoniste efficace d'un 2-aminothiophène substitué de formule I selon la revendication 1.

6. Procédé selon les revendications 3 à 5, caractérisé par le fait que la plante cultivée est l'orge, le blé, le maïs, le sorgho cultivé ou le riz.
